# EUROPEAN PATENT APPLICATION

(11) **EP 2 260 839 A2**
(43) Date of publication of application: **15.12.2010**
(21) Application number: 10181505.8
(22) Date of filing: 23.10.2003
(51) Int. Cl.: A61K 31/13, A61P 25/00, A61K 31/445, A61K 31/55, A61K 31/16, A61K 31/435

(54) **Combination therapy using 1-aminocyclohexane derivatives and acetylcholinesterase inhibitors**

(30) Priority: 24.10.2002 US 420918 P
(62) Divisional of application: 03758338.2
(71) Applicant: Merz Pharma GmbH & Co. KGaA, 60318 Frankfurt (DE)
(72) Inventor: Moebius, Hans-Joerg, 60322 Frankfurt am Main (DE)
(74) Representative: Goddard, Christopher Robert

(57) **Abstract**

A drug combination therapy useful in the treatment of dementia associated with disorders of the central nervous system, e.g. to delay the onset or progression of Alzheimer's disease, cerebrovascular disease or Down's syndrome, comprises a combination of a 1-aminocyclohexane derivative such as memantine or neramexane and an acetylcholinesterase inhibitor such as galantamine, tacrine, donepezil or rivastigmine.

## Description

### FIELD OF THE INVENTION

The present invention relates to the combinations of 1-aminocyclohexane derivatives and acetylcholinesterase inhibitors and their use in the treatment of dementia.

### BACKGROUND OF THE INVENTION

Dementia is a serious disorder affecting as many as 10% of individuals older than 65 years and more than 24% of those older than 85 years (Hofman et al., Int. J. Epidemiol., 1991, 20:736-748; Jorm and Jolley, Neurology, 1998, 51:728-733; Lobo et al., Neurology, 2000, 54(Suppl. 5):S4-S9). Alzheimer's disease (AD) is an increasingly prevalent form of neurodegeneration that accounts for approximately 50 % - 60 % of the overall cases of dementia among people over 65 years of age. AD is characterized clinically by progressive loss of memory, cognition, reasoning, judgement, and emotional stability that gradually leads to profound mental deterioration and ultimately death. AD is a progressive disorder with a mean duration of around 8.5 years between onset of clinical symptoms and death. AD is believed to represent the fourth most common medical cause of death and affects about 2-3 million people in the United States. Prevalence of AD doubles every 5 years beyond age 65 (National Institute on Aging: Prevalence and costs of Alzheimer's disease. Progress Report on Alzheimer's Disease. NIH Publication No. 99 3616, November 1998; Polvikoski et al., Neurology, 2001, 56:1690-1696). AD currently affects about 15 million people worldwide (including all races and ethnic groups) and owing to the relative increase of elderly people in the population its prevalence is likely to increase over the next two to three decades. AD is at present incurable. No treatment that effectively prevents AD or reverses its symptoms and course is currently known.

AD is associated with death of pyramidal neurons and loss of neuronal synapses in brains regions associated with higher mental functions (Francis et al., 1999, J. Neurol. Neurosurg. Psychiatry, 66:137-147). The brains of individuals with AD exhibit characteristic lesions termed senile (or amyloid) plaques, amyloid angiopathy (amyloid deposits in blood vessels) and neurofibrillary tangles. Smaller numbers of these lesions in a more restricted anatomical distribution are also found in the brains of most aged humans who do not have clinical AD. Amyloid plaques and amyloid angiopathy also characterize the brains of individuals with Trisomy 21 (Down's Syndrome) and Hereditary Cerebral Hemorrhage with Amyloidosis of the Dutch-Type (HCHWA-D). At present, a definitive diagnosis of AD usually requires observing the aforementioned lesions in the brain tissue of patients who have died with the disease or, rarely, in small biopsied samples of brain tissue taken during an invasive neurosurgical procedure.

AD is associated with a profound loss of cholinergic neurons within the nucleus basalis of Meynert (Perry et al., Br. Med. J., 1978, 2:1456-1459; Geula and Mesulam, Cholinergic systems and related neuropathological predilection patterns in Alzheimer disease. In: Alzheimer's Disease. Terry et al. eds.; New York: Raven Press; 1994, pp. 263-291). The signaling in these neurons is mediated by the extracellularly released neurotransmitter acetylcholine (ACh). Recognition of the role of dysfunction of ACh signaling system in the cognitive impairments associated with AD as well as a number of other neurological and psychiatric disorders including Parkinson's disease, schizophrenia, epilepsy, depression, obsessive compulsive disorders, and bipolar disorders has led to the development of drugs that selectively enhance cholinergic function by inhibition of the cholinergic catabolic enzyme acetylcholinesterase (AChE), which destroys ACh after the latter has been secreted into the synaptic clefts (Goff and Coyle, Am. J. Psychiatry, 2001, 158: 1367-1377). At present, the most widely clinically used acetylcholinesterase inhibitors (AChEI) are tacrine (THA; 1,2,3,4-tetrahydro-9-aminoacridine hydrochloride), DFP (diisopropylfluorophosphate), physostigmine, donepezil, galantamine, and rivastigmine. Many of AChEI selectively inhibit AChE, but agents that also target butyrylcholinesterase (BuChE) may provide added benefits as AD progresses and ACh regulation may become increasingly dependent on BuChE. Dual inhibition may also help to slow the formation of amyloidogenic compounds (Ballard, Eur. Neurol., 2002, 47:64-70).

Donepezil ([(R,S)-1-benzyl-4-[(5,6-dimethoxy-1-indanon)-2y1]-methylpiperidine hydrochloride]; ARICEPT, previously E-2020) is a reversible, noncompetitive, piperidine-type AChEI, which is selective for AChE rather than BuChE (Sugimoto et al., Curr. Med. Chem., 2000, 7:303-39). Dooley et al. (Drugs Aging, 2000, 16:199-226) have demonstrated that donepezil administered at doses of 5 and 10 mg/day significantly improved cognition and global clinical function compared with placebo in short term trials (14 to 30 weeks) in 161 to 818 patients with mild to moderate AD (*see* also Rogers et al., Arch. Int. Med., 1998; 158:1021-1031). Long-term efficacy data obtained in these studies suggest that improvements in cognition, global function or activities of daily living (ADL) are maintained for about 21 to 81 weeks and that donepezil is generally well tolerated with the majority of adverse events being mild and transient.

Galantamine (REMINYL) is a reversible, competitive, tertiary alkaloid AChEI, which is selective for AChE rather than BuChE. As demonstrated by Scott et al. (Drugs, 2000; 60:1095-122), 285 to 978 patients with mild to moderate AD receiving galantamine at doses of 16 or 24 mg/day achieved significant improvements in cognitive and global symptoms relative to placebo recipients in trials of 3 to 6 months' duration. Adverse events associated with galantamine in these studies were usually mild to moderate in intensity and transient. Similar results were obtained by Coyle et al. (Biol. Psychiatry, 2001, 49:289-99).

Rivastigmine (EXELON) is a dual inhibitor of AChE and BuChE that has demonstrated benefits across the spectrum of AD severity (Ballard , Eur. Neurol., 2002, 47:64-70). Unlike tacrine and donepezil, which are classified as short-acting or reversible agents, rivastigmine is an intermediate-acting or pseudo-irreversible agent, which inhibits AChE for up to 10 hours. Preclinical biochemical studies indicated that rivastigmine has central nervous system (CNS) selectivity over peripheral inhibition. Rivastigmine was shown to ameliorate memory impairment in rats with forebrain lesions; and in the two large multicenter clinical trials (total 1324 patients) at doses of 6-12 mg/day it was superior to placebo on three cognitive and functioning scales (Jann, Pharmacotherapy, 2000, 20:1-12).

The excessive or pathological activation of glutamate receptors, particularly those that are selectively activated by N-methyl-D-aspartate (NMDA), has also been implicated in the processes that underlie the degeneration of cholinergic cells in the brains of AD patients (Greenamyre et al., Neurobiol. Aging, 1989, 10:593 - 602; Francis et al., J. Neurochem., 1993, 60:263-291; Li et al., J. Neuropathol. Exp. Neurol., 1997, 56:901-911; Wu and Rowan, Neuroreport, 1995, 6:2409 - 2413). The NMDA receptor is very well established to be pivotal for several physiologic synaptic plasticity processes, *e.g*., memory and learning (Collinridge and Singer, Trends Pharmacol. Sci., 1990, 11: 290-296). The functioning of the NMDA receptor requires the activation of both the agonist binding site for glutamate and the allosteric co-agonist site which is activated by glycine and D-serine (Kleckner and Dingledine, Science, 1988, 241:835 - 837; McBain et al., Mol. Pharmacol., 1989, 36:556-565; Danysz and Parsons, Pharmacol. Rev., 1998, 50:597-664). Activation of the D-serine-sensitive modulatory site on the NMDA receptor has been shown to be a prerequisite for induction of long - term potentiation (Bashir et al., Neurosci Lett., 1990, 108:261-266), an *in vitro* correlate of memory and learning. Furthermore, the cognitive deficits associated with psychiatric disorders such as schizophrenia have been shown to be alleviated by oral treatment with D-serine (Tsai et al., Biol Psychiatry, 1998, 44:1081-1089). Even though NMDA receptor activation is critical for learning, moderate affinity uncompetitive NMDA receptor antagonists have been found to correct/reverse cognitive impairment in both human AD and animal models of Alzheimer's dementia. To the degree that excessive glutamatergic function is a contributor in AD, effective pharmacological antagonism of the NMDA receptor, particularly by open channel blockers, may be able to slow the progression of AD (Parsons et al., Neuropharmacol., 1999, 38:735-767; Danysz and Möbius, 2002, Alzheimer's Disease Neuroprotection - Therapeutic Potential of Ionotropic Glutamate Receptor Antagonists and Modulators, In: Therapeutic Potential of Ionotropic Glutamate Receptor Antagonists and Modulators, Lodge et al. eds., 2002, in press, F.P. Graham Publishing Co., New York).

NMDA receptor antagonists potentially have a wide range of therapeutic applications in numerous CNS disorders such as acute neurodegeneration ( *e.g.,* associated with stroke and trauma), chronic neurodegeneration (*e.g*., associated with Parkinson's disease, AD, Huntington's disease, and amyotrophic lateral sclerosis [ALS]), epilepsy, drug dependence, depression, anxiety, and chronic pain (for reviews *see*: Parsons et al., Drug News Perspect., 1998, 11:523-533; Parsons *et al.,* 1999, *supra*; Jentsch and Roth, Neuropsychopharmacology, 1999, 20: 201-205; Doble, Therapie, 1995, 50: 319-337). Functional inhibition of NMDA receptors can be achieved through actions at different recognition sites within the NMDA receptor complex, such as: the primary transmitter site (competitive), the phencyclidine site located inside the cation channel (uncompetitive), the polyamine modulatory site and the strychnine-insensitive, co-agonistic glycine site (glycine B) (Parsons *et al.,* 1999, *supra*)*.* As NMDA receptors also play a crucial physiological role in various forms of synaptic plasticity such as those involved in learning and memory (*see, e.g.,* Collingridge and Singer, Trends Pharmacol. Sci., 1990, 11:290-296), neuroprotective agents possessing high affinity for the NMDA receptors are likely to impair normal synaptic transmission and thereby cause numerous side effects. Indeed, many NMDA receptor antagonists identified to date produce highly undesirable side effects at doses within their putative therapeutic range. Thus, clinical trials failed to support good therapeutic utility due to numerous side effects for such NMDA receptor antagonists as Dizocilpine ((+)MK-801; (+)-5-methyl-10,11-dihydro-5H-dibenzocyclohepten-5,10-imine maleate), Cerestat (CNS-1102), Licostinel (ACEA 1021), Selfotel (CGS-19755), and D-CPP-ene (Leppik , Epilepsia, 1998, 39 (Suppl 5):2-6; Sveinbjornsdottir et al., Epilepsia, 1993, 34:493-521; SCRIP 2229/30, 1997, p. 21). The challenge in the field has therefore been to develop NMDA receptor antagonists that prevent the pathological activation of NMDA receptors but allow their physiological activity.

Memantine (1-amino-3,5-dimethyl adamantane) is an analog of 1-aminocyclohexane (disclosed, *e.g*., in U.S. Patents No. 4,122,193; 4,273,774; 5,061,703). Neramexane (1-amino-1,3,3,5,5-pentamethylcyclohexane) is also a derivative of 1-aminocyclohexane (disclosed, *e.g*., in U.S. Patent No. 6,034,134). Memantine, neramexane as well as some other 1-aminoalkyl-cyclohexanes are systemically-active noncompetitive NMDA receptor antagonists having moderate affinity for the receptor. They exhibit strong voltage dependent characteristics and fast blocking/unblocking kinetics (Parsons *et al*., 1999, *supra*; Görtelmeyer et al., Arzneim-Forsch/Drug Res., 1992, 42:904-913; Winblad et al., Int. J. Geriat. Psychiatry, 1999, 14:135-146; Rogawski, Amino Acids, 2000, 19: 133-49; Danysz et al., Curr. Pharm. Des., 2002, 8:835-43; Jirgensons et. al., Eur. J. Med. Chem., 2000, 35: 555-565). These compounds dissociate from the NMDA receptor channels much more rapidly than the high affinity NMDA receptor antagonists such as (+)MK-801 and attenuate disruption of neuronal plasticity produced by tonic overstimulation of NMDA receptors probably by causing an increase of the signal-to-noise ratio. Due to their relatively low affinity for the receptor, strong voltage dependency and fast receptor unblocking kinetics, these compounds are essentially devoid of the side effects of other NMDA receptor antagonists at doses within the therapeutic range (Kornhuber et al., Eur. J. Pharmacol., 1991, 206:297-311). Indeed, memantine has been applied clinically for over 15 years showing good tolerability with the number of treated patients exceeding 200,000 (Parsons *et al*., 1999, *supra*).

Memantine, neramexane as well as other 1-aminoalkylcyclohexanes have been suggested to be useful in alleviation of various progressive neurodegenerative disorders such as dementia in AD, Parkinson's disease, and spasticity (*see*, *e.g.,* U. S. Patents No. 5,061,703; 5,614,560, and 6,034,134; Parsons *et al*., 1999, *supra*; Möbius, ADAD, 1999,13:S172-178; Danysz et al., Neurotox. Res., 2000, 2:85-97; Winblad and Poritis, Int. J. Geriatr. Psychiatry, 1999, 14:135-146; Görtelmeyer *et al*., 1992, *supra*; Danysz et al., Curr. Pharm. Des., 2002, 8:835-843; Jirgensons et. al., Eur. J. Med. Chem., 2000, 35: 555-565). These diseases result from disturbances of glutamatergic transmission, *i*.*e*., the excessive influx of calcium through NMDA receptor channels, leading to the destruction of brain cells in specific brain areas (Choi, J. Neurobiol., 23: 1261-1276, 1992; Rothman and Olney, Trends Neurosci., 10: 299, 1987; Kemp et al., Trends Pharmacol. Sci., 8: 414, 1987). Chronic treatment of adult rats with memantine has been shown to enhance the formation of hippocampal long-term potentiation, increase the durability of synaptic plasticity, improve spatial memory abilities, and reverse the memory impairment produced by NMDA receptor agonists (Barnes et al., Eur. J. Neurosci., 1996; 8:65-571; Zajaczkowski et al., Neuropharm, 1997, 36:961-971). 1-Aminocyclohexane derivatives, and specifically memantine, have also been suggested to be useful in the treatment of AIDS dementia (U.S. Patent No. 5,506,231), neuropathic pain (U.S. Patent No. 5,334,618), cerebral ischemia (U.S. Patent No. 5,061,703), epilepsy, glaucoma, hepatic encephalopathy, multiple sclerosis, stroke, and tardive dyskinesia (Parsons *et al*., 1999, *supra*). Furthermore, relatively high doses of memantine and neramexane were shown to selectively block thermal hyperalgesia and mechanical allodynia in some models of chronic and neuropathic pain without obvious effects on motor reflexes. 1-Aminoacyclohexane derivatives were also demonstrated to possess immunomodulatory, antimalarial, anti-Borna virus, and anti-Hepatitis C activities (*see*, *e.g.,* U.S. Patent No. 6,034,134 and references cited therein).

1-Aminocyclohexane derivatives such as memantine and neramexane (*see* U.S. Patent Application No. 09/597,102 and its corresonding international patent application PCT EP 01/06964 published as WO 01/98253 on December 27, 2001; U.S. Patent No. 6,034,134) have also been suggested to function via non-NMDA- mediated pathways. Thus, memantine was shown to inhibit 5HT3-mediated current (in the native N1E-115 and heterologous HEK-293 cells) and NMDA receptor-mediated currents (in rat hippocampal slices) with approximately equal affinity (Parsons *et al*., 1999, *supra*; Rammes et al., 2001, Neurosci. Lett., 306:81-84). 5HT3 receptor antagonists are known to improve learning and memory in animals (Carli et al., 1997, Behav. Brain Res., 82:185-194; Reznik and Staubli, 1997, J. Neurophysiol., 77:517-521).

As disclosed above, the loss of cholinergic neurons within the basal forebrain, which underlies various aspects of dementia, may result from the disruption in ACh-mediated signalling and/or excessive activation of NMDA receptors. Accumulating experimental evidence indicates that ACh- and NMDA receptor-mediated signalling systems are interconnected, *i.e*., that blockade of NMDA receptors can increase the extracellular release of ACh. Thus, it has been demonstrated that systemic administration of the NMDA receptor antagonist, (+)MK-801, produces a dose-dependent increase in the extracellular release of ACh in rat parietal and frontal cortices (Hasegawa et al., 1993, Neurosci. Lett., 150:53-56; Aquas et al., 1998, Neuroscience, 85:73-83). Similarly, intracerebroventricular (i.c.v.) administration of another NMDA receptor antagonist, CPP, has been shown to increase ACh release in the rat parietal cortex and hippocampus (Giovannini et al., 1994, Neurochem. Intl., 25:23-26; Giovannini et al., 1994, J. Neurosci., 14:1358-1365). It has been proposed that glutamate, by acting through the NMDA receptors on GABAergic and noradrenergic neurons, maintains a tonic inhibitory control over the basal forebrain cholinergic neurons projecting to the cerebral cortex (Kim et al., 1999, Mol. Psychiat., 4:344-352). Based on this circuit, in addition to possible blocking of NMDA overactivation, systemic administration of an NMDA receptor antagonist would be expected to decrease the inhibitory control of GABA on ACh neurons resulting in the increased release of ACh in the cortex.

Although drug therapies have been designed to either enhance cholinergic function by inhibiting AChE (*e.g*., using galantamine, tacrine, donepezil, or rivastigmine) or by attenuating NMDA receptor function (*e.g*., using 1-aminocyclohexane derivatives such as memantine or neramexane), it has not been recognized or suggested that a combination of these two therapeutic approaches may be even more beneficial at slowing the progression of the dementia (*e.g*., associated with AD).

On the contrary, a number of research groups published evidence indicating that memantine could potentially undermine the beneficial effects provided by AChEI. Thus, it has been reported that memantine can attenuate the inhibition of AChE produced by the reversible AChEIs carbofuran (Gupta et al., J. Toxicol. Environ. Hlth., 1989, 28:111-122) and aldicarb (Gupta et al., Drug Dev. Res., 1991, 24:329-341) and the irreversible AChEIs soman (Mclean et al., Toxicol. Appl. Pharmacol, 1992, 112:95-103).

The present inventor has conceived and demonstrated for the first time that the clinical combination of a 1-aminocyclohexane derivative such as memantine or neramexane with an AChEI such as galantamine, tacrine, donepezil, or rivastigmine, is an unexpectedly valuable pharmacotherapeutic approach to dementia. The present inventor hypothesized and demonstrated that, when administered in combination to subjects with AD, the effects of 1-aminocyclohexane derivatives and AChEIs would be of unexpected benefit and, at least over a period of time result in an unexpectedly superadditive relief of symptoms, evidenced by symptom reversal, and in this way would be particularly beneficial in the treatment of dementia.

### BRIEF DESCRIPTION OF THE DRAWINGS

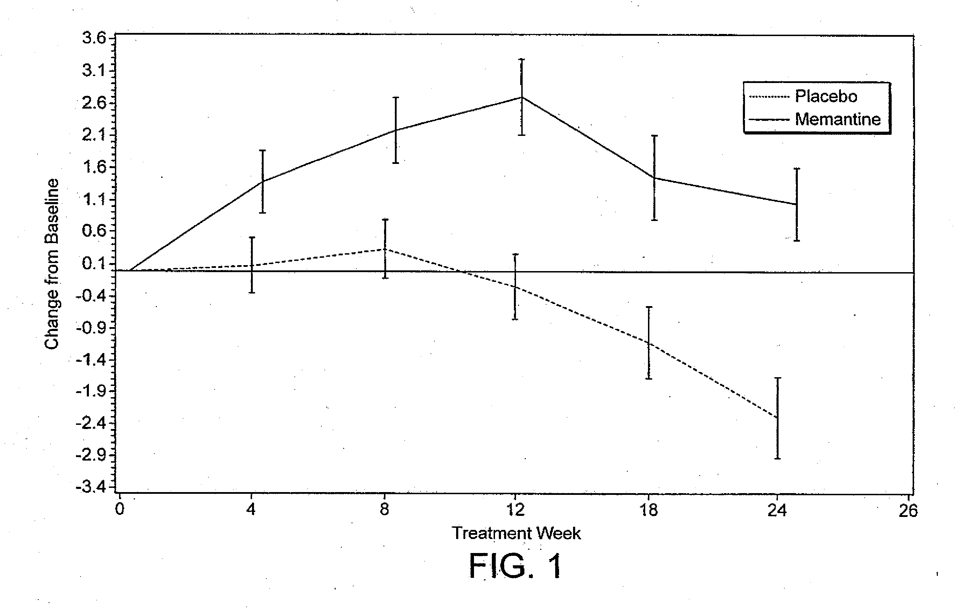

**Figure 1** shows Severe Impairment Battery (SIB) analysis of cognition demonstrating superior efficacy of the combined memantine/donepezil treatment compared to donepezil alone. Change from Baseline to Endpoint (LOCF): LS mean change (SE) is -2.5 (0.69) for placebo group and 0.9 (0.67) for memantine group; p value <0.001.

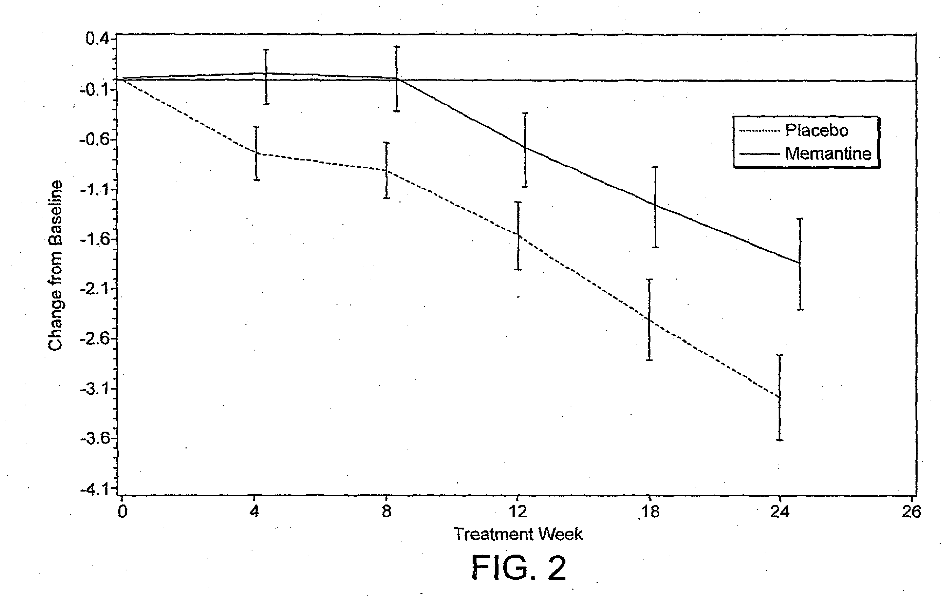

**Figure 2** shows Alzheimer's Disease Cooperative Study-Activities of Daily Living Inventory (ADCS-ADL) assesment of daily functions demonstrating superior efficacy of the combined memantine/donepezil treatment compared to donepezil alone. Change from Baseline to Endpoint (LOCF): LS mean change (SE) is - 3.4 (0.51) for placebo group and -2.0 (0.50) for memantine group; p value <0.028.

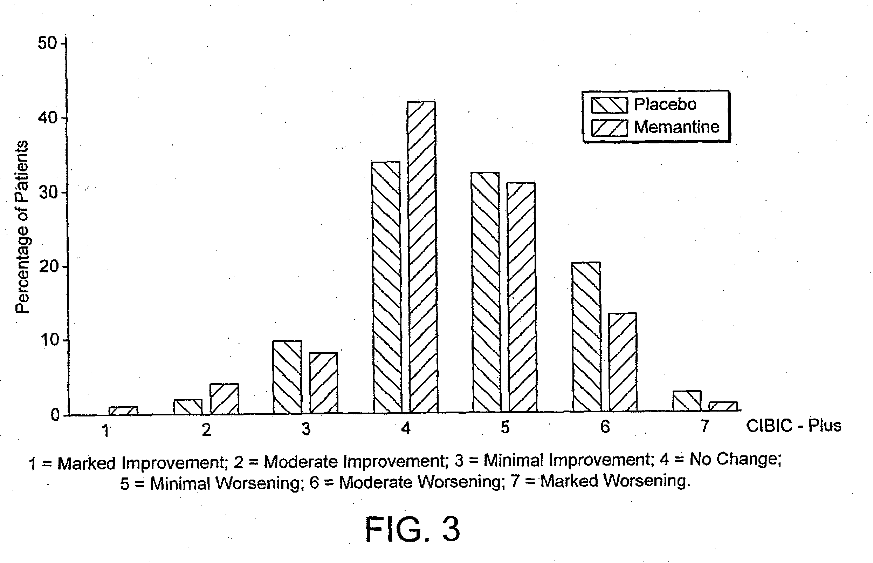

**Figure 3** shows Clinician's Interview-Based Impression of Change-Plus (CIBIC-Plus) global assesment demonstrating superior efficacy of the combined memantine/donepezil treatment compared to donepezil alone. Change from Baseline to Endpoint (LOCF): LS mean (SD) is 4.7 (1.05) for placebo group and 4.4 (1.05) for memantine group; p value <0.027.

### SUMMARY OF THE INVENTION

The instant invention provides a novel drug combination useful for treating, preventing, arresting, delaying the onset of and/or reducing the risk of developing dementia associated with a central nervous system (CNS) disorder in a mammal. In another aspect, the invention provides a method for accomplishing one or more of the foregoing, comprising administering to said mammal an 1-aminocyclohexane derivative and an acetylcholinesterase inhibitor (AChEI) in amounts effective for this purpose. In a specific embodiment, the 1-aminocyclohexane derivative useful in the combination therapy of the invention is memantine or neramexane and the AChEI is galantamine, tacrine, donepezil, rivastigmine, huperzine A, zanapezil, ganstigmine, phenserine, phenethylnorcymserine (PENC), cymserine, thiacymserine, SPH 1371 (galantamine plus), ER 127528, RS 1259, or F3796. Preferably, the mammal is human and the CNS disorder is Alzheimer's disease (AD), cerebrovascular disease (VaD), or Down's Syndrome.

In a more specific embodiment, the invention provides a method for delaying cognitive impairment or dementia, or reducing the risk of further cognitive decline or impairment, or arresting, or reversing or reducing cognitive decline or impairment resulting from dementia.

Accordingly, one object of the instant invention is to administer the above-described combination to human subjects who either do not yet show clinical signs of cognitive impairment or AD, but who are at risk of developing AD ( *e.g*., due to being homozygous or heterozygous mutants in Apolipoprotein E isoform 4; see also genetic screening and clinical analysis described in Goate, 1991, Nature, 349:704-706), or to individuals who may already show signs of mild cognitive impairment or may be at risk of such impairment (*e.g*., individuals having elevated levels of β-amyloid peptide [βAP] as described in Example 2, *infra*; see also references cited therein). By providing the combination comprising an 1-aminocyclohexane derivative and an AChEI, the invention provides compositions and methods for reducing the risk of developing AD or delaying the onset of AD in such individuals. In addition, as disclosed herein, such combination therapy will halt or reduce the rate of further cognitive decline and, over a period of time, reverse cognitive decline, as measured by at least one marker or method.

Another object of the present invention is to provide the above-described combination to persons who already have clinical signs of cognitive impairment or clinically manifest AD. By providing the combination therapy comprising administering an 1-aminocyclohexane derivative and an AChEI, the invention provides compositions and methods for halting or slowing the progression of AD in such persons, and over a period of time reversing the decline in at least one marker or symptom of AD in such persons. Examples of such symptoms or markers are patients' ADL, SIB or CIBIC scores.

As disclosed herein, preferably, the 1-aminocyclohexane derivative and the AChEI are administered conjointly, most preferably, simultaneously, and, even more preferably, in one composition. Preferably, these drugs are administered in therapeutically effective dosages, which are in the range 1-200 mg/day for each drug. Most preferably, the AChEI will be administered at 1-40 mg/day, and especially at 5-24 mg/day. Most preferably, the 1-aminocyclohexane derivative will be administered at 5-60 mg/day and especially at 10-40 mg/day.

Also provided herein are pharmaceutical compositions comprising therapeutically effective amounts of an 1-aminocyclohexane derivative and an AChEI as well as, optionally, at least one carrier or excipient (pharmaceutically acceptable). Further provided are methods for preparing such compositions comprising admixing each active ingredient with the pharmaceutically acceptable carrier or excipient. Also provided herein are kits comprising a first composition comprising an 1-aminocyclohexane derivative, in a first amount, and a second composition comprising an AChEI, in a second amount, said amounts in combination being therapeutically effective to treat dementia associated with a CNS disorder. In a preferred embodiment, the amount of one or the other active ingredient or both is suboptimal or subthreshold. In another preferred embodiment, the amount of each ingredient is sufficient to bring about a reversal of at least one symptom or marker, upon the conjoint administration of the two active ingredients.

### DETAILED DESCRIPTION OF THE INVENTION

### Combination of the Invention

As specified above, in one aspect, the instant invention provides a novel drug combination useful for treating, preventing, arresting, delaying the onset of and/or reducing the risk of developing, or reversing at least one symptom of dementia associated with a central nervous system (CNS) disorder, especially Alzheimer's disease (AD), cerebrovascular disease (VaD), or Down's Syndrome, in a mammal comprising administering to said mammal an 1-aminocyclohexane and an acetylcholinesterase inhibitor (AChEI). Preferably, the 1-aminocyclohexane derivative and the AChEI are administered at therapeutically effective dosages which, when combined, provide a beneficial effect.

### Definitions

The term "combination" applied to active ingredients is used herein to define a single pharmaceutical composition (formulation) comprising both drugs of the invention (*i.e.,* an 1-aminocyclohexane derivative and an AChEI) or two separate pharmaceutical compositions (formulations), each comprising a single drug of the invention (*i.e.,* an 1-aminocyclohexane derivative or an AChEI), to be administered conj ointly.

Within the meaning of the present invention, the term "conjoint administration" is used to refer to administration of the 1-aminocyclohexane derivative and AChEI simultaneously in one composition, or simultaneously in different compositions, or sequentially. For the sequential administration to be considered "conjoint", however, the 1-aminocyclohexane derivative and AChEI must be administered separated by a time interval that still permits the resultant beneficial effect for treating, preventing, arresting, delaying the onset of and/or reducing the risk of developing a dementia associated with a central nervous system (CNS) disorder in a mammal. For example, the 1-aminocyclohexane derivative and AChEI must be administered on the same day (*e.g*., each - once or twice daily), preferably within an hour of each other, and most preferably simultaneously.

The term "treat" is used herein to mean to relieve or alleviate at least one symptom of a disease in a subject. For example, in relation to dementia, the term "treat" may mean to relieve or alleviate cognitive impairment (such as impairment of memory and/or orientation) or impairment of global functioning (activities of daily living, ADL) and/or slow down or reverse the progressive deterioration in ADL or cognitive impairment. Within the meaning of the present invention, the term "treat" also denote to arrest, delay the onset (*i.e.,* the period prior to clinical manifestation of a disease) and/or reduce the risk of developing or worsening a disease. The term "protect" is used herein to mean prevent delay or treat, or all, as appropriate, development or continuance or aggravation of a disease in a subject. Within the meaning of the present invention, the dementia is associated with a CNS disorder, including without limitation neurodegenerative diseases such as Alzheimer's disease (AD), Down's Syndrome and cerebrovascular dementia (VaD). Preferably, the dementia is associated with Alzheimer's disease (AD).

For example, as disclosed herein, a prophylactic administration of an 1-aminocyclohexane derivative in combination with an AChEI can protect a recipient subject at risk of developing dementia (*e.g*., individuals having elevated levels of β-amyloid peptide [βAP] as described in Example 2, *infra*; individuals, who are homozygous or heterozygous mutants in Apolipoprotein E isoform 4; *see* also genetic screening and clinical analysis described in Goate, 1991, Nature, 349:704-706). Similarly, according to the present invention, a therapeutic administration of an 1-aminocyclohexane derivative conjointly with an AChEI can lead to slow-down in the development of clinical symptoms or even regression of symptoms.

The term "acetylcholinesterase inhibitor" or "AChEI" is used herein to refer to a drug that enhances function of cholinergic neurons by inhibiting the catabolic enzyme acetylcholinesterase (AChE). The term encompasses reversible, pseudo-reversible and irreversible AChEIs as well as AChEIs that selectively inhibit AChE, and AChEIs, that are less selective (*e.g*., also target butyrylcholinesterase, BuChE). Preferably, AChEIs useful in the methods and compositions of the present invention are reversible or pseudo-reversible. Specific examples of AChEIs useful in the methods and compositions of the present invention include, but are not limited to, tacrine (THA; 1,2,3,4-tetrahydro-9-aminoacridine hydrochloride), donepezil, galantamine, rivastigmine, huperzine A, zanapezil, ganstigmine, phenserine, phenethylnorcymserine (PENC), cymserine, thiacymserine, SPH 1371 (galantamine plus), ER 127528, RS 1259, and F3796.

Within the meaning of the present invention, the term "NMDA antagonist drugs" is used to refer to drugs, that can suppress the normal triggering of NMDA receptor-mediated neuronal firings. Preferred NMDA antagonist drugs of the invention are 1-aminocyclohexane derivatives such as memantine and neramexane. These compounds also have 5HT₃ antagonist activity and/or neuronal nicotinic receptor antagonist activity.

The term "analog" or "derivative" is used herein in the conventional pharmaceutical sense, to refer to a molecule that structurally resembles a reference molecule (such as 1-aminocyclohexane), but has been modified in a targeted and controlled manner to replace one or more specific substituents of the referent molecule with an alternate substituent, thereby generating a molecule which is structurally similar to the reference molecule. Synthesis and screening of analogs (*e.g*., using structural and/or biochemical analysis), to identify slightly modified versions of a known compound which may have improved or biased traits (such as higher potency and/or selectivity at a specific targeted receptor type, greater ability to penetrate mammalian blood-brain barriers, fewer side effects, etc.) is a drug design approach that is well known in pharmaceutical chemistry.

The term "1-aminocyclohexane derivative" is used herein to describe a compound which is derived from 1-aminocyclohexane (or an available derivative thereof, such as neramexane or memantine) in the process used to create a similar but slightly different drug.

The 1-aminocyclohexane derivatives of the present invention can be represented by the general formula (I): 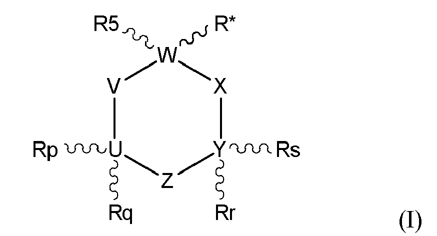 wherein:
- R^{*} is -(A)ₙ-(CR¹R²)ₘ-NR³R⁴,

   n+m = 0, 1, or 2,
   A is selected from the group consisting of linear or branched lower alkyl (C₁-C₆),linear or branched lower alkenyl (C₂-C₆), and linear or branched lower alkynyl (C₂-C₆),
   R¹ and R² are independently selected from the group consisting of hydrogen, linear or branched lower alkyl (C₁-C₆), linear or branched lower alkenyl (C₂-C₆), linear or branched lower alkynyl (C₂-C₆) aryl, substituted aryl and arylalkyl,
   R³ and R⁴ are independently selected from the group consisting of hydrogen, linear or branched lower alkyl (C₁-C₆), linear or branched lower alkenyl (C₂-C₆), and linear or branched lower alkynyl (C₂-C₆), or together form alkylene (C₂-C₁₀) or alkenylene (C₂-C₁₀) or together with the N form a 3-7-membered azacycloalkane or azacycloalkene, including substituted (alkyl (C₁-C₆), alkenyl (C₂-C₆)) 3-7-membered azacycloalkane or azacycloalkene; or independently R³ or R⁴ may join with R^{p}, R^{q}, R^{r}, or R^{s} to form an alkylene chain -CH(R⁶)-(CH₂)ₜ-, wherein t= 0 or 1 and the left side of the alkylene chain is attached to U or Y and the right side of the alkylene chain is attached to N and R⁶ is selected from the group consisting of hydrogen, linear or branched lower alkyl (C₁-C₆), linear or branched lower alkenyl (C₂-C₆), linear or branched lower alkynyl (C₂-C₆), aryl, substituted aryl and arylalkyl; or independently R³ or R⁴ may join with R⁵ to form an alkylene chain represented by the formula -CH₂-CH₂-CH₂-(CH₂)ₜ-, or an alkenylene chain represented by the formulae -CH=CH-CH₂-(CH₂)ₜ-, -CH=C=CH-(CH₂)ₜ- or -CH₂-CH=CH-(CH₂)ₜ-, wherein t= 0 or 1, and the left side of the alkylene or alkenylene chain is attached to W and the right side of the alkylene ring is attached to N;

- R⁵ is independently selected from the group consisting of hydrogen, linear or branched lower alkyl (C₁-C₆), linear or branched lower alkenyl (C₂-C₆), and linear or branched lower alkynyl (C₂-C₆), or R⁵ combines with the carbon to which it is attached and the next adjacent ring carbon to form a double bond,
- R^{p}, R^{q}, R^{r}, and R^{s}, are independently selected from the group consisting of hydrogen, linear or branched lower alkyl (C₁-C₆), linear or branched lower alkenyl (C₂-C₆), linear or branched lower alkynyl (C₂-C₆), cycloalkyl (C₃-C₆) and aryl, substituted aryl and arylaklyl or R^{p}, R^{q}, R^{r}, and R^{s} independently may form a double bond with U or with Y or to which it is attached, or R^{p}, R^{q}, R^{r}, and R^{s} may combine together to represent a lower alkylene -(CH₂)ₓ- or a lower alkenylene bridge wherein x is 2-5, inclusive, which alkylene bridge may, in turn, combine with R⁵ to form an additional lower alkylene -(CH₂)_{y}- or a lower alkenylene bridge, wherein y is 1-3, inclusive,
- the symbols U, V, W, X, Y, Z represent carbon atoms,
   and include optical isomers, diastereomers, polymorphs, enantiomers, hydrates, pharmaceutically acceptable salts, and mixtures of compounds within formula (I).

The ring defined by U-V-W-X-Y-Z is preferably selected from the group consisting of cyclohexane, cyclohex-2-ene, cyclohex-3-ene, cyclohex-1,4-diene, cyclohex-1,5-diene, cyclohex-2,4-diene, and cyclohex-2,5-diene.

Representative C₁₋₆ alkyl groups include methyl, ethyl, propyl and butyl groups, whilst representative C₂₋₆ alkenyl and alkynyl groups include ethenyl, propenyl, ethynyl and propynyl. Aryl groups which may be present include C_{6 -12} groups such as phenyl or naphthyl, optionally substituted by, for example, one or more substituents selected from C₁₋₆ alkyl groups such as methyl or ethyl, C₁₋₆ alkoxy groups such as methoxy or ethoxy, hydroxy groups and halogen atoms such as chlorine or bromine. Aralkyl groups which may be present include C_{6 -12} aryl-C₁₋₄ alkyl groups such as benzyl or phenethyl.

Non-limiting examples of 1-aminocyclohexane derivatives used according to the invention include the 1-aminoalkylcyclohexane derivatives selected from the group consisting of:
1-amino-1,3,5-trimethylcyclohexane,
1-amino-1(trans),3(trans),5-trimethylcyclohexane,
1-amino-1(cis),3 (cis),5-trimethylcyclohexane, 1-amino-1,3,3,5-tetramethylcyclohexane,
1-amino-1,3,3,5,5-pentamethylcyclohexane(neramexane),
1-amino-1,3,5,5-tetramethyl-3-ethylcyclohexane,
1-amino-1,5,5-trimethyl-3,3-diethylcyclohexane,
1-amino-1,5,5-trimethyl-cis-3-ethylcyclohexane,
1-amino-(1S,5S)cis-3-ethyl-1,5,5-trimethylcyclohexane,
1-amino-1,5,5-trimethyl-trans-3-ethylcyclohexane,
1-amino-(1R,5S)trans-3-ethyl-1,5,5-trimethylcyclohexane,
1-amino-1-ethyl-3,3,5,5-tetramethylcyclohexane,
1-amino-1-propyl-3,3,5,5-tetramethylcyclohexane,
N-methyl-1-amino-1,3,3,5,5-pentamethylcyclohexane,
N-ethyl-1-amino-1,3,3,5,5-pentamethyl-cyclohexane,
N-(1,3,3,5,5-pentamethylcyclohexyl)pyrrolidine,
3,3,5,5-tetramethylcyclohexylmethylamine,
1-amino-1-propyl-3,3,5,5-tetramethylcyclohexane,
1 amino-1,3,3,5(trans)-tetramethylcyclohexane (axial amino group),
3-propyl-1,3,5,5-tetramethylcyclohexylamine semihydrate,
1-amino-1,3,5,5-tetramethyl-3-ethylcyclohexane,
1-amino-1,3,5-trimethylcyclohexane,
1-amino-1,3-dimethyl-3-propylcyclohexane,
1-amino-1,3(trans),5(trans)-trimethyl-3(cis)-propylcyclohexane,
1-amino-1,3-dimethyl-3-ethylcyclohexane,
1-amino-1,3,3-trimethylcyclohexane,
cis-3-ethyl-1(trans)-3(trans)-5-trimethylcyclohexamine,
1-amino-1,3(trans)-dimethylcyclohexane,
1,3,3-trimethyl-5,5-dipropylcyclohexylamine,
1 -amino-1-methyl-3(trans)-propylcyclohexane,
1-methyl-3(cis)-propylcyclohexylamine,
1-amino-1-methyl-3(trans)-ethylcyclohexane,
1-amino-1,3,3-trimethyl-5(cis)-ethylcyclohexane,
1-amino-1,3,3-trimethyl-5(trans)-ethylcyclohexane,
cis-3-propyl-1,5,5-trimethylcyclohexylamine,
trans-3-propyl-1,5,5-trimethylcyclohexylamine,
N-ethyl-1,3,3,5,5-pentamethylcyclohexylamine,
N-methyl-1-amino-1,3,3,5.5-pentamethylcyclohexane,
1-amino-1-methylcyclohexane,
N,N-dimethyl-1-amino-1,3,3,5,5-pentamethylcyclohexane,
2-(3,3,5,5-tetramethylcyclohexyl)ethylamine,
2-methyl-1-(3,3,5,5-tetramethylcyclohexyl)propyl-2-amine,
2-(1,3,3,5,5-pentamethylcyclohexyl-1)-ethylamine semihydrate,
N-(1,3,3,5,5-pentamethylcyclohexyl)-pyrrolidine,
1-amino-1,3(trans),5(trans)-trimethylcyclohexane,
1-amino-1,3(cis),5(cis)-trimethylcyclohexane,
1-amino-(1R,SS)trans-5-ethyl-1,3,3-trimethylcyclohexane,
1-amino-(1S,SS)cis-5-ethyl-1,3,3-trimethylcyclohexane,
1-amino-1,5, 5-trimethyl-3(cis)-isopropyl-cyclohexane,
1-amino-1,5,5-trimethyl-3(trans)-isopropyl-cyclohexane,
1-amino-1-methyl-3(cis)-ethyl-cyclohexane,
1-amino-1-methyl-3(cis)-methyl-cyclohexane,
1-amino-5,5-diethyl-1,3,3-trimethyl-cyclohexane,
1-amino-1,3,3,5,5-pentamethylcyclohexane,
1-amino-1,5,5-trimethyl-3,3-diethylcyclohexane,
1-amino-1-ethyl-3,3,5,5-tetramethylcyclohexane,
N-ethyl-1-amino-1,3,3,5,5-pentamethylcyclohexane,
N-(1,3,5-trimethylcyclohexyl)pyrrolidine or piperidine,
N-[1,3(trans),5(trans)-trimethylcyclohexyl]pyrrolidine or piperidine,
N-[1,3(cis),5(cis)-trimethylcyclohexyl]pyrrolidine or piperidine,
N-(1,3,3,5-tetramethylcyclohexyl)pyrrolidine or piperidine,
N-(1,3,3,5,5-pentamethylcyclohexyl)pyrrolidine or piperidine,
N-(1,3,5,5-tetramethyl-3-ethylcyclohexyl)pyrrolidine or piperidine,
N-(1,5,5-trimethyl-3,3-diethylcyclohexyl)pyrrolidine or piperidine,
N-(1,3,3-trimethyl-cis-5-ethylcyclohexyl)pyrrolidine or piperidine,
N-[(1S,SS)cis-5-ethyl-1,3,3-trimethylcyclohexyl]pyrrolidine or piperidine,
N-(1,3,3-trimethyl-trans-5-ethylcyclohexyl)pyrrolidine or piperidine,
N-[(1R,SS)trans-5-ethyl,3,3-trimethylcyclohexyl]pyrrolidine or piperidine,
N-(1-ethyl-3,3,5,5-tetramethylyclohexyl)pyrrolidine or piperidine,
N-(1-propyl-3,3,5,5-tetramethylcyclohexyl)pyrrolidine or piperidine,
N-(1,3,3,5,5-pentamethylcyclohexyl)pyrrolidine,
   their optical isomers, diastereomers, enantiomers, hydrates, their pharmaceutically acceptable salts, and mixtures thereof.

Neramexane (1-amino-1,3,3,5,5-pentamethylcyclohexane) is disclosed, *e.g*., in U.S. Patent Application No. 09/597,102 and U. S. Patent No. 6,034,134.

Certain 1-aminocyclohexane derivatives of general formula (I) include the case where three axial alkyl substituent, *e.g*., R^{p}, R^{r} and R⁵, all together form a bridgehead to yield compounds (so called 1-aminoadamantanes) illustrated by the formulae IIb and IId below: 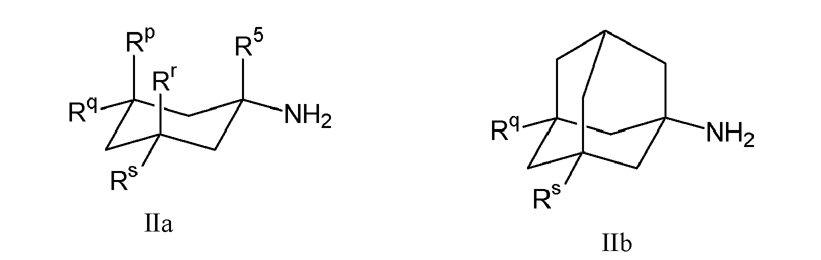 or

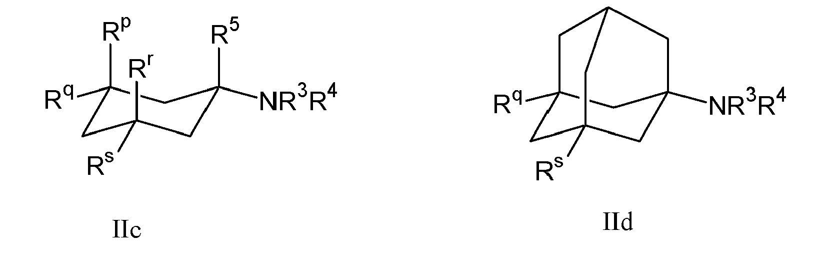

Certain 1-aminocyclohexane derivatives of forumula (I) wherein n + m = 0, U, V, W, X, Y and Z form a cyclohexane ring, and one or both of R³ and R⁴ are independently joined to said cyclohexane ring via alkylene bridges formed through R^{p}, R^{q}, R^{r}, R^{s} or R⁵ are represented by the following formulae IIIa-IIIc: 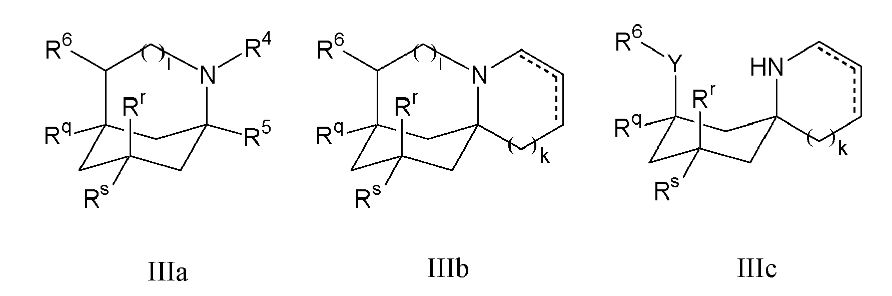 where R^{q}, R^{r}, R^{s}, R^{r} and R⁵ are as defined above for formula (I), R⁶ is hydrogen, linear or branched lower alkyl (C₁-C₆), linear or branched lower alkenyl (C₂-C₆), linear or branched lower alkynyl (C₂-C₆), aryl, substituted aryl or arylalkyl, Y is saturated or may combine with R⁶ to form a carbon-hydrogen bond with the ring carbon to which it is attached, 1= 0 or 1 and k= 0, 1 or 2 and ----- represents a single or double bond.

Non-limiting examples of 1-aminocyclohexane derivatives used according to the invention include 1-amino adamantane and its derivatives selected from the group consisting of:
1-amino-3-phenyl adamantane,
1-amino-methyl adamantane,
1-amino-3,5-dimethyl adamantane (memantine),
1-amino-3-ethyl adamantane,
1-amino-3-isopropyl adamantane,
1-amino-3-n-butyl adamantane,
1-amino-3,5-diethyl adamantane,
1-amino-3,5-diisopropyl adamantane,
1-amino-3,5-di-n-butyl adamantane,
1-amino-3-methyl-5-ethyl adamantane,
1-N-methylamino-3,5-dimethyl adamantane,
1-N-ethylamino-3,5-dimethyl adamantane,
1-N-isopropyl-amino-3,5-dimethyl adamantane,
1-N,N-dimethyl-amino-3,5-dimethyl adamantane,
1-N-methyl-N-isopropyl-amino-3-methyl-5-ethyl adamantane,
1-amino-3-butyl-5-phenyl adamantane,
1-amino-3-pentyl adamantane,
1-amino-3,5-dipentyl adamantane,
1-amino-3-pentyl-5-hexyl adamantane,
1-amino-3-pentyl-5-cyclohexyl adamantane,
1-amino-3-pentyl-5-phenyl adamantane,
1-amino-3-hexyl adamantane,
1-amino-3,5-dihexyl adamantane,
1-amino-3-hexyl-5-cyclohexyl adamantane,
1-amino-3-hexyl-5-phenyl adamantane,
1-amino-3-cyclohexyl adamantane,
1-amino-3,5-dicyclohexyl adamantane,
1-amino-3-cyclohexyl-5-phenyl adamantane,
1-amino-3,5-diphenyl adamantane,
1-amino-3,5,7-trimethyl adamantane,
1-amino-3,5-dimethyl-7-ethyl adamantane,
1-amino-3,5-diethyl-7-methyl adamantane,
1-N-pyrrolidino and 1-N-piperidine derivatives,
1-amino-3-methyl-5-propyl adamantane,
1-amino-3-methyl-5-butyl adamantane,
1-amino-3-methyl-5-pentyl adamantane,
1-amino-3-methyl-5-hexyl adamantane,
1-amino-3-methyl-5-cyclohexyl adamantane,
1-amino-3-methyl-5-phenyl adamantane,
1-amino-3-ethyl-5-propyl adamantane,
1-amino-3-ethyl-5-butyl adamantane,
1-amino-3-ethyl-5-pentyl adamantane,
1-amino-3-ethyl-5-hexyl adamantane,
1-amino-3-ethyl-5-cyclohexyl adamantane,
1-amino-3-ethyl-5-phenyl adamantane,
1-amino-3-propyl-5-butyl adamantane,
1-amino-3-propyl-5-pentyl adamantane,
1-amino-3-propyl-5-hexyl adamantane,
1-amino-3-propyl-5-cyclohexyl adamantane,
1-amino-3-propyl-5-phenyl adamantane,
1-amino-3-butyl-5-pentyl adamantane,
1-amino-3-butyl-5-hexyl adamantane,
1-amino-3-butyl-5-cyclohexyl adamantane, their optical isomers, diastereomers, enantiomers, hydrates, N-methyl, N,N-dimethyl, N-ethyl, N-propyl derivatives, their pharmaceutically acceptable salts, and mixtures thereof.

Memantine (1-amino-3,5-dimethyl adamantane), for example, is the subject matter of U.S. Patents No. 4,122,193 and 4,273,774.

The 1-amino adamantane derivatives of formulae IIb and IId, including memantine, are generally prepared by alkylation of halogenated adamantanes, preferably bromo- or chloroadamantanes. The di- or tri-substituted adamantanes are obtained by additional halogenation and alkylation procedures. The amino group is introduced either by oxidation with chromiumtrioxide and bromination with HBr or bromination with bromine and reaction with formamide followed by hydrolysis. The amino function can be alkylated according to generally-accepted methods. Methylation can, for example, be effected by reaction with chloromethyl formate and subsequent reduction. The ethyl group can be introduced by reduction of the respective acetamide. For more details on synthesis *see, e.g.,* U.S. Patents No. 5,061,703 and 6,034,134. Additional synthetic techniques for the foregoing compounds can be found in provisional applications Ser. No. 60/350,974 filed November 7, 2001, Ser. No. 60/337,858 filed November 8, 2001, and Ser. No. 60/366,386 filed March 21, 2002, all incorporated by reference, as well as in the Synthesis Examples below.

According to the invention, the 1-aminocyclohexane derivatives of formula (I) may be applied as such or used in the form of their pharmaceutically-acceptable salts including, for example, the acid addition salts such as hydrochlorides, hydrobromides, sulfates, acetates, succinates or tartrates, or their acid addition salts with fumaric, maleic, citric, or phosphoric acids.

In addition, using methods known to those skilled in the art, analogs and derivatives of the compounds of the invention can be created which have improved therapeutic efficacy in controlling dementia, *i.e*., higher potency and/or selectivity at a specific targeted receptor type, either greater or lower ability to penetrate mammalian blood-brain barriers (*e.g*., either higher or lower blood-brain barrier permeation rate), fewer side effects, etc.

Various salts and isomers (including stereoisomers and enantiomers) of the drugs listed herein can be used. The term "salts" can include addition salts of free acids or free bases. Examples of acids which may be employed to form pharmaceutically acceptable acid addition salts include inorganic acids such as hydrochloric, sulfuric, or phosphoric acid, and organic acids such as acetic, maleic, succinic, or citric acid, etc.. All of these salts (or other similar salts) may be prepared by conventional means. The nature of the salt or isomer is not critical, provided that it is non-toxic and does not substantially interfere with the desired pharmacological activity.

The term "therapeutically effective" applied to dose or amount refers to that quantity of a compound or pharmaceutical composition that is sufficient to result in a desired activity upon administration to a mammal in need thereof. As used herein with respect to the pharmaceutical compositions comprising an 1-aminocyclohexane derivative and/or an AChEI, the term "therapeutically effective amount/dose" is used interchangeably with the term "neurologically effective amount/dose" and refers to the amount/dose of a compound or pharmaceutical composition that is sufficient to produce an effective neurological response upon administration to a mammal. Note that when a combination of active ingredients is adminstered the effective amount of the combination may or may not include amounts of each ingredient that are individually effective.

The term "subthreshold" referring to the amount of an active ingredient means an amount inadequate to produce a response, *i.e*., an amount below the minimum effective amount. The term "suboptimal" in the same context means an amount of an active ingredient that produces a response but not to its full extent, which would be achieved with a higher amount.

The phrase "pharmaceutically acceptable", as used in connection with compositions of the invention, refers to molecular entities and other ingredients of such compositions that are physiologically tolerable and do not typically produce untoward reactions when administered to a mammal (*e.g*., human). Preferably, as used herein, the term "pharmaceutically acceptable" means approved by a regulatory agency of the Federal or a state government or listed in the U.S. Pharmacopeia or other generally recognized pharmacopeia for use in mammals, and more particularly in humans.

The term "carrier" applied to pharmaceutical compositions of the invention refers to a diluent, excipient, or vehicle with which an active compound (*e.g*., an 1-aminocyclohexane derivative and/or an AChEI) is administered. Such pharmaceutical carriers can be sterile liquids, such as water, saline solutions, aqueous dextrose solutions, aqueous glycerol solutions, and oils, including those of petroleum, animal, vegetable or synthetic origin, such as peanut oil, soybean oil, mineral oil, sesame oil and the like. Suitable pharmaceutical carriers are described in "Remington's Pharmaceutical Sciences" by E.W. Martin, 18th Edition.

The term "subject" as used herein refers to a mammal (*e.g*., rodent such as mouse or rat). In particular, the term refers to humans.

The term "about" or "approximately" usually means within 20%, more preferably within 10%, and most preferably still within 5% of a given value or range. Alternatively, especially in biological systems, the term "about" means within about a log (*i.e.,* an order of magnitude) preferably within a factor of two of a given value.

### Pharmaceutical Compositions

In conjunction with the methods of the present invention, also provided are pharmaceutical compositions comprising a therapeutically effective amount of an 1-aminocyclohexane derivative (such as memantine or neramexane) and/or a therapeutically effective amount of an acetylcholinesterase inhibitor (AChEI) (such as galantamine, tacrine, donepezil, or rivastigmine) as well as, optionally, an additional carrier or excipient (all pharmaceutically acceptable). Said 1-aminocyclohexane derivative and AChEI can be either formulated as a single composition or as two separate compositions, which can be administered conjointly. Preferably, they are formulated as a single composition or as two separate compositions, which are preferably administered simultaneously. The compositions can be formulated for once-a-day administration or twice-a-day administration. Thus, the aminocyclohexane derivative can be administered b-i-d and the AChEI can be administered b-i-d as one or as two different compositions for each administration. Or the aminocyclohexane deritave can be administered b-i-d and the AChEI can be administered once a day (or vice-versa). Or they can each be administered once a day as one or as two different compositions.

In the disclosed compositions, preferably, both the 1-aminocyclohexane derivative and AChEI are present in therapeutically effective amounts. The optimal therapeutically effective amount should be determined experimentally, taking into consideration the exact mode of administration, form in which the drug is administered, the indication toward which the administration is directed, the subject involved (*e.g*., body weight, health, age, sex, etc.), and the preference and experience of the physician or veterinarian in charge. As disclosed herein, for human administration, both the 1-aminocyclohexane derivatives and AChEIs are administered in suitable form in doses ranging from about 1 to 200 mg per day for each drug. More specifically, the 1-aminocyclohexane derivatives are preferably administered at doses 5-60 mg/day, and especially 10-40 mg/day; the AChEIs are preferably administered at doses 1-40 mg/day, and especially 5-24 mg/day. It may also be desirable in certain cases to administer one or the other of the active ingredients in a suboptimal or subthreshold amount, and such administration would also be within the invention.

The invention also provides a method for preparing pharmaceutical compositions comprising admixing an 1-aminocyclohexane derivative and/or an AChEI in therapeutically effective amounts, and optionally one or more physiologically acceptable carriers and/or excipients and/or auxiliary substances.

### Administration

The active agents of the present invention may be administered orally, topically, parenterally, or mucosally (*e.g*., buccally, by inhalation, or rectally) in dosage unit formulations containing conventional non-toxic pharmaceutically acceptable carriers. It is usually desirable to use the oral route. The active agents may be administered orally in the form of a capsule, a tablet, or the like (*see* Remington's Pharmaceutical Sciences, Mack 5 Publishing Co., Easton, PA). The orally administered medicaments may be administered in the form of a time-controlled release vehicle, including diffusion-controlled systems, osmotic devices, dissolution-controlled matrices, and erodible/degradable matrices.

For oral administration in the form of a tablet or capsule, the active drug component can be combined with non-toxic, pharmaceutically acceptable excipients such as binding agents (*e.g*., pregelatinized maize starch, polyvinylpyrrolidone or hydroxypropyl methylcellulose); fillers (*e.g*., lactose, sucrose, glucose, mannitol, sorbitol and other reducing and non-reducing sugars, microcrystalline cellulose, calcium sulfate, or calcium hydrogen phosphate); lubricants (*e.g*., magnesium stearate, talc, or silica, steric acid, sodium stearyl fumarate, glyceryl behenate, calcium stearate, and the like); disintegrants (*e.g*., potato starch or sodium starch glycolate); or wetting agents (*e.g*., sodium lauryl sulphate), coloring and flavoring agents, gelatin, sweeteners, natural and synthetic gums (such as acacia, tragacanth or alginates), buffer salts, carboxymethylcellulose, polyethyleneglycol, waxes, and the like. For oral administration in liquid form, the drug components can be combined with non-toxic, pharmaceutically acceptable inert carriers (*e.g*., ethanol, glycerol, water), suspending agents (*e.g*., sorbitol syrup, cellulose derivatives or hydrogenated edible fats), emulsifying agents (*e.g*., lecithin or acacia), non-aqueous vehicles (*e.g*., almond oil, oily esters, ethyl alcohol or fractionated vegetable oils), preservatives (*e.g*., methyl or propyl-p-hydroxybenzoates or sorbic acid), and the like. Stabilizing agents such as antioxidants (BHA, BHT, propyl gallate, sodium ascorbate, citric acid) can also be added to stabilize the dosage forms.

The tablets can be coated by methods well known in the art. The compositions of the invention can be also introduced in microspheres or microcapsules, *e.g*., fabricated from polyglycolic acid/lactic acid (PGLA) (*see, e.g.,* U.S. Patents No. 5,814,344; 5,100,669 and 4,849,222; PCT Publications No. WO95/11010 and WO93/07861). Liquid preparations for oral administration can take the form of, for example, solutions, syrups, emulsions or suspensions, or they can be presented as a dry product for reconstitution with water or other suitable vehicle before use. Preparations for oral administration can be suitably formulated to give controlled or postponed release of the active compound. A particular example of an oral time-controlled release pharmaceutical formulation is described in U. S. Patent No. 5,366,738.

The active drugs can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines, as is well known.

Drugs of the invention may also be delivered by the use of monoclonal antibodies as individual carriers to which the compound molecules are coupled. Active drugs may also be coupled with soluble polymers as targetable drug carriers. Such polymers can include polyvinyl-pyrrolidone, pyran copolymer, polyhydroxy - propyl methacrylamide-phenol, polyhydroxy-ethyl-aspartamide-phenol, or polyethyleneoxide-polylysine substituted with palmitoyl residues. Furthermore, active drug may be coupled to a class of biodegradable polymers useful in achieving controlled release of a drug, for example, polylactic acid, polyglycolic acid, copolymers of polylactic and polyglycolic acid, polyepsilon caprolactone, polyhydroxybutyric acid, polyorthoesters, polyacetals, polyhydropyrans, polycyanoacrylates, and cross-linked or amphipathic block copolymers of hydrogels.

For administration by inhalation, the therapeutics according to the present invention can be conveniently delivered in the form of an aerosol spray presentation from pressurized packs or a nebulizer, with the use of a suitable propellant, *e.g*., dichlorodifluoromethane, trichlorofluoromethane, dichlorotetrafluoroethane, carbon dioxide, or other suitable gas. In the case of a pressurized aerosol, the dosage unit can be determined by providing a valve to deliver a metered amount. Capsules and cartridges of, *e.g*., gelatin for use in an inhaler or insufflator can be formulated containing a powder mix of the compound and a suitable powder base such as lactose or starch.

The formulations of the invention can be delivered parenterally, *i.e*., by intravenous (i.v.), intracerebroventricular (i.c.v.), subcutaneous (s.c.), intraperitoneal (i.p.), intramuscular (i.m.), subdermal (s.d.), or intradermal (i.d.) administration, by direct injection, via, for example, bolus injection or continuous infusion. Formulations for injection can be presented in unit dosage form, *e.g*., in ampoules or in multi-dose containers, with an added preservative. The compositions can take such forms as excipients, suspensions, solutions, or emulsions in oily or aqueous vehicles, and can contain formulatory agents such as suspending, stabilizing and/or dispersing agents. Alternatively, the active ingredient can be in powder form for reconstitution with a suitable vehicle, *e.g*., sterile pyrogen-free water, before use.

Compositions of the present invention can also be formulated for rectal administration, *e.g*., as suppositories or retention enemas (*e.g*., containing conventional suppository bases such as cocoa butter or other glycerides).

As disclosed herein, an 1-aminocyclohexane derivative and AChEI can be mixed with excipients which are pharmaceutically acceptable and compatible with the active ingredients. In addition, if desired, the preparations may also include minor amounts of auxiliary substances such as wetting or emulsifying agents, pH buffering agents, and/or agents that enhance the effectiveness of the pharmaceutical composition. These auxiliary molecules can be delivered systemically or locally as proteins or by expression of a vector that codes for expression of the molecule. The techniques described above for the delivery of 1-aminocyclohexane derivatives and AChEIs can also be employed for the delivery of auxiliary molecules.

Although the active agents of the present invention may be administered in divided doses, for example, two or three times daily, a single daily dose of each of the 1-aminocyclohexane derivative and AChEI is preferred, with a single daily dose of both agents in one composition or in two separate compositions administered simultaneously being most preferred.

The instant invention also encompasses a process for preparing pharmaceutical compositions comprising combining an 1-aminocyclohexane derivative and/or an AChEI with a pharmaceutically acceptable carrier and/or excipient.

Preferred specific amounts of the 1-aminocyclohexane derivative which may be used in unit dosage amounts of the invention include, for example, 5mg, 10 mg, 15 mg, and 20 mg for memantine and 5 mg, 10 mg, 20 mg, 30 mg, and 40 mg for neramexane. Preferred specific amounts of the AChEI which may be used in unit dosage amounts of the invention include, for example, 1.5 mg, 3 mg, 4.5 mg, and 6 mg for rivastigmine, 4 mg, 8 mg and 12 mg for galantamine, and 5 mg and 10 mg for donepezil.

The invention also provides a pharmaceutical pack or kit comprising one or more containers containing one or more of the ingredients of the formulations of the invention. In a related embodiment, the present invention provides a kit for the preparation of the pharmaceutical compositions of the invention, said kit comprising an 1-aminocyclohexane derivative in a first container, and an AChEI in a second container, and, optionally, instructions for admixing the two drugs and/or for administration of the compositions. Each container of the kit may also optionally include one or more physiologically acceptable carriers and/or excipients and/or auxiliary substances. Associated with such container(s) can be a notice in the form prescribed by a governmental agency regulating the manufacture, use or sale of pharmaceuticals or biological products, which notice reflects approval by the agency of manufacture, use or sale for human administration.

The compositions may, if desired, be presented in a pack or dispenser device which may contain one or more unit dosage forms containing the active ingredient. The pack may, for example, comprise metal or plastic foil, such as a blister pack. The pack or dispenser device may be accompanied by instructions for administration. Compositions of the invention formulated in a compatible pharmaceutical carrier may also be prepared, placed in an appropriate container, and labeled for treatment of an indicated condition.

### Effective Dose and Safety Evaluations

According to the methods of the present invention, the pharmaceutical compositions described herein are administered to a patient at therapeutically effective doses, preferably, with minimal toxicity. The Section entitled "Definitions" provides definitions for the terms "neurologically effective dose" and "therapeutically effective dose". Preferably, the 1-aminocyclohexane derivative and the AChEI are each used at a dosage which, when combined, provide an enhanced effect, most preferably, an effect not observed upon administration of each agent alone.

The efficacy of the 1-aminocyclohexane derivatives of the invention can be determined using such *in vitro* pharmacological tests as measurements of displacement of [³H]MK-801 binding in rat or human brain tissue, blocking of NMDA receptor channels in cultured neurones and heterologous expression systems, anticonvulsive effects *in vivo,* correlation between channel-blocking and anticonvulsive action, protection against cerebral ischemia, protection against NMDA-induced mortality, etc. (see, *e.g*., U.S. Patent No. 5,061,703).

The efficacy of the AChEIs of the invention can be determined *in vitro* using such well-known methods as the spectrophotometric assay of AChE activity described by Ellman et al. (Biochem. Pharmacol., 7: 86 -95, 1961; *see* also Wenk et al., Life Sci., 2000, 66:1079 -1083).

Following methodologies which are well-established in the art, effective doses and toxicity of the compounds and compositions of the instant invention, which performed well in *in vitro* tests, are then determined in preclinical studies using small animal models (*e.g*., mice or rats) in which both the 1-aminocyclohexane derivatives and AChEIs has been found to be therapeutically effective and in which these drugs can be administered by the same route proposed for the human clinical trials. Preferred animal models of the invention are transgenic models of AD disclosed in Example 2, *infra.*

For any pharmaceutical composition used in the methods of the invention, the therapeutically effective dose can be estimated initially from animal models to achieve a circulating plasma concentration range that includes the IC₅₀ (*i.e.,* the concentration of the test compound which achieves a half-maximal inhibition of NMDA receptor activity and/or AChE enzymatic activity in the relevant areas of the brain). Dose-response curves derived from animal systems are then used to determine testing doses for the initial clinical studies in humans. In safety determinations for each composition, the dose and frequency of administration should meet or exceed those anticipated for use in the clinical trial.

As disclosed herein, the dose of the components in the compositions of the present invention is determined to ensure that the dose administered continuously or intermittently will not exceed an amount determined after consideration of the results in test animals and the individual conditions of a patient. A specific dose naturally varies depending on the dosage procedure, the conditions of a patient or a subject animal such as age, body weight, sex, sensitivity, feed, dosage period, drugs used in combination, seriousness of the disease. The appropriate dose and dosage times under certain conditions can be determined by the test based on the above-described indices but may be refined and ultimately decided according to the judgment of the practitioner and each patient's circumstances (age, general condition, severity of symptoms, sex, etc.) according to standard clinical techniques. As disclosed herein, an appropriate dose of an 1-aminocyclohexane derivative is generally in the range of 0.05 -1.00 mg per kg of body weight, and an appropriate dose of or an AChEI is generally in the range of 0.015 -0.57 mg per kg of the body weight.

Toxicity and therapeutic efficacy of the compositions of the invention can be determined by standard pharmaceutical procedures in experimental animals, *e.g*., by determining the LD₅₀ (the dose lethal to 50% of the population) and the ED₅₀ (the dose therapeutically effective in 50% of the population). The dose ratio between therapeutic and toxic effects is the therapeutic index and it can be expressed as the ratio ED₅₀/LD₅₀. Compositions that exhibit large therapeutic indices are preferred.

The data obtained from animal studies can be used in formulating a range of doses for use in humans. The therapeutically effective doses of 1-aminocyclohexane derivatives and AChEIs in humans lie preferably within a range of circulating concentrations that include the ED₅₀ with little or no toxicity. For example, such therapeutically effective circulating concentration for memantine is 1 µM and for tacrine (AChEI) is 8-30 nM (Roberts et al., Eur. J. Clin. Pharmacol., 1998, 54: 721-724). The dosage can vary within this range depending upon the dosage form employed and the route of administration utilized. Ideally, a single dose of each drug should be used daily.

The drug combinations of the invention are not only highly effective at relatively low doses but also possess low toxicity and produce few side effects. Indeed, the only common side effect for the AChEIs used in the invention is minor gastric irritation (reflected, *e.g*., in nausea, diarrhea, or vomiting), while the most common side effect resulting from the use of 1-aminocyclohexane derivatives in the invention is a minor motor and cognitive impairment (reflected, *e.g*., in nausea, vomiting, dizziness, or confusion).

### EXAMPLES

### A. SYNTHESIS EXAMPLES

The following Synthesis Examples are given by way of illustration only, and are not to be construed as limiting.

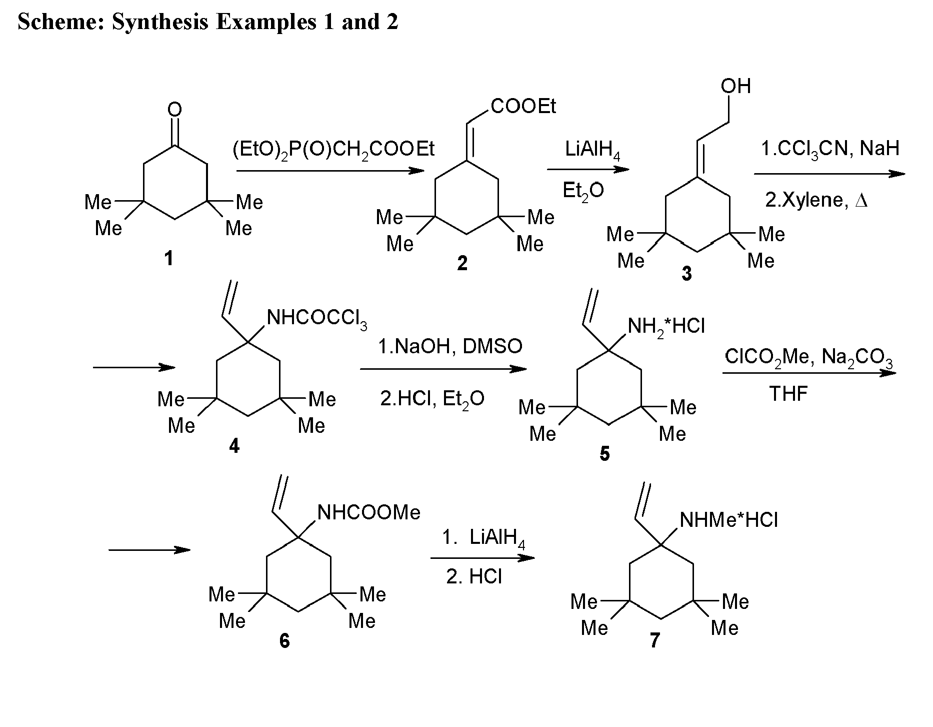

### Synthesis Example 1.

### 3,3,5,5-Tetramethyl-1-vinylcyclohexanamine hydrochloride (5).

### a) Ethyl 2-(3,3,5,5-tetramethylcyclohexylidene)acetate (2).

To a stirred solution of triethyl phosphonoacetate (49.32 g, 222 mmol) in dry THF (180 ml) under argon NaH (8.8 g, 222 mmol, 60% suspension in mineral oil) was added in small portions while cooling with ice water. Stirring was continued for 1 h at room temperature, then a solution of 3,3,5,5-tetramethylcyclohexanone (30.85 g, 200 mmol) was added over 10 min and the resulting mixture was refluxed for 22 h. It was then poured onto ice (400 g) and the product was extracted with diethyl ether (4x150 ml), and the extracts dried over MgSO₄. After solvent evaporation *in vacuo* an oily residue was distilled at 145°C (11 mm Hg) to give 36.8 g (86%) of **2** as an oil. ¹H NMR (CDCl₃, TMS) δ: 0.96 and 0.98 (total 12H, both s, 3,5-CH₃); 1.27 (3H, t, CH₃-ethyl); 1.33 (2H, m, 4-CH₂); 1.95 and 2.65 (total 4H, both s, 2,6-CH₂); 4.14 (2H, q, CH₂-ethyl) and 5.69 ppm (1H, s, =C-H).

### b) 2-(3,3,5,5-Tetramethylcyclohexylidene)ethanol (3).

To a stirred solution of LiAlH₄ (1.7 g, 45 mmol) in dry ether (60 ml) a solution of acetate **2** (3.2 g, 15 mmol) in ether (20 ml) was added dropwise while cooling with ice water. Stirring was continued for 1 h and the residual LiAlH₄ was destroyed with water. The aqueous layer was separated and twice extracted with ether (30 ml). The combined extracts were washed with brine (50 ml) and dried over MgSO₄. After concentration *in vacuo* an oily residue was purified by Kugelrohr short path distillation (150-170°C, 11 mm Hg) to give **3** (2.3 g, 89%) as an oil. ¹H NMR (CDCl₃, TMS) δ: 0.92 (6H, s, 3,5-CH₃); 1.10 (1H, br s, OH); 1.28 (2H, s, 4-CH₂); 1.87 and 1.94 (total 4H, both s, 2,6-CH₂); 4.16 (2H, d, 7 Hz, CH₂O) and 5.50 ppm (1H, t, 7 Hz, =C-H).

### c) 2,2,2-Trichloro-N-(3,3,5,5-tetramethyl-1-vinylcyclohexyl)acetamide (4).

To a solution of alcohol **3** (0.8 g, 4.7 mmol) in diethyl ether (5 ml) NaH (0.22 g of a 55% dispersion in mineral oil (0.22 mmol)) was added. The reaction mixture was cooled to -10°C and a solution of trichloroacetonitrile (0.68 g, 4.7 mmol) in diethyl ether (3 ml) was added dropwise. The solution was allowed to warm to room temperature and the solvent evaporated. Pentane (8 ml) containing methanol (0.018 ml) was added to the residue. The resulting mixture was filtered through a pad of celite and evaporated. The residual oil was dissolved in xylene (10 ml) and refluxed for 10 h. Main amount of xylene was distilled off at reduced pressure (11 mm Hg) and the residue purified by flash chromatography on silica gel (hexane, hexane - ethyl acetate, 10:1) to give **4** (0.98 g, 66 %) as an oil. ¹H NMR (CDCl₃, TMS) δ: 0.95 (6H, s, 3,5-CH₃); 1.18 (6H, s, 3,5-CH₃); 1.1-1.5 (2H, m, 4-CH₂); 1.32 (2H, d, 15 Hz, 2,6-CH₂); 2.15 (2H, d, 15 Hz, 2,6-CH₂); 5.08 (1H, d, 11 Hz, =CH₂); 5.13 (1H, d, 18 Hz, =CH₂); 5.85 (1H, dd, 18 and 11 Hz, -HC=) and 6.7 ppm (1H, br s, NH).

### d) 3,3,5,5-Tetramethyl-1-vinylcyclohexanamine hydrochloride (5).

A mixture of amide **4** (0.32 g, 1 mmol) and powdered NaOH (0.4 g, 10 mmol) in DMSO (3 ml) was stirred for 7 days at room temperature. The reaction mixture was diluted with H₂O (20 ml) and stirred overnight at room temperature. The product was extracted with hexane (3x10 ml). The combined extracts were washed with brine (20 ml), dried over NaOH and filtered through a pad of celite. To the solution obtained 4 M HCl in dry ethyl ether (0.5 ml) was added and the solvent was evaporated. The residue was treated with acetonitrile (10 ml) and the precipitate was collected on a filter and dried over P₂O₅ *in vacuo* to give **5** (0.12 g, 53%) as a colorless solid. ¹H NMR (CDCl₃, TMS) δ: 0.98 and 1.01 (total 12H, both s, 3,5-CH₃); 1.19 and 1.29 (total 2H, both d, 14 Hz, 4-CH₂); 1.62 (2H, d, 13.5 Hz, 2,6-CH₂); 1.72 (2H, br s, H₂O); 2.16 (2H, d, 13.5 Hz, 2,6 -CH₂); 5.46 and 5.73 (2H, both d, 18 and 11 Hz, =CH₂); 6.16 (1H, dd, 18 and 11 Hz, =CH) and 8.24 ppm ( 3H, br s, NH₃⁺).

### Synthesis Example 2.

### N,3,3,5,5-Pentamethyl-1-vinylcyclohexylamine hydrochloride (7).

### a) Methyl 3,3,5,5-tetramethyl-1-vinylcyclohexylcarbamate (6).

A mixture of amine hydrochloride **5** (0.25 g, 1.2 mmol) and Na₂CO₃ (0.73 g, 6.9 mmol) in THF (6 ml) was stirred at room temperature for 1 h. Methyl chloroformate (0.27 ml, 3.45 mmol) was added and the reaction mixture was stirred at room temperature for 15 h. The mixture was diluted with diethyl ether (20 ml), filtered and evaporated to the dryness. The crude product was purified by flash chromatography on silica gel (light petroleum ether - ethyl acetate, 10:1) to give **6** (0.24 g, 87%) as a colorless solid with m.p. 61-63°C. ¹H-NMR (CDCl₃, TMS) δ: 0.92 and 1.15 (total 12H, both s, 3,5-CH₃); 1.00-1.40 (4H, m, 4-CH₂ and 2,6-CH); 2.00 (2H, d, 14 Hz, 2,6-CH); 3.62 (3H, s, CH₃N); 4.72 (1H, br s, NH); 5.00 and 5.06 (total 2H, both d, 10.5 and 17 Hz, =CH₂) and 5.83 ppm (1H, dd, 10.5 and 17 Hz, =CH).

### b) N,3,3,5,5-Pentamethyl-1-vinylcyclohexylamine hydrochloride (7).

A mixture of LiAlH₄ (0.28 g, 7.4 mmol) and carbamate **6** (0.22 g, 0.92 mmol) in THF (22 ml) was refluxed for 12 h. Then it was cooled in an ice bath and water (20 ml) was added dropwise. The resulting suspension was extracted with hexane (3x20 ml) and the combined extracts were washed with brine (20 ml). The extract was dried over NaOH, filtered and treated with 2.4 M HCl solution in diethyl ether (1 ml). The resulting suspension was evaporated to the dryness. The residue was treated with diethyl ether (10 ml) and acetonitrile (1 ml). The precipitate was collected on a filter and dried in *vacuo* over P₂O₅ to give **7** (0.11 g, 52%) as a colorless solid. ¹H-NMR (CDCl₃, TMS) δ: 1.00 and 1.02 (total 12H, both s, 3,5-CH₃); 1.23 and 1.32 (total 2H, both d, 15 Hz, 4-CH₂); 1.72 (2H, d, 13 Hz, 2,6 - CH); 2.15 (2H, d, 13 Hz, 2,6 - CH); 2.45 (3H, t, 5 Hz, CH₃N); 5.64 and 5.69 (total 2H, both d, 11 and 17 Hz, =CH₂); 5.98 (1H, dd, 11 and 17 Hz, =CH) and 9.30 ppm (2H, br s, NH₃⁺).

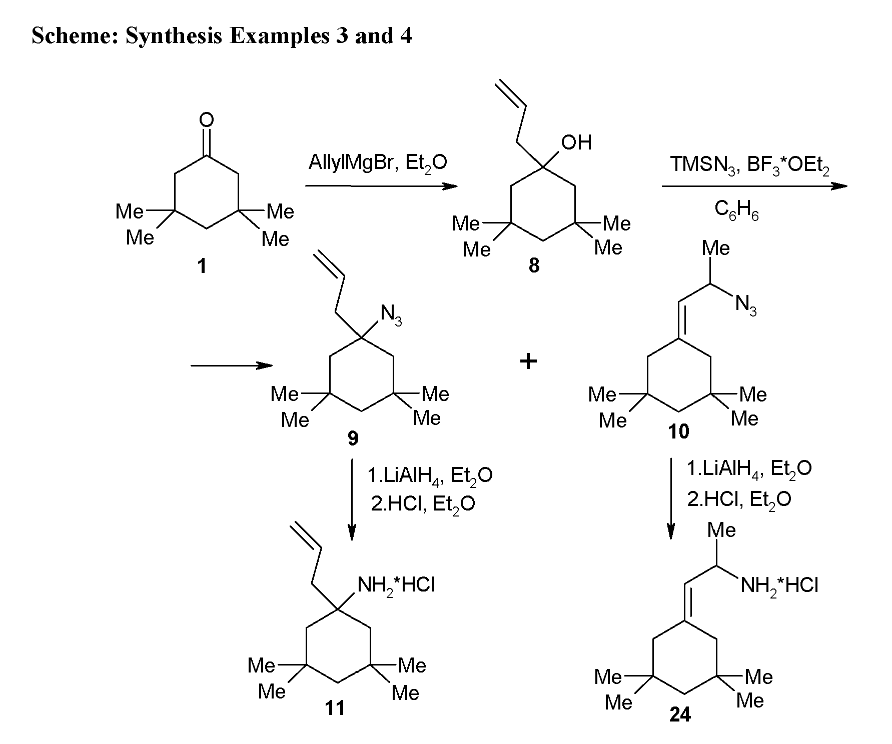

### Synthesis Example 3.

### 1-Allyl-3,3,5,5-tetramethylcyclohexanamine hydrochloride (11).

### a) 1-Allyl-3,3,5,5-tetramethylcyclohexanol (8).

To a stirred 1 M etheral solution of allyllmagnesium bromide (60 ml, 60 mmol) was added dropwise a solution of 3,3,5,5-tetramethylcyclohexanone (3.86 g, 25 mmol) in dry ether (20 ml). The mixture was stirred for 1 h at ambient temperature and boiled at reflux for 10 min. Then it was cooled with ice water and carefully treated with saturated aqueous NH₄Cl (40 ml). The organic layer was separated and washed with water and brine. After drying over anhydrous MgSO₄, the solution was concentrated *in vacuo.* The residue was fractionally distilled at reduced pressure to give 3.5 g (72%) of **8** with b.p. 98-100°C/12 mm Hg. ¹H NMR (CDCl₃, TMS) δ: 0.88 (6H, s, 3,5-CH_{3eq}); 1.20 (6H, s, 3,5-CH₃ₐₓ); 0.95-1.60 (6H, m, 2,4,6-CH₂); 2.15 (2H, d, 7.5 Hz, CH₂C=); 4.95-5.30 (2H, m, =CH₂) and 5.65-6.20 ppm (1H, m, =CH).

### b) 1-Allyl-1-azido-3,3,5,5-tetramethylcyclohexane (9) and 1-Methyl-2-(3,3,5,5-tetramethyl-cyclohexylidene)ethyl azide (10).

To a solution of cyclohexanol **8** (1.96 g, 10 mmol) in dry benzene (20 ml) under argon was added azidotrimethylsilane (12 mmol). To this cooled (5°C) solution was slowly added BF₃*OEt₂ (12 mmol) via syringe within 20 min. The mixture was stirred for 6 h, then water was slowly added. The organic layer was separated and washed with saturated aqueous NaHCO₃, and with brine, and dried over MgSO₄. Filtration and evaporation of the solvent keeping the temperature below 25°C gave an oil which was separated by column chromatography on silica gel (light petroleum ether). A fraction with Rf 0.85 (hexane) was collected. Evaporation of the solvent provided **9** as a colorless oil (0.26 g, 11.7%). ¹H NMR (CDCl₃, TMS) δ: 0,89 (6H, s, 3,5-CH_{3eq}); 0.90 (1H, d, 14 Hz, 4-CHₐₓ); 1.05 (2H, d, 14 Hz, 2,6-CHₐₓ); 1.18 (6H, s, 3,5-CH₃ₐₓ); 1.37 (1H, d, 14 Hz, 4-CH_{eq}); 1.60 (2H, d, 14 Hz, 2,6-CH_{eq}), 2.29 (2H, d, 7 Hz, CH₂C=); 4.95-5.25 (2H, m, =CH₂) and 5.65-6.15 ppm(1H, m, =CH). Evaporation of additional fraction (Rf 0.65 (hexane)) gave 0.425 g (20.3%) of azide **10** as a colorless oil. ¹H NMR (CDCl₃, TMS) δ: 0.91 (6H, s), 0.94 (3H, s) and 0.96 (3H, s, 3',5'-CH₃); 1.23 (3H, d, 6.5 Hz, 1-CH₃); 1.26 (2H, s, 4'-CH₂);1.89 (2H, s) and 1.96 (2H, s, 2',6'-CH₂); 4.31(1H, dq, 6.5 and 9.5 Hz, 1-CH) and 5.21 ppm (1H, dm, 9.5 Hz, =CH).

### c) 1-Allyl-3,3,5,5-tetramethylcyclohexanamine hydrochloride (11).

A solution of azide **9** (0.221 g, 1.0 mmol) in dry ether (4 ml) was added dropwise to a stirred suspension of lithium aluminum hydride (0.152 g , 4 mmol) in ether (10 ml) within 10 min. The mixture was stirred for 4 h, then it was treated with 20% aqueous NaOH (8 ml). The aqueous layer was separated and extracted with diethyl ether (2x15 ml). The combined organic extracts were washed with brine and dried over NaOH. The filtered solution was treated with dry HCl solution in diethyl ether and evaporated. Dry diethyl ether was added to the solid residue and it was collected on filter, and washed with dry ether to give **11** (0.105 g, 47%) as a colorless solid. ¹H NMR (CDCl₃, TMS) δ: 1.03 (6H, s, 3,5-CH_{3eq}); 1.06 (6H, s, 3,5-CH₃ₐₓ); 1.29 (2H, s, 4-CH₂); 1.63 (2H, d, 13 Hz, 2,6 - CHₐₓ); 1.80 (2H, d, 13 Hz, 2,6 - CH_{eq}), 2.71 (2H, d, 7 Hz, CH₂C=); 5.10-5.40 (2H, m, =CH₂); 5.75-6.25 (1H, m, =CH) and 8.25 ppm (3H, br s, NH₃⁺).

### Synthesis Example 4.

### 1-(3,3,5,5-Tetramethylcyclohexylidene hydrochloride (24).

A solution of 1-methyl-2-(3,3,5,5-tetramethylcyclohexylidene)ethyl azide (**10**) (0.33 g, 1.5 mmol) in dry diethyl ether (4 ml) was added dropwise to a stirred suspension of lithium aluminum hydride (0.152 g , 4 mmol) in ether (15 ml) within 10 min. The mixture was stirred for 4 h, then it was treated with 20% aqueous NaOH (8 ml). The aqueous layer was extracted with ether (2x15 ml). The organic extracts were combined, washed with brine and dried over NaOH. The filtered solution was treated with dry HCl solution in ether and evaporated *in vacuo.* Dry ether was added to the solid residue and it was collected on filter and washed with dry ether to give **24** (0.18 g, 54%) as a colorless solid. ¹H NMR (CDCl₃, TMS) δ: 0.89 (6H, s), 0.92 (3H, s) and 0.98 (3H, s, 3',5'-CH₃); 1.27 (2H, s, 4'-CH₂); 1.47 (3H, d, 6.5 Hz, 3-CH₃); 1.84 (1H, d, 13.5 Hz, 2'-CH); 1.87 (2H, s, 6'-CH₂), 2.06 (1H, d, 13.5 Hz, 2'-CH); 4.17 (1H, dq, 6.5 and 9.5 Hz, 2-CH); 5.35 (1H, d, 9.5 Hz, =CH) and 8.25 ppm (3H, br s, NH ₃⁺).

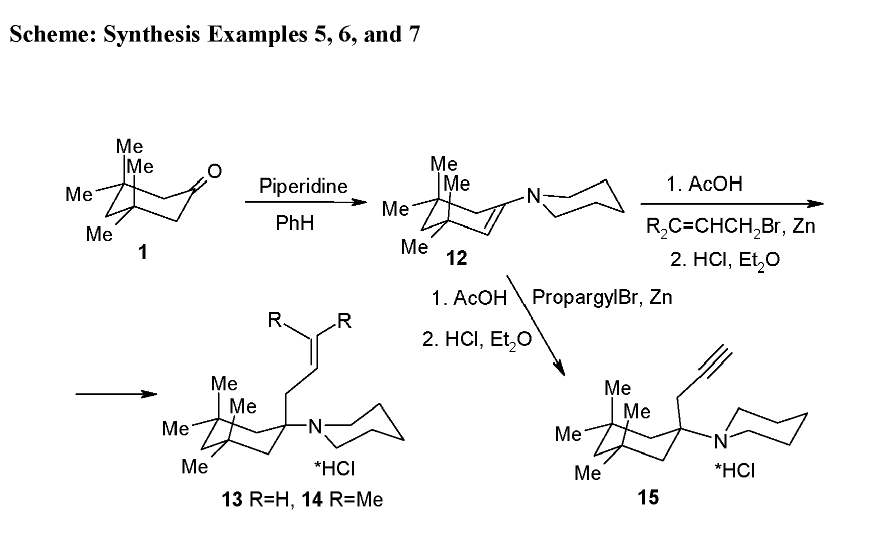

### Synthesis Example 5.

### 1-(1-Allyl-3,3,5,5-tetramethylcyclohexyl)piperidine hydrochloride (13).

### a) 1-(3,3,5,5-Tetramethyl-1-cyclohexenyl-1)piperidine (12).

Prepared by condensation of piperidine (1.2 equivalents) and 3,3,5,5 - tetramethylcyclohexanone by heating in benzene with azeotropic removal of water. Crude product was obtained by removing starting materials at vacuum distillation conditions (100°C/ 10 mm Hg). Amber oil. ¹H NMR (CDCl₃, TMS) δ: 0.94 (6H, s) and 0.97 (6H, s, 3',5'- CH₃); 1.25 (2H, s, 4'- CH₂); 1.40-1.70 (6H, m, piperidine 3,4,5 - CH₂); 1.76 (2H, s, 6'- CH₂); 2.60-2.85 (4H, m, piperidine 2,6-CH₂) and 4.40 ppm (1H, s, =CH).

### b) 1-(1-Allyl-3,3,5,5-tetramethylcyclohexyl)piperidine hydrochloride (13).

To a solution of enamine **12** (2.1 g, 9 mmol) in THF (20 ml) was added acetic acid 0.675 g, 11.25 mmol). The mixture was stirred for 5 min and zinc powder (0.74 g, 11.25 mgA) was added. Then a solution of allylbromide (1.63 g, 13.5 mmol) in THF (5 ml) was added dropwise and the mixture was stirred at ambient temperature for 6 h. Aqueous Na₂CO₃ was added and the resulting mixture was extracted with ether. The extract was washed with brine, dried over anhydrous MgSO₄, and concentrated *in vacuo.* The residue was separated by column chromatography on silica gel (hexane, 5% EtOAc in hexane). The fraction with Rf 0. 85 (hexane-EtOAc, 13:2) was collected, evaporated and treated with dry HCl solution in ether. The precipitate was filtered and washed with hexane-EtOAc mixture to give **13** (0.79 g, 29%) as a colorless solid. ¹H NMR (CDCl₃, TMS) δ: 1.07 (6H, s, 3',5'-CH_{3eq}), 1.10 (6H, s, 3',5-CH₃ₐₓ); 1.34 (1H, d, 12.2 Hz) and 1.45 (1H, d, 12.2 Hz, 4'-CH₂); 1.70-1.95 (6H, m, 2',6'-CHₐₓ and piperidine 3,5-CH, 4-CH₂,); 2.37 (2H, d, 13.4 Hz, 2',6'- CH_{eq}); 2.40-2.70 (2H, m, piperidine 3,5-CH); 2.76 (2H, d, 7.2 Hz, CH₂ C=); 2.75-3.00 (2H, m, piperidine 2,6-CH); 3.64 (2H, d, 11.6 Hz, piperidine 2,6 -CH); 5.13 (1H, d, 9.6 Hz) and 5.24 (1H, d, 17.8 Hz, =CH₂); 5.85-6.15 (1H, m, =CH) and 10.72 ppm (1H, br s, NH).

### Synthesis Example 6.

### 1-[3,3,5,5-Tetramethyl-1-(3-methyl-2-butenyl)cyclohexyl]piperidine hydrochloride (14).

Prepared from piperidine **12** according to the procedure for compound **13 (Synthesis Example 5, b)** using 4-bromo-2-methyl-2-butene instead of allylbromide. Yield: 20%. ¹H NMR (CDCl₃, TMS) δ: 1.07 and 1.08 (total 12H, both s, 3',5'- CH₃), 1.32 and 1.44 (2H, both d, 14.2 Hz, 4'- CH₂); 1.69 and 1.76 (6H, both s, =C(CH₃)₂); 1.68-1.96 (4H, m, 3,5 - CH and 4 - CH₂,); 1.84 (2H, d, 13.4 Hz, , 2',6'-CHₐₓ)_{;} 2.31 (2H, d, 13.4 Hz, , 2',6'-CH_{eq}); 2.40-2.80 (4H, m, N(CH)₂, 3,5-CH); 2.60 (2H, d, 7.2 Hz, CH₂C=); 3.63 (2H, d, 10.4 Hz, N(CH)₂); 5.31 (1H, t, 6.8 Hz, =CH) and 10.55 ppm (1H, br s, NH).

### Synthesis Example 7.

### 1-[3,3,5,5-Tetramethyl-1-(2-propynyl)cyclohexyl]piperidine hydrochloride (15).

Prepared from piperidine **12** according to the procedure for compound **13 (Synthesis Example 5, b)** using 3-bromopropyne instead of allylbromide. Yield: 6%. ¹H NMR (CDCl₃, TMS) δ: 1.07 (6H, s, 3',5'-CH_{3eq}), 1.11 (6H, s, 3',5'-CH₃ₐₓ); 1.23 and 1.44 (total 2H, both d, 14.3 Hz, 4'-CH₂); 1.75 - 2.00 (4H, m, piperidine 3,5-CH, 4-CH₂,); 1.91 (2H, d, 13.2 Hz, 2',6'-CH ₐₓ); 2.28 (1H, s, HCC); 2.34 (2H, d, 13.2 Hz, 2',6'- CH_{eq}); 2.40 - 2.70 (2H, m, piperidine 3,5-CH); 2.81 (2H, s, CH₂ CC); 2.85-3.10 (2H, m, piperidine 2,6-CH); 3.69 (2H, d, 10.2 Hz, piperidine 2,6- CH) and 11.12 ppm (1H, br s, NH).

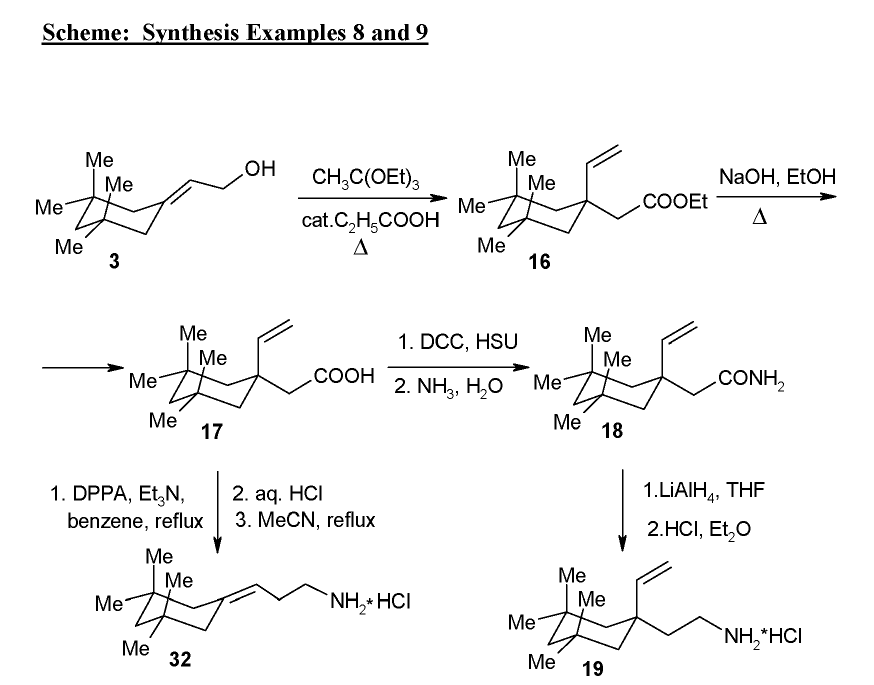

### Synthesis Example 8.

### 2-(3,3,5,5-Tetramethyl-1-vinylcyclohexyl)ethanamine hydrochloride (19).

### a) Ethyl 2-(3,3,5,5-tetramethyl-1-vinylcyclohexyl)acetate (16).

A mixture of triethyl orthoacetate (18.6 ml, 102 mmol), 2 - (3,3,5,5-tetramethyl-cyclohexylidene)ethanol **(3)** (4.63 g, 25.4 mmol) and propionic acid (0.19 ml, 2.5 mmol) was heated at 145 °C for 10 h. Ethanol was distilled off from the mixture in the course of reaction. The reaction mixture was cooled and poured into water (100 ml). The aqueous phase was extracted with hexane (2x50 ml) and the combined organic phases were washed with 5% aqueous KHSO₄ (50 ml) and brine (50 ml). The extract was dried over MgSO₄, filtered and evaporated. The residue was purified by flash chromatography on silica gel (light petroleum ether and light petroleum ether - ethyl acetate, 100:2) to give **16** (4.64 g, 73 %) as an oil. ¹H-NMR (CDCl₃, TMS) δ: 0.91 (6H, s, 3,5-CH₃); 1.01 (6H, s, 3,5-CH₃); 1.23 (3H, t, 7 Hz, ethyl CH₃); 1.00-1.30 (4H, m, 4-CH₂ and 2,6 - CH); 1.86 (2H, d, 13 Hz, 2,6-CH); 2.22 (2H, s, CH ₂C=O); 4.08 (2H, q, 7 Hz, ethyl CH₂); 5.06 and 5.07 (total 2H, both d, 11 and 17.5 Hz, =CH₂) and 5.95 ppm (1H, dd, 11 and 17.5 Hz, -CH=).

### b) 2-(3,3,5,5-Tetramethyl-1-vinylcyclohexyl)acetic acid (17).

A solution of NaOH (1.03 g, 25.8 mmol) and acetate **16** (1.3 g, 5.15 mmol) in methanol (26 ml) was refluxed for 3 h. The mixture was cooled to room temperature and poured into water (100 ml). The aqueous phase was acidified by conc. aqueous HCl and extracted with hexane (3x30 ml). The combined organic phases were washed with brine and dried over CaCl ₂, filtered and evaporated. The residue was purified by flash chromatography on silica gel (light petroleum ether - ethyl acetate, 10:1) to give **17** (0.7 g, 71%) as a colorless solid with m.p. 92-94 °C. ¹H-NMR (CDCl₃, TMS) δ: 0.92 (6H, s, 3,5-CH₃); 1.02 (6H, s, 3,5 - CH₃); 1.00-1.30 (4H, m, 4-CH₂ and 2,6-CH); 1.90 (2H, d, 14 Hz, 2,6 -CH); 2.27 (2H, s, CH₂ C=O); 5.11 and 5.13 (total 2H, both d, 11 and 18 Hz, =CH₂); 5.99 (1H, dd, 18 and 11 Hz, =CH) and 10.80 ppm (1H, br s, COOH). **c) 2-(3,3,5,5-Tetramethyl-1-vinylcyclohexyl)acetamide (18).**

N-Hydroxysuccinimide (0.25 g, 2.2 mmol) and N,N'-dicyclohexyl carbodiimide (0.45, 2.2 mmol) was added to a solution of cyclohexylacetic acid **17** (0.45 g, 2 mmol) in THF (5 ml). The mixture was stirred for 18 h at room temperature and cooled in an ice bath. 25% aqueous NH₄OH (2 ml) was added in one portion and the mixture was stirred at room temperature for 2 h. The precipitate was filtered off and washed with diethyl ether (30 ml). The organic phase of filtrate was separated and washed with 5% aqueous KHSO₄ (10 ml) and brine. The extract was dried over MgSO₄, filtered and evaporated. The residue was purified by flash chromatography on silica gel (light petroleum ether - ethyl acetate, 4:1 to 1:1) to give **18** (0.34 g, 76%) as a colorless solid with m.p. 44 - 46°C. ¹H-NMR (CDCl₃, TMS) δ: 0.91 (6H, s, 3,5-CH₃); 1.02 (6H, s, 3,5-CH₃); 1.00 -1.30 (4H, m, 4-CH₂ and 2,6 -CH); 1.85 (2H, d, 14 Hz, 2,6-CH); 2.13 (2H, s, CH₂C=O); 5.18 and 5.19 (total 2H, both d, 18 and 11 Hz, =CH₂); 5.40 and 5.60 (total 2H, both br s, NH₂) and 6.03 ppm (1H, dd, 18 and 11 Hz, =CH).

### d) 2-(3,3,5,5-Tetramethyl-1-vinylcyclohexyl)ethanamine hydrochloride (19).

A mixture of LiAlH₄ (0.41 g, 11 mmol) and amide **18** (0.30 g, 1.4 mmol) in THF (18 ml) was refluxed for 17 h. Then it was cooled in an ice bath and water (30 ml) was added dropwise. The resulting suspension was extracted with hexane (3x30 ml) and the combined organic phases were washed with brine. The extract was dried over NaOH, filtered and concentrated to ∼10 ml volume. 4.8 M HCl solution in diethyl ether (1 ml) was added and the resulting suspension was evaporated to dryness. The residue was treated with acetonitrile (5 ml) and the precipitate was collected on filter and dried *in vacuo* over NaOH to give **19** (0.16 g, 50%) as a colorless solid. ¹H-NMR (CDCl₃, TMS) δ: 0.89 (6H, s, 3,5-CH₃); 1.02 (6H, s, 3,5 - CH₃); 0.90-1.80 (8H, m, ring protons and ethanamine - 2-CH₂); 2.92 (2H, br s, CH₂N); 5.05 and 5.15 (2H, both d, 18 and 11 Hz, =CH₂); 5.77 (1H, dd, 18 and 11Hz), =CH) and 8.10 ppm (3H, br s, NH₃⁺ ).

### Synthesis Example 9.

### 3-(3,3,5,5-Tetramethylcyclohexylidene)propanamine hydrochloride (32).

Triethylamine (0.25 ml, 1.76 mmol) and diphenylphosphoryl azide (0.38 ml, 1.76 mmol) were added to a solution of acid **17** (0.36 g, 1.6 mmol) in benzene (6 ml). The mixture was refluxed for 2 h, cooled to room temperature and evaporated to dryness. Cold (∼5°C) conc. aqueous HCl (3 ml) was added to the residue. The resulting mixture was stirred at room temperature for 18 h and made strongly alkaline by addition of 10% aqueous NaOH. Hexane (20 ml) was added to the mixture and both phases filtered. The precipitate was washed with hexane (2x5 ml) and water (2x5 ml). The organic phase of the filtrate was separated. The aqueous phase was washed with hexane (2x10 ml). The combined organic phases were washed with brine (10 ml), dried over NaOH and filtered. 4.8 M HCl solution in diethyl ether (1 ml) was added and the resulting suspension was evaporated. The residue was recrystallized from acetonitrile and dried *in vacuo* over P₂O₅ to give **32** (0.1 g, 43%) as a colorless solid. ¹H-NMR:
(CDCl₃, TMS) δ: 0.90 and 0.92 (total 12H, both s, c-Hex-3,5-CH₃); 1.23 (2H, s, c -Hex-4-CH₂); 1.86 and 1.92 (total 4H, both s, c-Hex-2,6 -CH₂); 2.49 (2H, q, 7 Hz, propanamine - 2-CH₂); 2.98 (2H, t, 7 Hz, propanamine - 1- CH₂); 5.15 (1H, t, 7 Hz, =CH-) and 8.30 ppm (3H, br s, NH₃⁺).

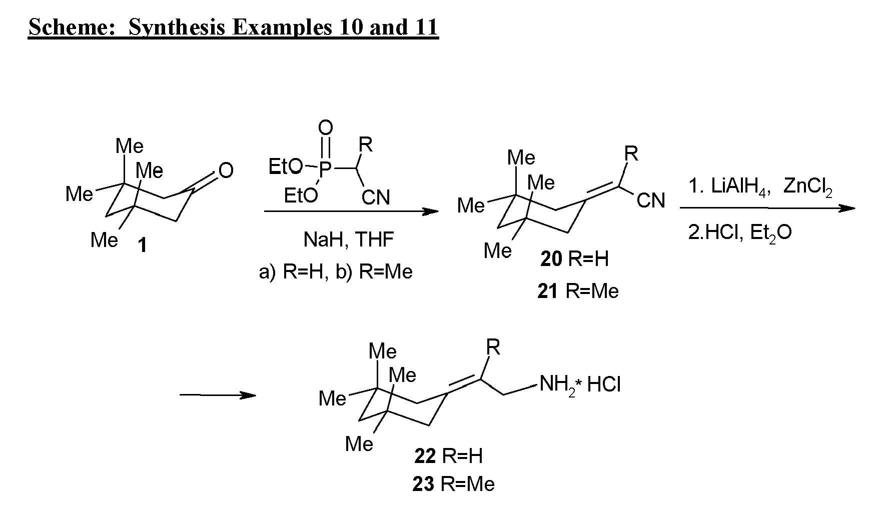

### Synthesis Example 10.

### 2-(3,3,5,5-Tetramethylcyclohexylidene)ethanamine hydrochloride (22).

### a) 3,3,5,5-Tetramethylcyclohexylideneacetonitrile (20).

60% NaH dispersion in mineral oil (0.96 g, 24 mmol) was added to a solution of diethyl cyanomethylphosphonate (4.25 g, 24 mmol) in THF (30 ml) while cooling with ice water. The mixture was stirred for 30 min and a solution of 3,3,5,5 - tetramethylcyclohexanone (3.08 g, 20 mmol) in THF (10 ml) was added dropwise. Cooling bath was removed and the mixture was stirred at room temperature for 72 h. It was poured into ice water (100 ml) and extracted with diethyl ether (3x50 ml). The combined organic phases were washed with brine, dried over MgSO₄, filtered and evaporated. The crude product was purified by flash chromatography on silica gel (light petroleum ether - ethyl acetate, 10:1) to give **20** (2.38 g, 71 %) as a colorless oil. ¹H-NMR (CDCl₃, TMS) δ: 0.97 and 1.01 (total 12H, both s, 3',5'-CH₃); 1.36 (2H, s, 4'-CH₂); 2.01 (2H, s, 2'-CH₂); 2.26 (2H, s, 6'-CH₂) and 5.14 ppm (1H, s, =CH).

### b) 2-(3,3,5,5-Tetramethylcyclohexylidene)ethanamine hydrochloride (22).

A suspension of LiAlH₄ (0.68 g, 18 mmol) in diethyl ether (30 ml) was cooled in an ice bath and 1M ZnCl₂ solution in diethyl ether (9 ml, 9 mmol) was added. The resulting mixture was stirred for 15 min and a solution of nitrile **20** (1 g, 6 mmol) in diethyl ether (30 ml) was added dropwise keeping the temperature at 0 - 5°C. Ice bath was then removed and the mixture was stirred at room temperature for 24 h. Water (30 ml) and 20% aqueous NaOH (20 ml) was added while cooling with an ice bath. The aqueous phase was extracted with diethyl ether (4x50 ml). The combined organic phases were washed with brine (50 ml) and dried over NaOH, filtered and evaporated. The residue was purified by Kugelrohr short path distillation at 160 ° C/ 20 mm Hg. The distillate was diluted with diethyl ether and 4. 8M HCl solution in diethyl ether (3 ml) was added. The resulting precipitate was collected on a filter, washed with diethyl ether (3x5 ml) and dried *in vacuo* over NaOH to give **22** as a colorless solid. ¹H-NMR (CDCl₃, TMS) δ: 0.91 and 0.92 (total 12H, both s, 3',5'-CH₃); 1.28 (2H, s, 4'-CH₂); 1.89 and 1.93 (total 4H, both s, 2',6'-CH₂); 3.62 (2H, d, 7 Hz, CH₂N); 5.41 (1H, t, 7 Hz, -C=CH) and 8.3 ppm (3H, br s, NH₃⁺).

### Synthesis Example 11.

### 2-(3,3,5,5-Tetramethylcyclohexylidene)nronanamine hydrochloride (23).

### a) 2-(3,3,5,5-Tetramethylcyclohexylidene)propionitrile (21).

Prepared according to the procedure for compound **20** (Synthesis **Example 10, a)** using diethyl (1-cyanoethyl)phosphonate. Nitrile **21** obtained as a colorless oil with 41% yield. ¹H-NMR: (CDCl₃, TMS) δ: 0.96 and 1.00 (total 12H, both s, c-Hex-3,5-CH₃); 1.34 (2H, s, c -Hex-4-CH₂); 1.91 (3H, s, propionitrile -3-CH₃); 2.04 and 2.28 ppm (total 4H, both s, c -Hex-2,6-CH₂).

### b) 2-(3,3,5,5-Tetramethylcyclohexylidene)propanamine hydrochloride (23).

Prepared from nitrile **21** according to the procedure for compound **22 (Synthesis Example 10, b).** Amine hydrochloride **23** obtained as a colorless solid. ¹H -NMR: (CDCl₃, TMS) δ: 0.92 and 0.93 (total 12H, both s, c-Hex-3,5-CH₃); 1.27 (2H, s, c-Hex-4-CH₂); 1.89 (3H, s, propanamine-3-CH₃); 1.99 and 2.01 (total 4H, both s, c - Hex-2,6-CH₂); 3.64 (2H, br s, propanamine -1-CH₂) and 8.40 ppm (3H, br s, NH₃⁺).

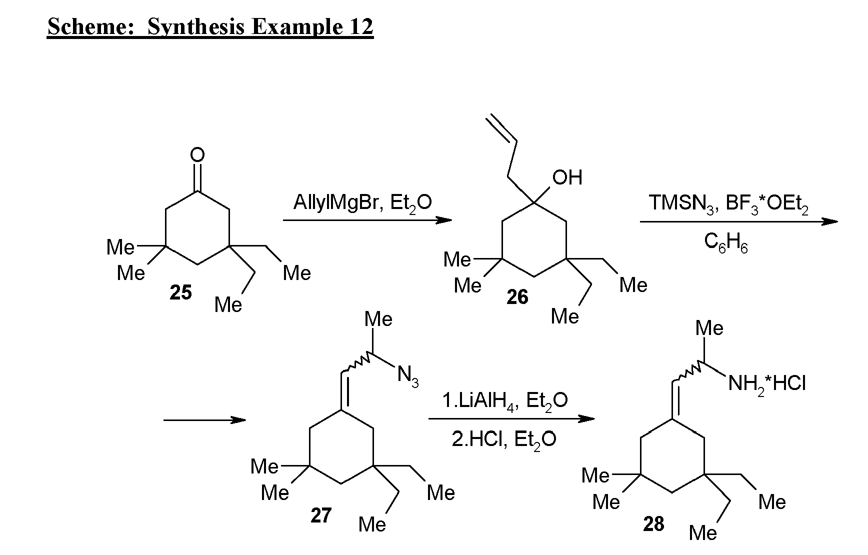

**Synthesis Example 12.**

### (E,Z)-1-(3,3-Diethyl-5,5-dimethylcyclohexylidene)-2-propanamine hydrochloride (28).

### a) 1-Allyl-3,3-diethyl-5,5-dimethylcyclohexanol (26).

To a stirred 1 M ethereal solution of allylmagnesium bromide (20 ml, 20 mmol) was added dropwise a solution of 3,3 -diethyl-5,5-dimethylcyclohexanone (25) (1.47 g, 8.06 mmol) in dry ether (5 ml). The mixture was stirred for 1 h at ambient temperature and boiled at reflux for 10 min. Then it was cooled with ice water and treated with saturated aqueous NH₄Cl (40 ml). The organic layer was separated and washed with water and brine. After drying over anhydrous MgSO₄, the solution was concentrated *in vacuo.* The residue was purified by column chromatography on silica gel (light petroleum ether). A fraction with Rf 0.7 (Hexane : EtOAc, 13:2) was collected. Evaporation of the solvent afforded **26** (1.35 g, 74%) as a colorless oil. ¹H NMR (CDCl₃, TMS) δ: 0.74 (6H, t, 7 Hz, 2CH₃ of ethyl); 0.88 (3H, s, 5-CH_{3eq}); 1.19 (3H, s, 5-CH₃ₐₓ); 0.80-2.05 (10H, m, 2,4,6-CH₂ and 2CH₂ of ethyl); 2.14 (2H, d, 7 Hz, CH₂C=); 4.95-5.30 (2H, m, =CH₂) and 5.65-6.20 ppm (1H, m, =CH). **b) (E,Z)-1-Methyl-2-(3,3-diethyl-5,5-dimethylcyclohexylidene)ethyl azide (27).**

Prepared from cyclohexanol **26** according to the procedure for compounds **9** and 10 **(Synthesis Example 3, b).** Azide **27** obtained as a colorless oil with 15% yield. ¹H NMR (CDCl₃, TMS) δ: 0.73 and 0.74 (total 6H, both t, 7 Hz, 2CH₃ ethyl); 0.91, 0.94 and 0.97 (total 6H, all s, 5',5'-CH₃); 1.10-1.45 (4H, m, 2CH₂ ethyl); 1.22 (3H, d, 6.5 Hz, 1-CH₃); 1.26 (2H, s, 4'-CH₂); 1.89 (2H, s) and 1.97 (2H, m, 2',6'-CH₂); 4.08-4.48 (1H, m, 1-CH) and 5.18 ppm (1H, dm, 9.5 Hz, =CH).

### c) (E,Z)-1-(3,3-Diethyl-5,5-dimethylcyclohexylidene)-2-propanamine hydrochloride (28).

Prepared from azide **27** according to the procedure for compound **24 (Synthesis Example 4).** Amine hydrochloride **28** obtained as a colorless solid in 16% yield. ¹H NMR (CDCl₃, TMS) δ: 0.72 (6H, br t, 7 Hz, 2CH₃ ethyl), 0.90, 0.92 and 0.98 (total 6H, all s, 5',5'-CH₃); 1.25 (6H, m, 4'-CH₂ and 2CH₂ ethyl); 1.47 (3H, d, 6.5 Hz, 2-CH₃); 1.70-2.25 (2H, br AB q, 13 Hz, 2'-CH₂); 1.87 (2H, s, 6'-CH₂), 4.18 (1H, m, 2-CH); 5.34 (1H, br d, 9.5 Hz, =CH) and 8.38 ppm (3H, br s, NH₃⁺).

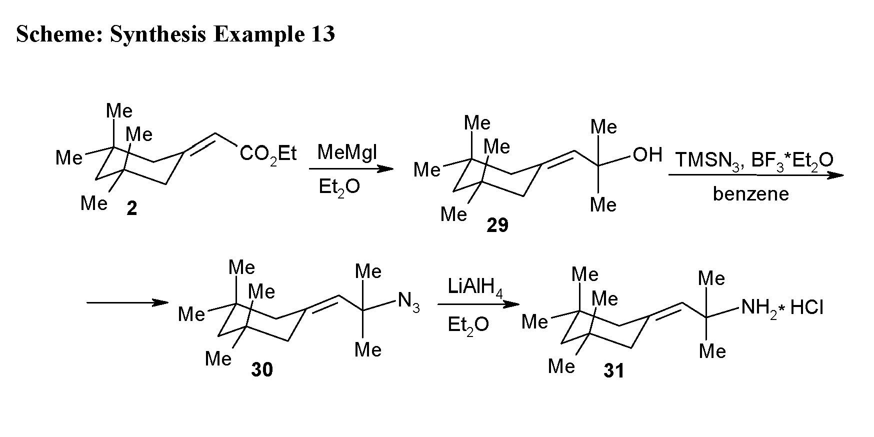

**Synthesis Example 13.**

### 2-Methyl-1-(3,3,5,5-tetramethylcyclohexylidene)-2-propanamine hydrochloride (31).

### a) 2-Methyl-1-(3,3,5,5-tetramethylcyclohexylidene)-2-propanol (29).

A solution of acetate **2** (2.14 g, 10 mmol) in diethyl ether (20 ml) was added to 1.6 M MeLi solution in diethyl ether (26 ml, 40 mmol), while cooling in an ice bath. The reaction mixture was stirred at room temperature for 1 h. It was then cooled in an ice bath and saturated aqueous NH₄ Cl (20 ml) was added dropwise. The aqueous phase was extracted with diethyl ether (2x30 ml). The combined organic phases were washed with brine (30 ml), dried over MgSO₄, filtered and evaporated. The residue was purified by Kugelrohr short path distillation (100° C/ 4 mm Hg) to give **29** (1.86 g, 86%) as a colorless oil. ¹H-NMR: (CDCl₃, TMS) δ: 0.91 and 0.96 (total 12H, both s, c-Hex-3,5-CH₃); 1.25 (2H, s, c -Hex- 4-CH₂); 1.38 (6H, s, - C(CH₃)₂O); 1.79 and 2.23 (both 2H, both s, c-Hex- 2,6-CH₂) and 5.39 ppm (1H, s, =CH -).

### b) 2-Azido-2-methyl-1-(3,3,5,5-tetramethylcyclohexylidene)propane (30).

BF₃Et₂O (0.3 ml, 2.4 mmol) was added to a solution of alcohol **29** (0.42 g, 2 mmol) and TMSN₃ (0.31 ml, 2.4 mmol) in benzene (4.5 ml) during 3 min, while cooling with an ice bath. The reaction mixture was stirred at 5-10°C for 1 h and filtered through a short silica gel column. The solution was evaporated and the residue was purified by flash chromatography on silica gel (light petroleum ether) to give **30** (0.30 g, 64%) as a colorless oil. ¹H-NMR (CDCl₃, TMS) δ: 0.92 and 0.98 (total 12H, both s, c-Hex-3,5-CH₃); 1.27 (2H, s, c -Hex- 4-CH₂); 1.40 (6H, s, -C(CH₃)₂N₃); 1.85 and 2.23 (both 2H, both s, c-Hex- 2,6-CH₂) and 5.27 ppm (1H, s, =CH -).

### c) 2-Methyl-1-(3,3,5,5-tetramethylcyclohexylidene)-2-propanamine hydrochloride (31).

Prepared from azide **30** by the same procedure as for amine **24 (Synthesis Example 4).** Amine hydrochloride **31** obtained as a colorless solid in 69% yield. ¹H-NMR (CDCl₃, TMS) δ: 0.91 and 0.98 (total 12H, both s, c-Hex-3,5-CH₃); 1.26 (2H, s, c-Hex- 4 -CH₂); 1.68 (6H, s, -C(CH₃)₂N); 1.84 and 2.10 (both 2H, both s, c -Hex- 2,6-CH₂); 5.15 (1H, s, =CH-) and 8.5 ppm (3H, br s, NH₃⁺).

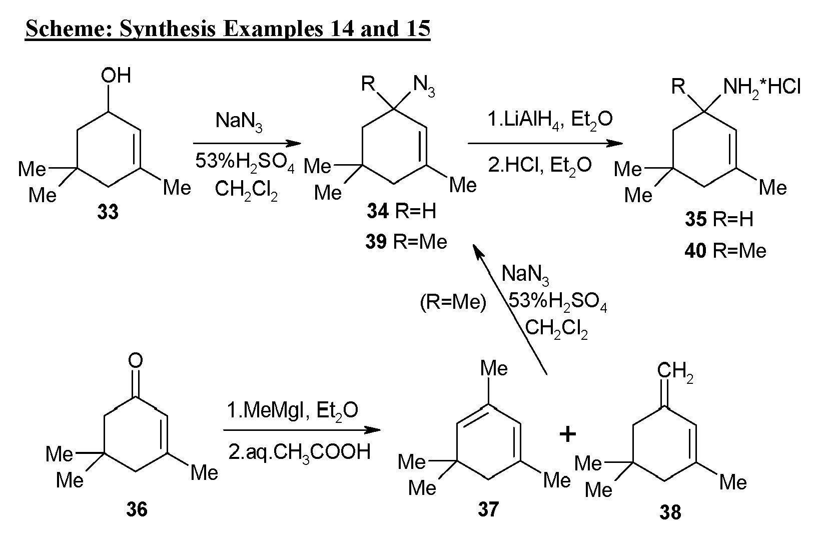

### Synthesis Example 14.

### 3,5,5-trimethyl-2-cyclohexen-1-amine hydrochloride (35).

### a) 3-Azido-1,5,5-trimethyl-1-cyclohexene (34).

To a cooled (0°C) suspension of sodium azide (0.81 g, 12.5 mmol) in CH₂Cl₂ (5 ml) was added dropwise 53% aqueous H₂ SO₄ (8 ml). The mixture was stirred for 10 min, then a solution of 3,5,5 -trimethyl-2-cyclohexanol **(33)** (0.70 g, 5 mmol) in CH₂Cl₂ (8 ml) was added. The mixture was stirred for 20 h, poured into ice water, neutralized with aqueous NH₄OH and extracted with CH₂Cl₂. The extract was washed with brine and dried over MgSO₄. Filtration and evaporation of the solvent keeping the temperature below 25°C gave an oil which was separated by column chromatography on silica gel (light petroleum ether). A fraction with Rf 0.8 (hexane) was collected. Evaporation of the solvent gave **34** as a colorless oil (0.365 g, 44%). ¹H NMR (CDCl₃, TMS) δ: 0.89 and 1.01 (total 6H, both s, 5,5-CH₃); 1.34 (1H, m, c-4-CH); 1.55-1.95 (3H, m, 4-CH, 6-CH₂); 1.71 (3H, s, 1-CH₃); 3.90 (1H, m, 3-CH) and 5.39 ppm (1H, s, C=CH).

### b) 3,5,5-trimethyl-2-cyclohexen-1-amine hydrochloride (35).

Prepared from azide **34** according to the procedure for compound **11 (Synthesis Example 3, c).** Amine hydrochloride **35** obtained as a colorless solid in 57% yield. ¹H NMR (CDCl₃, TMS) δ: 0.89 and 1.03 (total 6H, both s, 5,5-CH₃); 1.25-2.15 (4H, m, 4,6-CH₂); 1.72 (3H, s, 3-CH₃); 3.88 (1H, m, 1-CH); 5.41 (1H, s, C=CH) and 8.40 ppm (3H, br s, NH₃⁺).

### Synthesis Example 15.

### 1,3,5,5-Tetramethyl-2-cyclohexen-1-amine hydrochloride (40).

### a) 1,3,5,5-Tetramethyl-1,3-cyclohexadiene (37) and 1,5,5 -trimethyl-3-methylene-1-cyclohexene (38) mixture.

To a stirred 2 M ethereal solution of methylmagnesium iodide (15 ml, 30 mmol) was added dropwise a solution of 3,5,5 -trimethyl-2-cyclohexen-1-one **(36)** (1.38 g, 10 mmol) in dry ether (15 ml). The mixture was stirred for 1 h, cooled with ice water and carefully treated with 15% aqueous CH₃COOH (15 ml). The mixture was stirred for an additional hour. The organic layer was separated and washed with water and saturated aqueous NaHCO₃. After drying over MgSO₄, the solution was concentrated *in vacuo.* The residue was purified by flash chromatography (light petroleum ether, Rf 0.95 (hexane)) to give a mixture of **37** and **38** (0.955 g, 70%) (7:10, based on GC) as an oil. ¹H NMR (CDCl₃, TMS) δ: 0.89, 0.98 and 1.03 (total 10.2H, all s, 5,5-CH₃); 1.55 - 2.20 (total 12.6H, m, CH₂C= and CH₃C=); 4.69 (2H, dm, 4 Hz, =CH₂); 5.06 (0.7H, m, =CH); 5.50 (0.7H, sept, 1.5 Hz, =CH) and 5.92 ppm (1H, m, =CH).

### b) 3-Azido-1,5,5,5-tetramethyl-1-cyclohexene (39).

Prepared from 37 and **38** mixture according to the procedure for compound **34 (Synthesis Example 14, a).** Azide **39** obtained as a colorless oil with 43% yield. ¹H NMR (CDCl₃, TMS) δ: 0.93 and 0.99 (total 6H, both s, 5,5-CH₃); 1.31 (3H, s, 1-CH₃); 1.36 and 1.62 (total 2H, both d, 13 Hz, 4-CH₂); 1.72 (5H, s, 1 -CH₃, 6-CH₂); 5.32 (1H, s, C=CH).

### c) 1,3,5,5-Tetramethyl-2-cyclohexen-1-amine hydrochloride (40).

Prepared from azide **39** according to the procedure for compound **11 (Synthesis Example 3, c).** Amine hydrochloride **40** obtained as a colorless solid with 60% yield. ¹H NMR (CDCl₃, TMS) δ: 0.96 and 1.07 (total 6H, both s, 5,5-CH₃); 1.56 (3H, s, 1-CH₃); 1.73 (3H, s, 3 -CH₃); 1.60-2.05 (4H, m, 4,6 -CH₂); 5.49 (1H, s, C=CH) and 8.27 ppm (3H, br s, NH₃⁺).

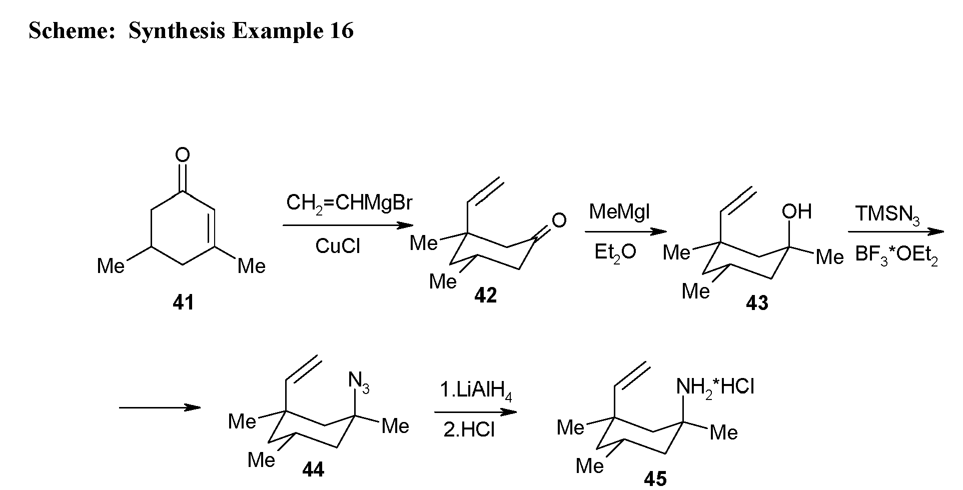

### Synthesis Example 16.

### 1,3, trans-5-trimethyl-cis-3-vinylcyclohexanamine hydrochloride (45).

### a) 3,5-dimethyl-3-vinylcylohexanone (42).

A 1M solution of vinylmagnesium bromide in THF (90 ml, 90 mmol) was cooled in dry ice-acetone bath to -20°C in an inert atmosphere and CuCl (4.45 g, 45 mmol) was added in one portion. The mixture was stirred for 30 min and a solution of 3,5-dimethyl-2- cyclohexen-1-one **(41)** (3.73 g, 30 mmol) in THF (40 ml) was added dropwise keeping the reaction temperature at -20°C. The cooling bath was removed and the reaction mixture was allowed to reach room temperature for 2 h. Saturated aqueous NH₄Cl (50 ml) was added thoroughly while cooling with ice bath. Hexane (150 ml) was then added and the aqueous layer was separated and extracted with hexane (2x100 ml).

The combined organic extracts were washed with 20% aqueous acetic acid (100 ml) and with saturated aqueous NaHCO₃ (3x200 ml). The extract was dried over MgSO₄, filtered and evaporated. The crude product was purified by flash chromatography on silica gel (light petroleum ether - ethyl acetate, 20: 1) to give **42** (2.4 g, 52 %) as a colorless oil. ¹H-NMR (CDCl₃, TMS) δ: 0.99 (3H, d, 6 Hz, 5-CH₃); 1.11 (3H, s, 3-CH₃); 1.2-2.6 (7H, m, ring protons); 4.94 and 5.01 (total 2H, both d, 17 and 10.5 Hz, CH₂=) and 5.64 ppm (1H, dd, 17 and 11 Hz, =CH).

### b) 1,3, trans-5-trimethyl-cis-3-vinylcyclohexanol (43).

A solution of ketone **42** (1g, 6.6 mmol) in diethyl ether (10 ml) was added to 1.6 M methyl lithium solution in diethyl ether (12 ml, 19.6 mmol) while cooling in an ice bath. The resulting mixture was stirred for 1 h at 0-5°C and saturated aqueous NH₄Cl (10 ml) was added thoroughly. The aqueous layer was separated and extracted with diethyl ether (2x15 ml). The combined organic phases were washed with brine (20 ml) and dried over MgSO₄. The extract was filtered and evaporated. The crude product was purified by flash chromatography on silica gel (3% ethyl acetate in light petroleum ether). Cyclohexanol **43** (0.82 g, 74%) was obtained as a colorless oil that was used in the next step without characterization.

### c) 1-Azido-1,3, trans-5-trimethyl-cis-3-vinylcyclohexane (44).

Prepared from cyclohexanol **43** according to the procedure for compound **9 (Synthesis Example 3, b).** Azide **44** obtained as a colorless oil with 17% yield. ¹H-NMR (CDCl₃, TMS) δ: 0.94 (3H, d, 6.5 Hz, 5-CH₃); 0.97 (3H, s, 3-CH₃); 1.27 (3H, s, 1-CH₃); 0.7-2.0 (7H, m, ring protons); 4.95 and 4.97 (total 2H, both d, 18 and 11 Hz, =CH₂) and 5.77 ppm (1H, dd, 18 and 11 Hz, =CH).

### d) 1,3, trans-5-trimethyl-cis-3-vinylcyclohexanamine hydrochloride (45).

Prepared from azide **44** according to the procedure for compound **11 (Synthesis Example 3, c).** Amine hydrochloride **45** obtained as a colorless solid with 32% yield. ¹H-NMR (CDCl₃, TMS) δ: 0.92 (3H, d, 6.5 Hz, 5-CH₃); 0.96 (3H, s, 3-CH₃); 1.45 (3H, s, 1-CH₃); 0.8-2.1 (9H, m, 2,4,6-CH₂, 5-CH and H₂O); 4.94 and 4.97 (2H, both d, 18 and 11 Hz, =CH₂); 5.76 (1H, dd, 18 and 11 Hz, =CH) and 8.26 ppm (3H, br s, NH₃⁺).

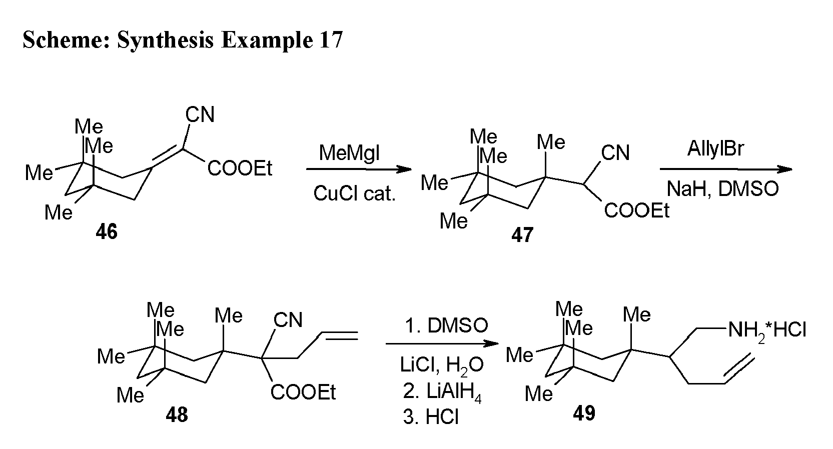

### Synthesis Example 17.

### 2-(1,3,3,5,5-Pentamethylcyclohexyl)-4-pentenylamine hydrochloride (49).

### a) Ethyl 2-cyano-2-(1,3,3,5,5-pentamethylcyclohexyl)acetate (47).

Copper (I) chloride (0.05 g, 0.5 mmol) was added to a cooled (- 10 °C ) in argon atmosphere 1M methylmagnesium iodide in ethyl ether (15 ml, 15 mmol) and stirred for 5 min. Then a solution of acetate **46** (2.5 g, 10 mmol) in THF (25 ml) was added dropwise within 20 min, keeping the temperature below 0°C. The mixture was stirred for 1 h, quenched with saturated aqueous NH₄Cl, and extracted with diethyl ether The extract was washed with brine, dried over anhydrous MgSO₄, filtered and evaporated. The residue was purified by flash chromatography on silica gel (light petroleum ether - ethyl acetate, 20:1) to give **47** (1.5 g, 56.5%) as a colorless oil. ¹H NMR(CDCl₃, TMS) δ: 1.01, 1.07 and 1.09 (total 12H, s, 3',5'-CH₃); 1.00-1.85 (6H, m, ring CH); 1.30 (3H, s, 1'-CH₃); 1.33 (3H, t, 7 Hz, CH₃ -ethyl); 3.44 (1H, s, 2 -CH) and 4.27 ppm (2H, q, 7 Hz, OCH₂).

### b) Ethyl 2-cyano-2-(1,3,3,5,5-pentamethylcyclohexyl)-4-pentenoate (48)

To a solution of cyanoacetate **47** (1.25 g, 4.71 mmol) in anhydrous DMSO (10 ml) was added sodium hydride (0.284 g, 7.09 mmol; 60% mineral oil dispersion). The mixture was stirred for 30 min at 50°C, and cooled to 20°C. To this was added allylbromide (0.86 g, 7.1 mmol) and the mixture was stirred for 3 h at room temperature, then for 30 min at 50°C. The mixture was cooled, treated with water and extracted with diethyl ether. The extract was washed with water and with brine, dried over anhydrous MgSO₄, filtered and evaporated. The residue was purified by flash chromatography on silica gel (light petroleum ether - ethyl acetate, 20:1) to give **48** (0.92 g, 63.7 %) as a colorless oil. ¹H NMR (CDCl₃, TMS) δ: 0.98 (6H, s, 3',5'-CH_{3eq}); 1.11 (6H, s, 3',5'-CH₃ₐₓ); 1.00-1.85 (6H, m, ring CH); 1.31 (3H, t, 7 Hz, CH₃-ethyl); 1.33 (3H, s, 1'-CH₃); 2.42 and 2.86 (total 2H, both dd, 13 and 7 Hz, 3 -CH₂); 4.02 (2H, q, 7 Hz, OCH₂); 5.05-5.37 (2H, m, =CH₂) and 5.55-6.05 ppm (1H, m, =CH).

### c) 2-(1,3,3,5,5-Pentamethylcyclohexyl)-4-pentenylamine hydrochloride (49)

To a solution of ester **48** (0.9 g, 2.95 mmol) in DMSO (10 ml) was added water (0.53 ml, 2.95 mmol) and lithium chloride (0.25 g, 5.9 mmol). The mixture was stirred for 3 h at 175-180 °C , then it was cooled and water (30 ml) was added. The mixture was extracted with diethyl ether. The extract was washed with water and with brine, dried over anhydrous MgSO₄, filtered and concentrated to 10 ml volume. The solution obtained was added dropwise to a suspension of lithium aluminum hydride (0.25 g, 6.6 mmol) in diethyl ether (15 ml) and stirred at reflux for 3 h. The mixture was cooled and treated with 20% aqueous NaOH, and extracted with diethyl ether. The extract was washed with brine, dried over NaOH, filtered and treated with anhydrous HCl solution in diethyl ether. After evaporation of the solvent, the residue was purified by chromatography on silica gel (chloroform - methanol, 20:1) to give **49** (0.245 g, 31 %) as a colorless solid. ¹H NMR (DMSO-D₆, TMS) δ: 0.92, 0,96 and 1.04 (total 15H, all s, 3',5'-CH₃ and 1'-CH₃), 1.00-1.65 (total 6H, m, ring -CH₂); 1.85-2.40 (3H, m, 3 - CH₂, 4-CH); 2.60-3.10 (2H, m, CH₂N); 4.90 -5.25 (2H, m, =CH₂); 5.62-6.10 (1H, m, =CH) and 7.92 ppm (3H, br s, NH₃⁺).

### Synthesis Example 18

### 1, exo-3,5-Trimethyl-6-azabicyclo[3.2.1]octane hydrochloride (1-1).

### a) 1, exo-3,5-Trimethyl-6-azabicyclo[3.2.1]oct-6-ene (5-1).

A mixture of of 1,3,3, *trans*-5-tetramethylcyclohexanamine **(4-1)** (3.88 g, 25 mmol), K₂CO₃ (28 g, 0.2 mol) and lead tetraacetate (22.2 g, 50 mmol) in dry benzene (125 ml) was stirred for 3 h while boiling at reflux. Then it was cooled with ice water and filtered. The precipitate was washed with diethyl ether and the filtrate evaporated under reduced pressure. The oily residue was separated by column chromatography on silica gel (dichlorometane - *iso*-propyl alcohol, 20:1, 10:1). A fraction with Rf 0.7 (EtOAc) was collected to give after concentration under reduced pressure 1.0 g (26%) of imine **5-1** as an amber oil. ¹H NMR (CDCl₃, TMS) δ: 0.86 (3H, d, 6 Hz, 3-CH₃), 0.90-1.80 (7H, m, ring CH); 1.12 (3H, s, 1 -CH₃); 1.34 (3H, s, 5-CH₃) and 7.36 ppm (1H, s, HC=).

### b) 1, exo-3,5-Trimethyl-6-azabicyclo[3.2.1]octane hydrochloride (1-1).

A solution of imine **5-1** (0.8 g, 5.3 mmol) in MeOH (2 ml) was added dropwise to a suspension of sodium borohydride (0.4 g, 10.6 mmol) in MeOH (6 ml). The mixture was stirred at room temperature for 24 h, then 10 ml of 5% aqueous NaOH was added. The mixture was extracted with diethyl ether. The organic phase was washed with saturated aqueous NaCl and dried over NaOH pellets. The filtered solution was treated with dry HCl solution in diethyl ether, evaporated under reduced pressure and the residue was recrystallized from dry CH₃CN to give compound **1-1** as a colorless solid (0.33 g, 35%). ¹H NMR (CDCl₃, TMS) δ: 0.96 (3H, d, 6 Hz, 3 -CH₃), 0.95-1.15 (1H, m, 2 -CH); 1.11 (3H, s, 1 -CH₃); 1.41 (1H, d, 12.4 Hz, 8 -CH); 1.55-1.70 (1H, m, 2-CH); 1.57 (3H, s, 5 -CH₃); 1.70 -1.90 (2H, m, 4 -CH and 8-CH); 2.00 - 2.30 (2H, m, 3-CH and 4-CH); 3.00 -3.25 (2H, m, 7-CH₂) and 9.30 -9.85 ppm (2H, br s, NH₂⁺).

### Synthesis Example 19.

### 5-Ethyl-1, exo-3-dimethyl-6-azabicyclo[3.2.1]octane hydrochloride (1-2).

### a) 1-Azido-1-ethyl-3,3, trans-5-trimethylcyclohexane (7).

A cooled (∼0°C) mixture of 1-ethyl-3,3, *trans*-5-trimethylcyclohexanol (6) (3.3 g, 18.1 mmol), sodium azide (2.36 g, 36.3 mmol) and trifluoroacetic acid (10.7 ml) in chloroform (50 ml) was stirred for 24 h. Then it was made basic by diluted aqueous ammonia addition. The organic phase was separated, washed with water and dried over K₂CO₃. Filtration and solvent evaporation under reduced pressure gave an oily residue which was separated by flash chromatography on silica gel eluting with light petroleum ether to give azide 7 (2.0 g, 56%) as light colorless oil. ¹H NMR (CDCl₃, TMS) δ: 0.64 (1H, d, 14 Hz, ring CH); 0.85-2.15 (8H, m, ring CH and EtCH₂);); 0.90 (3H, d, 7 Hz, 5-CH₃); 0.92 (3H, s, 3-CH_{23q}); 0.97 (3H, t, 7.5 Hz, Et-CH₃) and 1.10 ppm (3H, s, 3-CH₃ₐₓ).

### b) 1-Ethyl-3,3, trans-5-trimethylcyclohexanamine (4-2).

Azide 7 (1.97 g, 10 mmol) solution in diethyl ether (10 ml) was added dropwise to a suspension of lithium aluminum hydride (1.13 g, 30 mmol) in diethyl ether (30 ml). The mixture was stirred for 20 h at room temperature. Then it was carefully quenched with 10% aqueous NaOH. The organic phase was separated and the aqueous phase extracted with diethyl ether. The combined organic phases were washed with saturated aqueous NaCl and dried over NaOH. Filtration and solvent evaporation under reduced pressure gave amine 4-2 (1.36 g, 80%) as an oil. ¹H NMR (CDCl₃, TMS) δ: 0.55-2.15 (9H, m, ring CH and Et -CH₂); 0.88 (3H, s, 3 -CH_{3eq}); 0.89 (3H, d, 6.5 Hz, 5-CH₃); 0.89 (3H, t, 7 Hz, Et-CH₃) and 1.12 ppm (3H, s, 3 -CH₃ₐₓ). **c) 5-Ethyl-1, *exo*-3-dimethyl-6-azabicyclo[3.2.1]octane hydrochloride (1-2).**

Prepared in 30% yield from imine 5-2 according to procedure described in Example 18b. Colorless solid. ¹H NMR (CDCl₃, TMS) δ: 0.95-1.15 (7H, m, ring CH, 3-CH₃ and CH₃-Et); 1.12 (3H, s, 1-CH₃); 1.48 (1H, d, 13.6 Hz, 8 -CH); 1.55-1.76 (3H, m, ring CH and CH₂-Et); 1.84-2.04 (2H, m, ring CH) and 2.04-2.28 (2H, m, 4,8 - CH); 3.14 (2H, m, 7-CH₂) and 9.40 ppm (2H, br s, NH₂⁺).

### d) 5-Ethyl-1, exo-3-dimethyl-6-azabicyclo[3.2.1]oct-6-ene (5-2).

Prepared in 32% yield from amine 4-2 according to procedure described in Example 18a. An oil. ¹H NMR (CDCl₃, TMS) δ: 0.82-0.95 (1H, m, ring CH); 0.91 (3H, d, 6 Hz, 3-CH₃), 0.94 (3H, t, 7.5 Hz, Et -CH₃); 1.15-1.75 (6H, m, ring CH); 1.15 (3H, s, 1-CH₃); 1.71 (2H, q, 7.5 Hz, Et -CH₂) and 7.38 ppm (1H, s, HC=).

### Synthesis Example 20.

### exo-3-Ethyl-1,5-dimethyl-6-azabicyclo[3.2.1]octane hydrochloride (1-3).

### a) tert-Butyl trans-5-ethyl-1,3,3-trimethylcyclohexylcarbamate (8-1).

To a solution of 1,3,3-trimethyl-*trans*-5-ethylcyclohexanamine hydrochloride **(4-3)** (1.54 g, 7.5 mmol) in THF (20 ml) was added Na₂CO₃ (3.18 g, 30 mmol) and the mixture was stirred for 30 min. Then it was cooled with ice water, di-*tert*-butyl dicarbonate (1.7 g, 7.65 mmol) was added and stirring was continued for 20 h. Water was added and the mixture was twice extracted with diethyl ether. The combined extracts were washed with saturated aqueous NaCl, dried over MgSO₄ and evaporated. The solid residue was treated with hexane, filtered and washed with hexane.to give carbamate **8-1.** Additional amount of **8-1** was isolated after the filtrate was evaporated and treated with acetonitrile. Carbamate **8-1** (1.18 g, 57%) was obtained as a colorless solid with mp 70-71°C. ¹H NMR (CDCl₃, TMS) δ: 0.65-1.65 (7H, m, CH₂-Et and ring CH); 0.88 (3H, t, 6.5 Hz, CH₃-Et); 0.88 and 0.99 (both 3H, s, 3,3 -CH₃); 1.42 (9H, s, t - Bu); 1.85 (1H, dq, 13.5 and 2.5 Hz, 6 -CH _{eq}); 2.24 (1H, d, 14 Hz, 2 -CH _{eq}) and 4.30 ppm (1H, br s, NH). **b) *tert*-Butyl *exo*-3-ethyl-1,5-dimethyl-6-azabicyclo[3.2.1]octane-6-carboxylate (9-1).**

To a mixture of carbamate **8-1** (1.05 g, 3.85 mmol) and iodine (1.95 g, 7.7 mmol) in dry benzene (35 ml) was added lead tetraacetate (3.92 g, 8.85 mmol) in one portion. The mixture was stirred while boiling at reflux for 4 h then cooled with ice water and filtered. The precipitate was washed with diethyl ether and the filtrate carefully washed with saturated aqueous potassium metabisulfite followed by saturated aqueous NaHCO₃. The organic phase was washed with saturated aqueous NaCl , dried over MgSO₄ and evaporated. The residue was purified by flash chromatography on silica gel (light petroleum ether - ethyl acetate, 20:1) to give compound **9-1** (0.76 g, 73%) as a colorless oil. ¹H NMR (CDCl₃, TMS) δ: 0.86 (3H, t, 6.5 Hz, CH₃ -Et); 1.00 (3H, s, 1-CH₃); 1.00-1.80 (7H, m, CH₂ -Et and ring CH); 1.46 (12H, s, t -Bu and 5-CH₃); 1.95-2.45 (2H, m, ring -CH); 3.06 and 3.36 ppm (both 1H, d, 11 Hz, 7 -CH₂). **c) *exo*-3-Ethyl-1,5-dimethyl-6-azabicyclo[3.2.1]octane hydrochloride (1-3).**

Carbamate **9-1** (0.73 g, 2.7 mmol) was added to a solution of trifluoroacetic acid (3 ml) in dichloromethane (15 ml) and the mixture was stirred at room temperature for 10 h. The solution was evaporated under reduced pressure and the residue was treated with 10% aqueous NaOH (5 ml) and extracted with diethyl ether. The extract was washed with saturated aqueous NaCl and dried over NaOH. The filtered solution was treated with dry HCl solution in diethyl ether. The solvent was evaporated under reduced pressure and the residue was treated with dry acetonitrile to give amine hydrochloride **1-3** as colorless solid (0.34 g, 62%). ¹H NMR (CDCl₃, TMS) δ: 0.85-2.45 (9H, m, 2,4,8 -CH₂, 3-CH and CH₂-Et); 0.90 (3H, t, 7 Hz, CH₃-Et); 1.12 (3H, s, 1-CH₃); 1.59 (3H, s, 5-CH₃); 3.13 (2H, t, 6 Hz, 7 -CH₂) and 9.55 ppm (2H, br s, NH₂⁺).

### Synthesis Example 21.

### 1,3,3,5-Tetramethyl-6-azabicyclo [3.2.1]octane hydrochloride (1-4).

### a) tert-Butyl 1,3,3,5,5-pentamethylcyclohexylcarbamate (8-2).

Prepared in 70% yield from 1,3,3,5,5-pentamethylcyclohexanamine hydrochloride **(4-4)** according to the procedure described in **Example 20a.** Purified by flash chromatography on silica gel (light petroleum ether - ethyl acetate, 20:1). A colorless oil. ¹H NMR (CDCl₃, TMS) δ: 0.87 (6H, s, 3,5 -CH_{seq}); 0.90-1.45 (4H, m, 4-CH₂ and 2,6-CHₐₓ); 1.12 (6H, s, 3,5 -CH₃ₐₓ); 1.27 (3H, s, 1-CH₃); 1.42 (9H, s, t -Bu); 2.24 (2H, d, 15 Hz, 2,6 -CH_{eq}) and 4.30 ppm (1H, br s, NH).

### b) tert-Butyl 1,3,3,5-tetramethyl-6-azabicyclo[3.2.1]octane-6-carboxylate (9-2).

Prepared in 48% yield from carbamate **8-2** according to the procedure described in **Example 20b**. A colorless oil. ¹H NMR (CDCl₃, TMS) δ: 0.91, 0.94 and 0.99 (total 9H, all s, 1,3,3-CH₃); 0.80-1.75 (5H, m, ring CH); 1.34 and 1.52 (total 3H, both s, 5-CH₃); 1.41 and 1.44 (total 9H, both s, t-Bu); 1.91 and 2.09 (total 1H, both d, 14.5 Hz, 6-CH); 3.00 and 3.28 (one rotamer); and 3.03 and 3.33 (another rotamer; total 2H, all dd, 11 and 2 Hz, 7-CH₂ ).

### c) 1,3,3,5-Tetramethyl-6-azabicyclo [3.2.1]octane hydrochloride (1-4).

Prepared in 68% yield from carbamate **9-2** according to the procedure described in **Example 20c.** Colorless solid. ¹H NMR (CDCl₃, TMS) δ: 1.00, 1.13 and 1.29 (total 9H, s, 1,3,3-CH₃); 1.25-1.65 (4H, m, 2 -CH₂ and 4,8 -CH); 1.64 (3H, s, 5 - CH₃); 1.81 (1H, dt, 12.4 and 2.3 Hz, 4 -CH); 2.21 (1H, d, 14.5 Hz, 8 -CH); 3.10-3.40 (2H, m, 7-CH₂); 9.10 and 9.90 ppm (total 2H, both br s, NH₂⁺).

### Synthesis Example 22.

### 1,3,3,5,6-Pentamethyl-6-azabicyclo [3.2.1]octane hydrochloride (1-5).

### a) Methyl 1,3,3,5-tetramethyl-6-azabicyclo[3.2.1]octane-6-carboxylate (11).

Prepared in 50% yield from methyl 1,3,3,5,5 - pentamethylcyclohexylcarbamate **(10)** according to the procedure described in **Example 20b**. A colorless oil. ¹H NMR (CDCl₃, TMS) δ: 0.87 and 0.96 (total 9H, both s, 1,3,3-CH₃); 1.00-1.70 (4H, m, 2-CH₂ and 4,8-CH); 1.33 and 1.46 (total 3H, both s, 1-CH₃); 1.70-1.20 (2H, m, 4,8 -CH); 3.04 and 3.34 (major rotamer) and 3.10 and 3.39 (minor rotamer; total 2H, all dd, 11.5 and 1.5 Hz, 7 -CH₂); 3.59 (major) and 3.64 (total 3H, both s, OCH₃).

### b) 1,3,3,5,6-Pentamethyl-6-azabicyclo [3.2.1]octane hydrochloride (1-5).

A solution of carbamate **11** (1.0 g, 4.44 mmol) in diethyl ether (10 ml) was added to a suspension of lithium aluminum hydride (0.34 g, 9 mmol) in diethyl ether (25 ml). The mixture was stirred for 20 h at room temperature. Then it was cooled with ice water and carefully quenched with 10% aqueous NaOH. The organic phase was separated and the aqueous phase extracted with diethyl ether. The combined organic phases were washed with saturated aqueous NaCl and dried over NaOH. Filtered solution was treated with an excess amount of dry HCl solution in diethyl ether. The solvent was evaporated under reduced pressure and the residue was treated with dry acetonitrile and diethyl ether (2:1), and cooled in refrigerator for 24 h. The precipitate was filtered and washed with diethyl ether to give amine hydrochloride **1-5** (0.25 g, 26%) as a colorless solid. ¹H NMR (CDCl₃, TMS) δ: 1.03, 1.09, 1.16 and 1.22 (total 9H, all s, 1,3,3 -CH₃); 1.44 (3H, s, 5 -CH₃); 1.50-2.50 (6H, m, 2,4,8 -CH₂); 2.73 (d, 5 Hz) and 2.80 (total 3H, d, 5.5 Hz, N-CH₃); 2.55 (m) and 2.94 (total 1H, dd, 12 and 6 Hz, 7-CH); 3.73 (dd, 12 and 8.5 Hz) and 4.07 (total 1H, dd, 13 and 7 Hz, 7 -CH); 9.50 and 10.80 ppm (total 1H, br s, NH ⁺).

### Synthesis Example 23.

### 5-Ethyl-1,3,3-trimethyl-6-azabicyclo[3.2.1]octane hydrochloride (1-6).

### a) 5-Ethyl-1,3,3-trimethyl-6-azabicyclo[3.2.1]oct-6-ene (5-3).

Prepared in 28% yield from 1-ethyl -3,3,5,5-tetramethylcyclohexanamine (4-5) according to the procedure described in **Example 18a.** An oil. ¹H NMR (CDCl₃, TMS) δ: 0.93 (3H, s, 3-CH₃); 0.94 (3H, t, 7.4 Hz, Et -CH₃); 0.98 (3H, s, 3 -CH₃); 1.15 (3H, s, 1-CH₃); 1.20-1.50 (5H, m, ring CH); 1.57(1H, dt, 12.4 and 2 Hz, ring CH); 1.69 (2H, dq, 7.5 and 2.8 Hz, Et -CH₂) and 7.47 ppm (1H, s, HC=). **b) 5-Ethyl-1,3,3-trimethyl-6-azabicyclo[3.2.1]octane hydrochloride (1-6).**

Prepared in 33% yield from imine **5-3** according to the procedure described in **Example 18b.** Colorless solid. ¹H NMR (CDCl₃, TMS) δ: 1.01 (3H, s, 3 - CH₃); 1.03 (3H, t, 7.5 Hz, CH₃-Et); 1.13 and 1.31 (both 3H, s, 1,3 -CH₃); 1.25-1.35 (1H, m, ring CH); 1.35 -1.65 (4H, m, CH₂ -Et and ring CH); 1.69 (1H, d, 12 Hz, 2 -CH); 1.92-2.12 (2H, m, 4,8 -CH); 3.05-3.45 (2H, m, 7 -CH₂): 9.05 and 9.65 ppm (both 1H, br s, NH₂⁺).

### Synthesis Example 24.

### 1, exo-3,5, exo,endo-7-Tetramethyl-6-azabicyclo[3.2.1]octane hydrochloride (1-7). a) tert-Butyl cis-3-ethyl-1,3, trans-5-trimethylcyclohexylcarbamate (8-3).

Prepared in 81% yield from 1,3,5-trimethyl-*cis*-3-ethylcyclohexanamine hydrochloride (4-6) according to the procedure described in **Example 20a.** Purified by flash chromatography on silica gel (light petroleum ether - ethyl acetate, 20:1). A colorless oil. ¹H NMR (CDCl₃, TMS) δ: 0.63 (1H, d, 12.5 Hz, ring CH); 0.70 - 0.90 (1H, m, ring CH); 0.79 (3H, t, 7.5 Hz, CH₃-Et); 0.86 (3H, d, 6.4 Hz, 5 -CH₃); 1.28 (3H, s, 3-CH₃); 1.25-1.85 (6H, m, ring CH and CH₂-Et); 1.41 (9H, s, t -Bu); 1.52 (3H, s, 1-CH₃); 2.35 (1H, d, 12.5 Hz, 2 -CH) and 4.31 ppm (1H, br s, NH).

### b) tert-Butyl 1, exo-3,5, exo,endo -7-tetramethyl 6-azabicyclo[3.2.1]octane-6-carboxylate (9-3).

Prepared in 57% yield from carbamate 8-3 according to the procedure described in **Example 20b**. A colorless oil. ¹H NMR (CDCl₃, TMS) δ: 0.60-1.85 (6H, m, ring CH); 0.85-1.15 (6H, m, 1,3-CH₃); 1.35-1.55 (6H, m, 5,7-CH₃); 1.45 (9H, s, t - Bu); 2.06 and 2.27 (total 1H, m, ring CH); 3.36 and 3.51 ppm (total 1H, m, 7-CH).

### c) 1, exo-3,5, exo,endo-7-Tetramethyl-6-azabicyclo[3.2.1]octane hydrochloride (1-7).

Prepared in 70% yield from carbamate 9-3 according to the procedure described in **Example 20c.** A colorless solid. ¹H NMR (CDCl₃, TMS) δ: 0.96 (3H, d, 5.8 Hz, 3-CH₃); 1.00 (3H, s, 1-CH₃); 1.00-1.15 (1H, m, 2 -CH); 1.36 (1H, d, 12 Hz, 8 - CH); 1.43 (3H, d, 7.4 Hz, 7 -CH₃); 1.55-1.75 (2H, m, 2 -CH and 4-CH); 1.62 (3H, s, 5 - CH₃); 1.90 (1H, d, 12.6 Hz, 8 -CH); 2.15-2.35 (2H, m, 3 -CH and 4-CH); 3.65 (1H, m, 7 -CH); 9.00 and 9.95 ppm (total 2H, both br s, NH₂⁺).

### Synthesis Example 25.

### 1, exo-3,5-Trimethyl-exo,endo-7-phenyl-6-azabicyclo[3.2.1]octane hydrochloride (1-8).

### a) 3-Benzyl-3,5-dimethylcyclohexanone (13).

To a cooled (-20°C) 1M benzylmagnesium bromide solution in diethyl ether (50 ml) under argon was added CuCl (0.52 g, 5.3 mmol) and the mixture was stirred for 5 min. Then a solution of 3,5-dimethyl-2-cyclohexen-1-one (12) (4.4 g, 35.1 mmol) in diethyl ether (15 ml) was added dropwise keeping the temperature below - 10°C. The mixture was stirred for 2 h and quenched with 10% aqueous acetic acid (40 ml). The organic layer was separated, washed with water, saturated aqueous NaHCO₃ and saturated aqueous NaCl, and dried over MgSO₄. Filtration and concentration *in vacuo* afforded oily residue what was separated by flash chromatography on silica gel (light petroleum ether - ethyl acetate, 10:1). Cyclohexanone 13 (4.0 g, 53%) was obtained as a colorless oil. ¹H NMR (CDCl₃, TMS) δ: 0.92 (3H, s, 3-CH₃); 1.06 (3H, d, 6 Hz, 5-CH₃), 1.10-2.45 (7H, m, ring CH); 2.42 and 2.56 (total 2H, both d, 13 Hz, CH₂Ph) and 7.05 - 7.35 ppm (5H, m, Ph).

### b) cis-3-Benzyl-1,3,trans-5-trimethylcyclohexanol (14).

A solution of ketone 13 (3.9 g, 18.1 mmol) in diethyl ether (10 ml) was added dropwise to 1M MeMgI in diethyl ether (40 ml). The mixture was stirred for 1 h at room temperature. Etheral extract obtained after traditional workup for Grignard reactions was dried over Na₂SO₄, filtered and evaporated to give an oily residue what was purified by flash chromatography on silica gel (light petroleum ether - ethyl acetate). Cyclohexanol 14 (3.2 g, 76%) was obtained as a colorless oil. ¹H NMR (CDCl₃, TMS) δ: 0.75 (3H, s, 3 -CH₃); 0.95-1.25 (3H, m, ring CH); 0.92 (3H, d, 6.6 Hz, 5-CH₃), 1.23 (3H, s, 1-CH₃); 1.45-1.75 (3H, m, ring CH); 2.05 -2.25 (1H, m, 5-CH); 2.77 and 3.04 (both 1H, d, 13 Hz, CH₂Ph) and 7.05 - 7.35 ppm (5H, m, aryl CH). **c) N-(*cis*-3-Benzyl-1,3, *trans*-5-trimethylcyclohexyl)-2-chloroacetamide (15).**

Sulfuric acid (2.1 ml, 3.83 g, 39 mmol) was added dropwise to a stirred solution of cyclohexanol 14 (3.0 g, 13 mmol) and chloroacetonitrile (4.0 g, 52 mmol) in acetic acid (2.1 ml) while cooling with ice water. The mixture was stirred for 24 h at room temperature then poured into ice water (10 ml). The mixture was neutralised with 20% aqueous NaOH and extracted with diethyl ether (3×15 ml). The combined organic phases were washed with saturated aqueous NaCl and dried over MgSO₄. The extract was filtered and the solvent evaporated. The residue was purified by flash chromatography on silica gel eluting with a mixture of light petroleum ether and ethyl acetate (10:1) to give amide 15 (1.32 g, 33%) as a colorless oil. ¹H NMR (CDCl₃, TMS) δ: 0.73 (3H, s, 3 -CH₃); 0.90-1.40 (3H, m, ring CH); 0.98 (3H, d, 6.6 Hz, 5 - CH₃), 1.42 (3H, s, 1-CH₃); 1.63 (1H, m, ring CH); 1.80 -2.05 (1H, m, 5-CH); 2.12 (1H, dq, 13.8 and 3 Hz, 6 -CH); 2.33 (1H, d, 12.7 Hz, CH₂Ph); 2.51 (1H, dt, 15 and 2.2 Hz, 2 -CH); 3.17 (1H, d, 12.7 Hz, CH₂Ph); 3.95 and 3.96 (total 2H, both s, CH₂CO); 6.52 (1H, br s, NH) and 7.00 -7.35 ppm (5H, m, aryl CH).

### d) cis-3-Benzyl-1,3, trans-5-trimethylcyclohexanamine hydrochloride (4-7).

A solution of amide 15 (0.62 g, 2 mmol) and thiourea (0.18 g, 2.4 mmol) in a mixture of ethanol (5 ml) and acetic acid (1 ml) was refluxed for 10 h. The reaction mixture was cooled to room temperature and 20 ml of 10% aqueous NaOH was added while stirring. The resulting mixture was extracted with diethyl ether (3× 10 ml). The combined extracts were washed with saturated aqueous NaCl, dried over NaOH, filtered and treated with dry HCl solution in diethyl ether. The solvent was evaporated under reduced pressure and the residue treated with dry diethyl ether to give amine hydrochloride 4-7 (0.33 g, 35%) as a colorless solid. ¹H NMR (CDCl₃, TMS) δ: 0.68 (3H, d, 6.5 Hz, 5 -CH₃); 0.70-1.30 (3H, m, ring CH); 0.73 (3H, s, 3 -CH₃); 1.28 (3H, s, 1-CH₃); 1.50 (1H, d , 15.4 Hz, ring CH); 1.60 -1.85 (1H, m, ring CH); 2.05 (1H, d, 16 Hz, ring CH); 2.15-2.50 (1H, m, 5-CH); 2.47 and 3.33 (both 1H, d, 12.8 Hz, CH₂Ph); 7.00-7.35 (5H, m, aryl CH) and 8.42 ppm (3H, br s, NH₃⁺).

### e) 1, exo-3,5-Trimethyl-7-phenyl-6-azabicyclo[3.2.1]oct-6-ene (5-4).

Prepared in 40% yield from free amine 4-7 according to the procedure described in **Example 18a.** An oil. ¹H NMR (CDCl₃, TMS) δ: 0.94 (3H, d, 6.6 Hz, 3-CH₃); 0.90-1.15 (2H, m, ring CH); 1.26 (3H, s, 1 -CH₃); 1.30-1.90 (5H, m, ring CH); 1.43 (3H, s, 5-CH₃) and 7.30 -7.65 ppm (5H, m, aryl CH).

### f) 1, exo-3,5-Trimethyl-exo,endo-7-phenyl-6-azabicyclo[3.2.1]octane hydrochloride (1-8).

Prepared in 33% yield from imine 5-4 according to the procedure described in **Example 18b.** Colorless solid. ¹H NMR (CDCl₃, TMS) δ: 0.75-1.95 (4H, m, ring CH); 0.86 (3H, d, 5,8 Hz, 3 -CH₃); 1.20 (3H, s, 1-CH₃); 1.56 (3H, s, 5 -CH₃); 1.99 (1H, d, 14.4 Hz, 8 -CH); 2.05-2.15 (1H, m, ring CH); 2.20 -2.30 (1H, m, ring CH); 4.57 (1H, m, 7-CH); 7.24 and 7.65 (total 5H, both br s, Ph); 9.15 and 10.40 ppm (total 2H, both br s, NH₂⁺).

### Synthesis Example 26.

### 1,5, exo-7-Trimethyl-2-azabicyclo[3.3.1]nonane hydrochloride (1-9).

### a) 2-{cis-3-[(2-Chloroacetyl)amino]-1,3, trans-5-trimethylcyclohexyl}acetic acid (16).

To a solution of 1.5 g (4.9 mmol) of N-(*cis*-3-Benzyl-1,3, *trans -5-*trimethylcyclohexyl)-2-chloroacetamide (obtained from a fraction with Rf 0.7-0.8 (Hexanes-EtOAc, 2:1) separated after the synthesis of compound **15, Example 25c** ) in a mixture of acetonitrile (16 ml), tetrachloromethane (16 ml) and water (23 ml) was added sodium periodate (10.5 g, 49 mmol) and ruthenium dioxide (7 mg, 0.06 mmol). The mixture was stirred at room temperature for 72 h, then it was filtered and the filter cake was washed with dichloromethane. The organic phase of the filtrate was separated and the aqueous phase was extracted with dichloromethane. The combined organic phases were dried over CaCl₂, filtered and evaporated. The residue was purified by flash chromatography on silica gel eluting with chloroform to give acid **16** (0.55 g, 41%) as an oil. ¹H NMR (DMSO-d₆, TMS) δ: 0.8-2.6 (7H, m, ring CH); 0.84 and 0.85 (total 3H, d, 6 Hz, 5-CH₃); 0.92 and 1.01 (total 3H, s, 1-CH₃); 1.21 and 1.22 (total 3H, s, 3-CH₃); 2.19 and 2.39 (total 2H, both d, 13.5 Hz, CH₂ CO); 3.97 ppm (2H, s, CH₂Cl); 7.57 and 7.70 (total 1H, both br s, NH) and 12.05 ppm (1H, br s, COOH).

### b) Ethyl 2-{cis-3-[(2-chloroacetyl)amino]-1,3, trans-5-trimethylcyclohexyl}acetate (17).

Thionyl chloride (0.73 ml, 10 mmol) was added dropwise to a solution of acid **16** (0.55 g, 1.99 mmol) in dry ethanol (5 ml), while cooling with ice water. The resulting solution was stirred for 15 h at room temperature then evaporated under reduced pressure. The residue was purified by flash chromatography on silica gel eluting with a mixture of light petroleum ether and ethyl acetate (6:1) to give ethyl ester **17** (0.32 g, 54%) as an oil. ¹H NMR (CDCl₃, TMS) δ: 0.7-1.6 (4H, m, ring CH); 0.88 - 0.94 (3H, m, 5-CH₃); 1.04 and 1.14 (total 3H, s, 1-CH₃); 1.25 (2H, t, 7 Hz, CH₃ -ethyl); 1.35 and 1.36 (total 3H, s, 3 -CH₃); 1.6 -1.8 (1H, m, 5-CH); 2.05-2.35 (2H, m, ring CH); 2.16 and 2.79 (total 2H, d, 13 Hz, CH₂CO); 3.92 and 3.95 (total 2H, s, CH₂ Cl); 4.12 (2H, q, 7 Hz, CH₂O); 6.42 and 7.28 ppm (total 1H, br s, NH).

### c) 1,5, exo-7-Trimethyl-2-azabicyclo[3.3.1]nonan-3-one (18).

A solution of ethyl ester 17 (0.32 g, 1.07 mmol) and thiourea (0.098g, 1.3 mmol) in a mixture of ethanol (5 ml) and acetic acid (1.2 ml) was refluxed for 20 h. The reaction mixture was cooled to room temperature and the solvents evaporated. 10% aqueous NaOH was added and the mixture was extracted with chloroform (3× 10 ml). The combined organic extracts were dried over CaCl₂, filtered and evaporated. The residue was purified by flash chromatography on silica gel eluting with a mixture of light petroleum ether and ethyl acetate (6:1, 3:1). A fraction with Rf 0.4 (Hexane-EtOAc, 2:1) was collected to give lactam **18** (0.12 g, 39%) as a colorless solid with mp 176-177°C. ¹H NMR (CDCl₃, TMS) δ: 0.89 (3H, d, 5.8 Hz, 7-CH₃); 0.75-1.05 (3H, m, ring CH); 0.99 (3H, s, 5-CH₃); 1.20 (3H, s, 1-CH₃); 1.24-1.36 (1H, m, ring CH); 1.45 - 1.60 (2H, m, ring CH); 1.60-1.84 (1H, m, 7 -CH); 2.14 (2H, s, 4 -CH₂) and 5.40 ppm (1H, br s, NH).

### d) 1,5,7-Trimethyl-2-azabicyclo[3.3.1]nonane hydrochloride (1-9).

1 M Borane solution in tetrahydrofuran (2 ml, 2 mmol) was added to a solution of lactam **18** (0.07 g, 0.385 mmol) in tetrahydrofuran (2 ml) and refluxed for 15 h. The mixture was cooled to room temperature and made acidic by addition of conc. aqueous HCl. Solvents were evaporated under reduced pressure and hexane (10 ml) and 20% aqueous NaOH (10 ml) were added to the residue. The organic phase was separated and the aqueous phase was extracted with hexane (2×5 ml). The combined organic phases were washed with saturated aqueous NaCl (10 ml) and dried over NaOH. The extract was filtered and dry HCl solution in diethyl ether was added. The solvent was evaporated and the residue was treated with diethyl ether (5 ml). The precipitate was collected on a filter to give amine hydrochloride **1-9** (0.02 g, 25%) as a colorless solid. ¹H NMR (CDCl₃, TMS) δ: 0.80-1.85 (7H, m, ring CH); 0.88 (3H, d, 6.5 Hz, 7-CH₃); 0.96 (3H, s, 5-CH₃); 1.50 (3H, s, 1-CH₃); 2.10-2.40 (2H, m, 7-CH and 8-CH); 3.15-3.35 and 3.30-3.55 (both 1H, m, 3 -CH₂); 9.15 and 9.55 ppm (both 1H, br s, NH₂⁺).

### Synthesis Example 27.

### 7,7,9,9-Tetramethyl-1-azaspiro[4.5]decane hydrochloride (3-1).

### a) 3,3,5,5-Tetramethyl-1-(2-phenylethyl)cyclohexanol (20-1).

A solution of 3,3,5,5-pentamethylcyclohexanone **(19)** (1.54 g, 10 mmol) in diethyl ether (10 ml) was added to 0.85 M solution of phenylethylmagnesium bromide in diethyl ether (25 ml, 20 mmol), while cooling with an ice bath. The resulting mixture was stirred for 0.5 h and saturated aqueous NH₄Cl (30 ml) was added thoroughly. The organic phase was separated and the aqueous phase was washed with diethyl ether (2x20 ml). The combined organic phases were washed with saturated aqueous NaCl solution (20 ml) and dried over MgSO₄. Filtration and evaporation of the solution gave a residue what was purified by flash chromatography on silica gel eluting with a mixture of light petroleum ether and ethyl acetate (10:1) to give cyclohexanol **201** (2.1 g, 82%) as an oil. ¹H-NMR (CDCl₃, TMS) δ: 0.91 (6H, s, 3,5-CH₃); 1.23 (6H, s, 3,5-CH₃); 1.0-1.6 (7H, m, ring protons and OH); 1.6-1.8 (2H, m, PhCH₂CH₂); 2.6 -2.8 (2H, m, PhCH₂CH₂) and 7.0-7.4 ppm. (5H, m, Ph).

### b) 2-Chloro-N-[3,3,5,5-tetramethyl-1-(2-phenylethyl)cyclohexyl]acetamide (21-1).

Prepared in 96% yield from cyclohexanol **20-1** according to the procedure described in **Example 25c.** A colorless oil. ¹H-NMR (CDCl₃, TMS) δ: 0.93 (6H, s, 3,5-CH₃); 1.17 (6H, s, 3,5 -CH₃); 1.0-1.5 (4H, m, 4-CH₂, 2,6-CH); 2.0-2.2 (2H, m, PhCH₂CH₂); 2.24 (2H, d, 14 Hz, 2,6 -CH); 2.5-2.6 (2H, m, PhCH₂CH₂); 3.90 (2H, s, CH₂Cl); 6.60 (1H, br s, NH) and 7.1 -7.3 ppm (5H, m, Ph).

### c) 3-{1-[(2-Chtoroacetyl)amino]-3,3,5,5-tetramethylcyclohexyl}propanoic acid (22-1).

Prepared 53% yield from amide **21-1** in according to the procedure described in **Example 26a.** A colorless crystals with mp 130-131°C. ¹H-NMR (CDCl₃, TMS) δ: 0.92 (6H, s, 3,5-CH₃); 1.17 (6H, s, 3,5 -CH₃); 1.0-1.5 (4H, m, 4 -CH₂, 2,6-CH,); 2.0-2.4 (6H, m, OCCH₂CH₂, 2,6-CH); 3.97 (2H, s, CH₂Cl) and 6.6 ppm (1H, br s, NH);

### d) Ethyl 3-{1-[(2-chtoroacetyl)ammo]-3,3,5,5-tetramethytcydohexyl}propanoate (23-1).

Prepared in 82% yield from acid **22-1** according to the procedure described in **Example 26b.** An oil. ¹H-NMR (CDCl₃, TMS) δ: 0.91 (6H, s, 3,5 -CH₃); 1.14 (6H, s, 3,5-CH₃); 1.25 (3H, t, 7 Hz, CH₃CH₂O); 0.8-1.6 (4H, m, 4-CH₂, 2,6-CH); 2.0-2.4 (6H, m, OCCH₂CH₂, 2,6-CH); 3.95 (2H, s, CH₂Cl); 4.11 (2H, q, 7 Hz, CH₃CH₂O) and 6.50 ppm (1H, br s, NH).

### e) 7,7,9,9 -Tetramethyl-1-azaspiro[4.5]decan-2-one (24-1).

Prepared in 54% yield from ester 23-1 according to the procedure described in **Example 26c.** A colorless solid with mp 158-160°C. ¹H-NNM (CDCl₃, TMS) δ: 1.01 (12H, s, 7,9-CH₃); 1.19 (1H, d, 14 Hz, 8-CH); 1.27 (1H, d, 14 Hz, 8 - CH); 1.45 (4H, s, 6,10 -CH₂); 2.02 (2H, t, 7.5 Hz, 4 - CH₂); 2.36 (2H, t, 7.5 Hz, 3 -CH₂) and 5.8 ppm (1H, br s, NH).

### f) 7,7,9,9-Tetramethyl -1-azaspiro[4.5]decane hydrochloride (3 -1).

Prepared in 76% yield from spirolactam **24-1** according to the procedure described in **Example 26d.** Colorless solid. ¹H-NMR (CDCl₃, TMS) δ: 1.01 (6H, s, 7,9-CH₃); 1.08 (6H, s, 7,9 -CH₃); 1.23 (1H, d, 14 Hz, 8-CH); 1.35 (1H, d, 14 Hz, 8 - CH); 1.8 (4H, br s, 6,10 -CH₂); 2.0-2.2 (4H, m, 3,4 -CH ₂); 3.3 (2H, br s, 2 -CH₂) and 9.4 ppm (2H, br s, NH ₂⁺).

### Synthesis Example 28.

### 8,8,10,10-Tetramethyl-1-azaspiro[5.5]undecane hydrochloride (3-2).

### a) 3,3,5,5-Tetramethyl-1-(3-phenylpropyl)cyclohexanol (20-2).

Prepared in 90 % yield from ketone **19** according to the procedure described in **Example 27a.** A colorless oil. ¹H-NMR (CDCl₃, TMS) δ: 0.86 (6H, s, 3,5 -CH₃); 1.19 (6H, s, 3,5 -CH₃); 1.0-1.8 (11H, m, ring protons, OH and PhCH₂CH₂CH₂); 2.60 (2H, t, 7.5 Hz, PhCH₂CH₂CH₂) and 7.1-7.4 ppm (5H, m, Ph).

### b) 2-Chloro-N-[3,3,5,5-tetramethyl-1-(3-phenylpropyl)cyclohexyl]acetamide (21-2).

Prepared in 37% yield from cyclohexanol **20-2** according to the procedure described in **Example 25c.** Colorless solid with mp 83-85°C. ¹H-NMR (CDCl₃, TMS) δ: 0.89 (6H, s, 3,5 -CH₃); 1.13 (6H, s, 3,5 -CH₃); 0.9-1.9 (8H, m, 4 -CH₂, 2,6 -CH and PhCH₂CH₂CH₂); 2.15 (2H, d, 14.5 Hz, 2,6 -CH); 2.56 (2H, t, 8 Hz, PhCH₂CH₂CH₂); 3.93 (2H, s, CH₂Cl); 6.5 (1H, br s, NH) and 7.1 -7.4 ppm. (5H, m, Ph). **c) 4-{1-[(2-Chtoroacetyl)amino]-3,3,5,5-tetramethylcyclohexyl}butanoic acid (22-2).**

Prepared in 74% yield from amide **21-2** according to the procedure described in **Example 26a.** Colorless solid with mp 140-141°C. ¹H-NMR (CDCl₃, TMS) δ: 0.91 (6H, s, 3,5-CH₃); 1.15 (6H, s, 3,5 -CH₃); 0.9-1.8 (8H, m, 4 -CH₂, 2,6 -CH, OCCH₂CH₂CH₂); 2.17 (2H, d, 14.2 Hz, 2,6 -CH); 2.33 (2H, t, 7.2 Hz, OCCH₂CH₂CH₂); 3.97 (2H, s, CH₂ Cl) and 6.6 ppm. (1H, br s, NH).

### d) Ethyl 4-{1-[(2-chloroacetyl)amino]-3,3,5,5-tetramethylcyclohexyl}butanoate (23-2).

Prepared in 98% yield from acid **22-2** according to the procedure described in **Example 26b.** A colorless oil. ¹H-NMR (CDCl₃, TMS) δ: 0.91 (6H, s, 3,5-CH₃); 1.14 (6H, s, 3,5 -CH₃); 1.25 (3H, t, 7 Hz, CH₃ CH₂ O); 0.9-1.8 (4H, m, 4-CH₂, 2,6-CH, OCCH₂CH₂CH₂); 2.18 (2H, d, 15 Hz, 2,6 -CH); 2.26 (2H, t, 8.4 Hz, OCCH₂CH₂CH₂); 3.95 (2H, s, CH₂Cl); 4.13 (2H, q, 7 Hz, CH₃CH₂O) and 6.52 ppm. (1H, br s, NH).

### e) 8,8,10,10-Tetramethyl-1-azaspiro[5.5]undecan-2-one (24-2).

Prepared in 76% yield from ester **23-2** according to the procedure described in **Example 26c.** Colorless solid with mp 126-128°C. ¹H-NNM (CDCl₃, TMS) δ: 1.01 (6H, s, 8,10 -CH₃); 1.09 (6H, s, 8,10 -CH₃); 1.19 and 1.30 (both 1H, d, 14 Hz, 9 -CH₂); 1.39 and 1.46 (both 2H, d, 14 Hz, 7,11 -CH₂); 1.63-1.90 (4H, m, 4,5 - CH₂); 2.33 (2H, t, 6 Hz, 3 -CH₂) and 5.8 ppm. (1H, br s, NH).

### f) 8,8,10,10-Tetramethyl-1-azaspiro[5.5]undecane hydrochloride (3-2).

Prepared in 45% yield from spirolactam **24-2** according to the procedure described in **Example 26d.** A colorless solid. ¹H-NMR (CDCl₃, TMS) δ: 1.01 (6H, s, 8,10 -CH₃); 1.09 (6H, s, 8,10 -CH₃); 1.0-2.1 (12H, m, 3,4,5,7,9,11 -CH₂); 3.1 (2H, br s, 2 -CH₂) and 9.1 ppm. (2H, br s, NH₂⁺).

### Synthesis Example 29.

### 8,10,10-Trimethyl-6-azatricyclo[6.3.1.0^{1,6}]dodecane hydrochloride (2).

### a) 8,10,10-Trimethyl-6-azatricyclo[6.3.1.0^{1,6}]dodecan-5-one (25).

Prepared in 20% yield from spirolactam **24-2** according to the procedure described in **Example 20b.** An oil. ¹H-NMR (CDCl₃, TMS) δ: 0.95 (3H, s, 10 - CH₃); 1.00 (3H, s, 10 -CH₃); 1.08 (3H, s, 8 -CH₃); 1.20 (1H, d, 12 Hz) and 1.25 -1.70 (5H, m, 9,11,12 -CH₂); 1.75-1.90 (4H, m, 2,3 -CH₂); 2.25-2.40 (2H, m, 4 -CH₂); 3.14 and 3.43 ppm (both 1H, d, 12.0 Hz, 7 -CH₂). **b) 8,10,10-Trimethyl-6-azatricyclo[6.3.1.0^{1,6}]dodecane hydrochloride (2).**

Prepared in 36% yield from lactam **25** according to the procedure given in **Example 26d.** A colorless solid. ¹H-NMR (CDCl₃, TMS) δ: 0.85-2.45 (12H, m, 2,3,4,9,11,12 -CH₂); 0.99 (3H, s, 10 -CH₃); 1.05 (3H, s, 10 -CH₃); 1.19 (3H, s, 8 -CH₃); 3.12 (2H, m, 5 -CH₂); 3.20-3.75 (2H, m, 7-CH₂) and 9.05 ppm. (1H, br s, NH ⁺).

### B. THERAPY EXAMPLES

The following Therapy Examples illustrate the invention without limiting its scope.

### THERAPY EXAMPLE 1: Memantine in Combination with Acetylcholinesterase Inhibitors is Well Tolerated and Effective in Laree-Scale Human Clinical Trials

The present inventor conducted a study among German physicians. The study captured 158 demented patients with a mean age of 74 years, who were treated with memantine in combination with an AChEI. Memantine was prescribed at a wide range of daily doses (5-60 mg/day, mean 20 mg/day) and was combined with an AChEI, in 84% of the cases donepezil. Combination therapy was well tolerated for nearly all patients (98%), within an average observation period of 4 months at stable doses. No serious adverse events or changes in blood chemistry were experienced by most patients (96% and 80%, respectively); all 6 adverse events reported resolved without sequelae and without discontinuation of either drug. Global clinical status of most patients improved (54%) or remained stable (39%). These data indicated that the comedication of memantine with AChEIs is safe, well tolerated, and effective.

### Methods

The following drugs were used in the studies: Memantine (AXCTRA®, Merz Pharmaceuticals GmbH, Frankfurt, Germany; former brand name AKATINOL®); Donepezil (ARICEPT®, Eisai GmbH, Frankfurt and Pfizer GmbH, Karlsruhe, Germany); Rivastigmin (EXELON®, Novartis Pharma GmbH, Nürnberg, Germany); Tacrin (COGNEX®, OTL Pharma, Paris, France).

This study was conducted in accordance with applicable regulatory law in Germany. The study was administered through private practices and memory clinics in Germany. The information solicited included basic demographics, dosing, global clinical rating, tolerability, laboratory chemistry, and changes in concomitant medication. Data were collected only for patients titrated up to their individual best dose of both drugs, and on stable daily doses for at least 4 weeks. For most of the patients, the 4 months observation period started with the initiation of the comedication. Variables, collected at the beginning and at the end of the observation interval, were statistically evaluated by analyses of clinical impression and pre/post change assessment.

Of the 158 patients surveyed, 81 (51 %) were female and 69 (44%) were male; gender information was missing for 8 patients. Age ranged from 26 to 100 years and averaged 74 years. The 26 year-old patient was treated for organic cognitive impairment not further specified. Diagnoses [also given as ICD 10 codes] included Alzheimer's disease (AD) [F 00 and G 30] (121 patients, 77%), vascular dementia [F 01] (14 patients, 9%), unspecified dementia [F 03] (14 patients, 9%), degenerative nervous system disease [G 31] (2 patients, 1%), and dementia secondary to other diseases [F 02 and G 04] (2 patients, 1%); diagnosis was unspecified for 5 patients (3%). Many patients presented with no concomitant disease (43 patients, 27%), but circulatory system disease was commonly reported (58 patients, 37%).

Daily memantine dose ranged from 5 to 60 mg; the median dose was 20 mg/day as recommended (72, 46%). By far the most patients (132, 84%) received concomitantly donepezil; rivastigmine and tacrine were other AChEIs administered; galantamine was not fully marketed at the time; the AChEI was unspecified by the physician for 2 patients (Table 1).

**Table 1: Daily Doses of Memantine and AchEIs**

| **Memantine (mg/day)** | **n(%)** | **AChEI** | **n(%)** | **Dose (mg/day)/Range** |
|---|---|---|---|---|
| 5 | 7(4) | Doneprezil | 132(84) | 10.0 / 2.5 - 10 |
| 10 | 47(30) | Rivastigmine | 23(15) | 4.5 / 1.5 - 12 |
| 15 | 13(8) | Unspecified | 2(1) | 10.0 / 10 - 10 |
| 20 | 72(46) | Tacrine | 1(<1) | 160.0 / 160 - 160 |
| 30 | 12(8) | | | |
| 40 | 6(4) | | | |
| 60 | 1(<1) | | | |

| | | | | |
|---|---|---|---|---|
| Doses of AChEI are median. Abbreviations: n = number of patients | | | | |

At the reference date fixed for the statistical analyses, treatment duration was calculated as median; memantine has been administered for a median of 0.5 years (0.09-7.27, n=157) and AChEIs for a median of 0.6 years (0.1-4.99, n= 141). For 140 patients, the start sequence for the therapy with memantine and AChEIs, respectively, were given (Table 2).

**Table 2: Therapy sequence**

| **Therapy sequence** | **n (N = 140)** | **%** |
|---|---|---|
| Memantine after AChEI | 70 | 50 |
| Contemporaneous Start memantine and AChEI | 11 | 8 |
| Memantine before AChEI | 59 | 42 |

### Results

Physician rating of tolerability of the comedication was "very good" for most (89, 56%) patients and "good" for most of the remaining patients (66, 42%). Two tolerability assessments were missing and tolerability was judged "poor" for 1 patient. Out of 158 patients on combination therapy, 6 experienced adverse events; all adverse events resolved without sequelae and without drug discontinuation. Five of the 6 adverse events were reported during combination of memantine and donepezil while one occurred following comedication of memantine and rivastigmine. Out of these 6 adverse events, 2 were considered possibly and probably related to memantine (mild severity); one was considered probably related to donepezil (moderate severity), and for the rest no causality assessment was given. None of the adverse events were rated severe or unexpected.

To determine the efficacy of the combination therapy, the documentation form included a 4-point categorical scale for the assessment of a global clinical impression by the physician (very good/good/bad/worse). This assessment was performed for 155 patients. The vast majority of patients was rated either improved (84, 54%) or stablilized (60, 39%). For the remaining 11 patients, clinical status worsened (9, 6%) or their status was unspecified (2, >1%). In the non-operationalized free comment section of this observational form, physicians often added descriptions relating to improved communicative abilities and elevated mood.

### Discussion

Currently, there are two approved therapeutic principles available for AD: AChE inhibition or NMDA receptor antagonism. Given the nature of AD affecting various neurotransmitter systems, and with supporting data from this study, the present inventors hypothesized that the optimal pharmacotherapy and clinical efficacy can be enhanced by combination of several approaches. Prior to this study, the effectiveness, safety and tolerability of the combined intervention in humans involving both therapeutic agents were unknown.

AChEls promote cholinergic transmission and several are approved for mild to moderate AD (Farlow et al., Arch. Neurol., 2001, 58:417-422; Knapp et al., JAMA, 1994, 271:985-991; Mohs et al., Neurology, 2001, 14:481-488; Zurad, Drug Benefit Trends, 2001, 13:27-40). Memantine is assumed to reduce glutamate induced neuronal excitotoxicity and is symptomatically effective also in advanced AD (Winblad and Poritis, 1999, *supra*). It has recently obtained a positive opinion by the CPMP for European Union approval. Memantine has previously been on the German market for many years in a so-called "dementia syndrome" indication, which includes Alzheimer's disease and other dementias

The present study provides the first rational combination pharmacotherapy in AD by demonstrating for the first time the beneficial effect of combining memantine with AChEIs. More than half of the surveyed patients were rated by their physicians as clinically improved. This type of result has never been observed before. Previous therapies had achieved only a slowing down of deterioration. The present theraphy achieved significantly slower progression in certain symptoms and improvement (i.e., reversal of deterioration) in others. In addition, the clinical data reported herein shows the absence of any severe adverse events or drug reaction: a vast proportion of patients tolerated the combination well. In contrast to a controlled experimental setting, the present findings are based on real life conditions with a remarkably wide dosing range of memantine, in some instances well beyond the recommended 20 mg daily dose; the actually prescribed AChEI daily doses on the other hand appeared to range rather in the lower end of the dosage ranges for these substances, particularly for rivastigmine.

Encouraged by these results, the inventors reasoned that a therapy with memantine combined with a AChEI might slow down the disease progression by providing neuroprotection from excitotoxicity and enhance cognitive performance by improving both glutamatergic and cholinergic neurotransmission (Jacobson, Evidence Based Mental Health, 1999, 2:112-113; Parsons *et al.,* 1999, *supra*; Danysz *et al.,* 2000, *supra*).

### THERAPY EXAMPLE 2: The Use of Cell Cultures and Animal Models of Alzheimer's Disease in Evaluating Various Parameters of 1-aminocyclohexane Derivative/AChEI Combination Therapy

The clinical signs of AD in humans result from selective degeneration of neurons in brain regions critical for memory, cognitive performance and personality. Dysfunction and death of these neurons lead to reduced numbers of synaptic markers in their target fields; the disruption of synaptic communication is manifested by mental impairments and, finally, severe dementia.

Two types of protein aggregates found in the brain are a pathological hallmark of AD: intracellular neurofibrillary tangles and extracellular amyloid plaques (for a recent review *see* Wong et al., Nature Neurosci., 2002, 5: 633 -639). Both tangles and plaques are preferentially localized to the cortex, hippocampus and amygdala. Neurofibrillary tangles are inclusions located within cell bodies and proximal dendrites, and within filamentous swellings in distal axons and synaptic terminals. Hyperphosphorylated isoforms of the microtubule-associated protein *tau,* which assemble into poorly soluble paired helical filaments, are a central feature of these neurofibrillary tangles (Goedert et al., Curr. Opin. Neurobiol., 1998, 8: 619-632). The extracellular plaques result from elevated levels of an approximately 4.2 kilodalton (kD) protein of about 39-43 amino acids designated the β-amyloid peptide (βAP) or sometimes Aβ, AβP or β/A4 (*see, e.g.,* Glenner and Wong, Biochem. Biophys. Res. Commun., 120:885- 890, 1984; U.S. Patent No. 4,666,829). Molecular biological and protein chemical analyses have shown that βAP is a small fragment of a much larger precursor protein, referred to as the β-amyloid precursor protein (APP). APP is a type I transmembrane protein normally expressed in many different cell types, but particularly abundant in neurons. βAP monomers form oligomers and multimers, which assemble into protofilaments and then fibrils. Eventually, βAP fibrils are deposited as the amyloid cores of neuritic or senile plaques (amyloidosis), which are complex structures also containing dystrophic neurites, astrocytes and microglia.

Pathogenic βAP peptides are generated via cleavage of APP by three different proteases, termed α-, β- and γ-secretases. The α-secretase cleaves APP within Aβ to yield the secreted derivative, sAPPα, which precludes Aβ formation. In contrast, cleavages of APP by β- and γ-secretases result in βAP production leading to amyloid depositions (*see* Wong *et al.,* 2002, *supra*).

In some individuals with early-onset AD, the illness may be inherited as an autosomal dominant (*i.e.,* only a single copy of the mutant gene is necessary to cause the disease). Such mutations are identified in at least three different genes: APP, presenilin 1 (PS1) and presenilin 2 (PS2) (Price et al., Annu. Rev. Genet., 1998, 3 2: 461-493; Hardy et al., Science, 1998, 282: 1075 -1079; Tanzi, Neuron, 2001, 32: 181 - 184; Selkoe, *ibid.,* pp. 177-180; Sherrington et al., Nature, 1995, 375: 754 -760; Levy-Lahad et al., Science, 1995, 269: 973 -977; Rogaev et al., Nature, 1995, 376: 775-778 ).

A variety of APP mutations reported in cases of FAD (familial AD) are near cleavage sites involved in formation of βAP (*see, e.g.,* Goate et al., 1991, Nature, 349:704-706; Harlan et al., 1991, Nature, 353:844 -846; Murrell et al., 1991, Science, 254:97-99; and Mullan et al., 1992, Nature Genet., 1:345 -347). The APP 717 mutation is located near the C-terminus of βAP and facilitates β-secretase activity, leading to increased secretion of the longer and more toxic βAP peptide, βAP₄₂. This longer βAP₄₂ peptide is thought to promote the formation of βAP aggregates and amyloid plaques. The APPswe mutation, a double mutation at the N-terminus of βAP, enhances BACE1 cleavage and is associated with elevated levels of βAP peptides, including βAP₄₂. In contrast, APP mutations within the βAP peptide domain (for example, APP-E693Q, A692G or E693 G) do not elevate the level of βAP but may cause amyloidosis by increasing βAP oligomer or protofibril formation.

PS1 and PS2 encode highly homologous 43- to 50-kD multipass transmembrane proteins that are processed to stable N-terminal and C-terminal fragments, and are widely expressed but at low abundance in the central nervous system. PS1 influences APP processing (Borchelt et al. Neuron, 1997, 19: 939-945; Wong *et al.,* 2002, *supra*). The PS1 gene has been reported to harbor more than 80 different FAD mutations (*see* AD mutation database, http://molgen-www.uia.ac.be), whereas only a small number of mutations have been found in PS2 -linked families. The vast majority of abnormalities in PS genes are missense mutations that result in single amino acid substitutions, which in general seem to influence secretase activity and increase the generation of the βAP₄₂ peptide.

One of the current therapeutic strategies in AD is a reduction in the levels of the toxic βAP.

There is accumulating evidence supporting that AChEIs can affect APP processing. For example, tacrine has been shown to reduce the release of the secreted form of APP, sAPPα, and total βAP, βAP₄₀ and βAP₄₂ in human neuroblastoma cells in the absence of any detectable cellular damage or toxicity (Lahiri et al., Mol. Brain Res. 1998, 62: 131-140).

NMDA receptors have been also implicated in the signalling cascades affecting or affected by APP processing. Thus, in cultured hippocampal neurons, sAPPα, has been shown to selectively suppress NMDA-mediated currents (Furukawa and Mattson, Neurscience, 1998, 83: 429-438). The present inventor and co-workers have studied the effect of memantine (NMDA receptor antagonist) on the processing of APP in cultured human neuroblastoma cells SK-N-SH. Cells were treated with memantine (1-4 µM) or vehicle, and the levels of sAPP and βAP₄₀ in the conditioned media and the total intracellular APP were measured by Western immunoblotting or ELISA using specific antibodies. The results indicate that the treatment of human neuroblastoma cells with therapeutic concentrations of memantine (1-4 µM) results in a decrease in sAPP and βAP₄₀ levels in the condition media without affecting the levels of total intracellular APP. Cell viability and toxicity, as determined by MTT and LDH assays, were not affected by memantine at the above concentrations. The observed decrease in sAPP and βAP₄₀ levels occurring without a concomitant increase in the cellular APP levels by memantine suggests that memantine may potentially inhibit the amyloidogenic (amyloid formation) pathway. Therefore, it appears that memantine has the potential to decrease the deposition of fibrillogenic Aβ peptides in the brain.

The present inventors have also decided to determine the effects of administering various concentrations of the combination of 1-aminocyclohexane derivative (*e.g.*, memantine or neramexane) and AChEI (*e.g.*, galantamine, tacrine, donepezil, or rivastigmine) on the secretion and processing of various APP derivatives (sAPPα, and total βAP, βAP₄₀ and βAP₄₂) *in vitro* in a cell culture.

Suitable cell lines include human and animal cell lines, such as human neuroblastoma cell lines (*e.g.*, SK-N-SH), human neuroglioma cell lines, human HeLa cells, human kidney cell line HEK-293, primary human endothelial cells (*e.g.*, HUVEC cells), primary human fibroblasts or lymphoblasts, primary human mixed brain cells (including neurons, astrocytes, and neuroglia), Chinese hamster ovary (CHO) cells, and the like. Preferred for use according to the present invention are human cell lines that express APP variants or that overproduce βAP, *e.g.*, APP variants having one or several amino acid substitutions directly at the N-terminus of the βAP cleavage site (*e.g.*, K293 cells which express an APP DNA bearing a double mutation [Lys⁵⁹⁵ →Asn⁵⁹⁵ and met⁵⁹⁶→Leu⁵⁹⁶] found in a Swedish FAD family, which produce approximately six-to-eight-fold more βAP than cells expressing normal APP, as disclosed in the U.S. Patent No. 6,284,221).

The concentrations of 1-aminocyclohexane derivative and AChEI resulting in therapeutically meaningful decrease in the processing and/or secretion of the amyloidogenic βAP in cell cultures are further tested *in vivo* by monitoring of βAP levels in transgenic animal models of AD, such as the mouse animal models expressing APP minigenes that encode FAD-linked APP mutants (*e.g.*, swe or 717, as disclosed, *e.g.*, in U.S. Patent No. 5,912,410) or the double mutant mouse model descibed by Borchelt et al. (Neuron, 19: 939-945, 1997). The latter transgenic mice coexpress an early-onset familial AD (FAD)-linked human presenilin 1 (PS1) variant (A246E) and a chimeric mouse/human APP harboring mutations linked to Swedish FAD kindreds (APPswe). These mice develop numerous amyloid deposits much earlier than age-matched mice expressing APPswe and wild-type human PS1. Expression of APP minigenes that encode FAD-linked APP mutants and, in particular, co-expression of the mutant human PS1 A246E and APPswe elevates levels of βAP in the brain, and these mice develop numerous diffuse βAP deposits and plaques in the hippocampus and cortex (Calhoun et al., Proc. Natl. Acad. Sci. USA, 1999, 96: 14088-14093). These plaques contain neurites (some showing hyperphosphorylated *tau* immunoreactivity), astrocytes and microglia; however, neurofibrillary tangles are not present. To obtain mice with both plaques and tangles, mutant APP transgenic mice can be mated to mice expressing the P301L *tau* mutant (Lewis et al., Science, 2001, 293: 1487-1491), a mutation linked to familial frontotemporal dementia with parkinsonism (FTDP). These and other transgenic animal models of AD are characterized by various cognitive defects such as loss of neurons, learning deficits, problems in object recognition memory, and problems with alternation-spatial reference and working memory (Chen et al., Nature, 2000, 408: 975 -979). Improvement of such defects is also used to assess the effectiveness of the combination therapy of the invention (including the determination of additive and synergistic effects).

Specifically, four groups of transgenic animals are being studied: control group I receives no treatment, control group II receives the 1-aminocyclohexane derivative (such as memantine or neramexane), control group III receives AChEI (such as galantamine, tacrine, donepezil, or rivastigmine), and experimental group IV receives a combination treatment of 1-aminocyclohexane derivative and AChEI. Drug administration is carried on over defined periods of time and is followed by testing, *e.g.*, (i) learnig abilitites, (ii) memory, (iii) the level of βAP₄₀ or βAP₄₂ fragment in the body fluids, (iv) the amount of β-amyloid plaques within the brain, and (v) hyperphosphorylated *tau* immunoreactivity within the brain. The improvement in either of the first two criteria and decrease in either of the last three criteria in the experimental grop IV (as compared to control groups) is used as a measure of the effectiveness of the combination therapy of the invention. The transgenic animal models are further used to determine the optimal dosages, efficacy, toxicity as well as side effects associated with the combination therapy of the invention.

### THERAPY EXAMPLE 3: A Study of the Pharmacokinetic Interaction of Memantine and ARICEPT^{®} in Healthy Young Subjects Introduction

After oral administration in man, memantine has been shown to be completely absorbed (absolute bioavailability of approximately 100%). After administration of ¹⁴Cmemantine, 84% of the dose was recovered, mainly in the urine. The time to maximum plasma concentrations (Tₘₐₓ) following oral doses of 10 to 40 mg memantine ranges between 3 and 8 hours. Peak plasma concentrations (Cₘₐₓ) after a single 20 mg oral dose ranges between 22 and 46 ng/mL. The AUC and Cₘₐₓ values of memantine increase proportionally with dose over the dose range of 5 to 40 mg. The half-life of memantine is approximately 60-80 hours. *In vitro* studies have shown a low potential of drug interactions between memantine and drugs metabolized via the cytochrome P450 system.

Donepezil HCl (ARICEPT^{®}) is a piperidine-based, specific inhibitor of acetylcholinesterase (AChE) that is currently approved in the United States and elsewhere for the treatment of mild to moderate Alzheimer's disease (AD). The pharmacokinetics of donepezil after oral administration are linearly proportional to dose. Following a 5 mg/day dose of donepezil for 28 days in healthy subjects, peak plasma concentrations (Cₘₐₓ = 34.1 ng/mL) are obtained at approximately 3 hours (Tiseo et al., Br. J. Clin. Pharmacol., 46 (Suppl. 1): 13 -18, 1998). Donepezil has a half-life of approximately 70 hours.

The present inventors set out to determine (i) whether there is an *in* vivo pharmacokinetic interaction between memantine and donepezil and (ii) whether co-administration of memantine affects the ability of donepezil to inhibit AChE activity.

### Study Design

### Subject Population:

The trial was undertaken after informed written consent. Twenty four (24) young healthy subjects, (16 males and 8 females) were enrolled. The mean age, weight and height was 27.6 years (18-35 years), 73.6 kg and 171.4 cm, respectively. Six subjects were white and 18 were non-caucasian (black). A normal physical examination, vital signs (diastolic and systolic blood pressure, pulse rate, respiration rate, temperature and body weight), serum chemistry, hematology, urinalysis, negative Anti-HIV 1 and 2, HbsAg, anti-HCV and VDRL/RPR were required for enrollement and completion. Female subjects had to have a negative serum pregnancy test prior to the start of the study and a negative urine pregnancy test on Day 1, and to have been using a medically accepted method of birth control (not including oral contraceptives) for at least 30 days prior to screening and had to continue using it throughout the study. The subjects were non-smoking (have not smoked within the past 2 years).

Nineteen subjects completed the study. Subjects 8, 12, and 21 withdrew consent, subject 9 was excluded for noncompliance, and subject 22 was lost to follow up.

### Study Procedures

The subjects received one 10 mg memantine tablet on Study Day 1. A blood sampling profile for memantine was obtained starting on Day 1 followed by a 14-day washout period. Beginning on Day 15, subjects took one 5 mg ARICEPT^{®} tablet once daily for 7 days. Beginning on Day 22, subjects took two 5 mg ARICEPT^{®} tablet (10 mg donepezil) once daily for 22 days. On Day 42, subjects had a blood draw taken prior to the donepezil dose. Blood samples for the pharmacokinetic profiling of donepezil concentration and red blood cells (RBC) AChE activity were obtained. On Day 43, subjects received 10 mg memantine concomitantly with the last dose of 10 mg donepezil in the morning. Blood samples for the pharmacokinetic profiling of donepezil and memantine concentrations and the pharmacodynamic profiling of RBC AChE activity were obtained starting on Study Day 43.

### Blood Sample Collection and Processing

Forty three (43) plasma samples were collected during the study for pharmacokinetic and pharmacodynamic analyses. Blood samples for the determination of memantine concentration were collected following dosing on Day 1 at zero hour (pre-dose), as well as at 1, 2, 3, 4, 6, 8, 12, 24, 48, 72, 96, 120, 144, 168, and 192 hours post-dose. Blood samples for the determination of predose donepezil concentration were collected from each subject at 0 hour on Days 15, 40, and 41. Blood samples for the determination of donepezil concentration were collected on Day 42 at: 0 hr (predose), 1, 2, 3, 4, 6, 8 and 12 hours post-dose. Blood samples for the determination of donepezil and memantine concentrations were collected on Day 43 at 0 hr (pre-dose), as well as at 1, 2, 3, 4, 6, 8, 12, and 24 hours post-dose. Additional blood samples for determination of memantine concentrations, following dosing on Day 43, were collected at 48, 72, 96, 120, 144, 168, and 192 hours post-dose. Blood samples drawn on Day 15 (0 hr), Day 42 (0, 1, 2, 3, 4, 6, 8, and 12 hours) and Day 43 (0, 1, 2, 3, 4, 6, 8, 12, and 24 hours) were also used for the determination of AChE activity in RBCs.

*Blood Sample Processing for Memantine and Donepezil:*
Approximately 7 mL of blood were collected directly into prechilled 7 mL or 10 mL green top Vacutainer^{®} tubes (containing sodium heparin). Blood samples were centrifuged within 30 minutes from the time of draw at 2,500 g for 10 minutes at 4°C and the plasma was harvested and transferred into pre-chilled, coded polypropylene tubes. The samples were then flash frozen in an isopropyl alcohol/dry ice bath and stored in a 70°C freezer.

*Blood Sample Processing for RBC AChE Activity*: From the blood residue remaining after harvesting of the plasma on Days 15, 42 and 43, the buffy coat portion (upper portion of the centrifuged blood that remained between the RBCs and plasma) was removed and discarded. The remaining RBCs were placed into screwcap polypropylene tubes, flash frozen in an isopropylene alcohol bath and placed in a 70°C freezer. RBC samples had to be frozen within 20 minutes of blood draw.

### Analytical Procedures

### Memantine

Memantine plasma concentrations were measured using a high performance liquid chromatographic separation using mass spectrometric detection method (LC/MS/MS). A [²H₆]memantine internal standard was used. Plasma samples were acidified with 70 pL of 0.01 N hydrochloric acid. 0.5 M sodium bicarbonate buffer was added, and the compounds were extracted with ethyl acetate. The organic layer was evaporated under vacuum at room temperature. The dry residue was analyzed after reconstitution in mobile phase. The components of the reconstituted samples were separated on a Zorbax SBC8 column (4.6 x 150 µm, 3.5gm) and detected by atmospheric pressure chemical ionization (APCI) with a selected reaction monitoring (SRM) in the positive ion mode. The SRM used precursor positive product ions of *m*/*z* 180 →163 and *m*/*z* 186 →169 to monitor memantine and its internal standard, respectively. The protonated molecular ions of memantine and [²H₆]memantine are the precursor ions for the SRM mode. The peak height ratio of memantine product ion to that of its internal standard was the response used for quantification. The precision and accuracy for memantine quality control samples in human plasma were within 7.7% and ranged between 0.4 to 6.9%, respectively (including outliers). The lower limit of quantitation was 0.5 ng/mL.

### Donepezil

The analytical method for the determination of donepezil in human plasma involved liquid/liquid extraction and LC/MS/MS.

### Acetylcholinesterase Inhibition Assay

A radioenzyme assay was used to determine the AChE activity in red blood cells (RBC) and to determine the inhibition of AChE activity by donepezil in RBC. Aliquots of control and study sample RBC homogenates were incubated with [³H]acetylcholine iodide, which was hydrolyzed by the AChE in the RBC sample. The enzyme reaction was stopped by addition of chloroacetic acid and the hydrolysis product, [³H]acetate, was extracted into the liquid scintillation cocktail and counted. The AChE activity (nmoles acetylcholine hydrolyzed per minute) was calculated based on counts per minute. Activity measurements on Day 15 (prior to the first donepezil dose) were set to 100% activity.

### Pharmacokinetic and Pharmacodynamic Data Analysis

### Pharmacokinetic Analysis

The following parameters were determined from the plasma concentrations of memantine following single dose administration of memantine alone and in combination with donepezil: the area under the plasma concentration versus time curve (AUC₀₋ₜ and AUC_{0-∞}), maximum plasma concentration (Cₘₐₓ), time of maximum plasma concentration (Tₘₐₓ), elimination halflife (T_{1/2}), mean residence time (MRT), oral clearance (CL/F) and apparent volume of distribution (Vz/F).

Following multiple dose administration of donepezil alone and in combination with memantine, the following parameters were determined from the plasma donepezil concentration data: Cₘₐₓ, Tₘₐₓ, AUC₀₋₂₄, and CL/F.

Maximum plasma concentrations (Cₘₐₓ) and the time of maximum concentration (Tₘₐₓ) for memantine and donepezil were determined by observation.

### Pharmacodynamic Analysis

RBC AChE activity was determined at baseline (prior to donepezil) and following administration of donepezil alone (Day 42) and with memantine (Day 43). The following pharmacodynamic parameters were determined from the AChE activity data: Aₘₐₓ, Aₘᵢₙ, AUC_{A}, %Inhibition, Iₘₐₓ and AUC_{I}.

AChE activity measured on Day 15, prior to the first donepezil dose, represented baseline activity values and was set to 100% activity.

Aₘₐₓ is the maximum AChE activity, expressed as a percent of baseline (control), observed during the 0-24 hour interval on Days 42 and 43.

Aₘᵢₙ is the minimum AChE activity, expressed as a percent of baseline, observed during the 0 -24 hour interval on Days 42 and 4 3.

AUC_{A} is the area under the AChE activity (% of baseline) versus time curve, from 0 -24 hours.

% Inhibition is the inhibition of AChE activity by donepezil expressed as a percent change from predose values:
Iₘₐₓ is the maximum percent inhibition observed during the 0 -24 hour interval on Days 42 and 43.

AUC_{I} is the area under the % Inhibition versus time curve calculated using the linear trapezoidal rule.

### Statistical Evaluations

Subjects who did not complete the study were not included in the evaluation of pharmacokinetic and pharmacodynamic parameters.

Statistical comparisons between treatments (drug in combination versus drug alone) with respect to all pharmacokinetic and pharmacodynamic parameters except Tₘₐₓ were performed using Analysis of Variance (ANOVA). The Tₘₐₓ parameter was compared using the Wilcoxon test.

The possibility of a pharmacokinetic and pharmacodynamic interaction between memantine and donepezil was evaluated by constructing two one-sided, 90% confidence intervals for the following primary pharmacokinetic and pharmacodynamic parameters:
Memantine Cₘₐₓ, AUC₀₋ₜ and AUC_{0-∞} parameters following administration of memantine concomitantly with donepezil versus memantine administered alone.
Donepezil Cₘₐₓ and AUC₀₋₂₄ parameters following administration of donepezil concomitantly with memantine versus donepezil administered alone.
AChE Iₘₐₓ and AUC_{I} parameters following administration of donepezil concomitantly with memantine versus donepezil administered alone.
Statistical inferences for PK and PD parameters were based on log-transformed values. Comparisons for T_{1/2} MRT and Tₘₐₓ were based on original data.

The absence of a pharmacokinetic and pharmacodynamic interaction between single dose memantine and multiple dose donepezil was concluded if the 90% confidence intervals for the log-transformed primary PK and PD parameters were within the range of 80% to 125%.

Evaluation of steady-state for donepezil was performed by linear regression of the pre-dose donepezil concentrations on Days 40, 41, and 42. Attainment of steady-state was concluded if the p value for the slope of the regression line was greater than 0.05.

### Results

**Extent of Exposure to Memantine and Donepezil**

| Drug | No. of Subjects | Total Subject Days | Mean Days |
|---|---|---|---|
| Memantine | 24 | 43 | 1.8 |
| Donepezil | 22 | 601 | 27.3 |

### Adverse Events

No subjects discontinued from the study due to an adverse event. There were no serious adverse events reported. Twenty (83.3%) of the twenty four subjects reported a total of 27 adverse events. The majority of the adverse events occurred when the subjects were receiving donepezil alone. The events were generally mild to moderate in severity. The most common adverse events (i.e., occurring in 3 or more subjects) were headache, nausea, fatigue, weakness, dizziness diarrhea, vomiting, and lightheadedness.

### Memantine Pharmacokinetic Parameters

The rate of memantine absorption following a single dose administration of 10 mg oral tablet was moderate with peak plasma concentrations achieved at 6.5 hours with or without donepezil. The maximum concentration of memantine (Cₘₐₓ) was similar when memantine was administered alone (12.8 ng/mL) and during coadministration with donepezil (13.0 ng/mL). Terminal half-life, MRT and volume of distribution values were similar following administration of memantine alone and with donepezil. Mean oral memantine clearance was decreased by 5.6 % during co-administration of memantine with donepezil. The 90% confidence intervals for the comparison of the logtransformed Cₘₐₓ, AUC₀₋ₜ and AUC_{0-∞} were within the range of 80-125% indicating that multiple daily dosing of 10 mg donepezil did not significantly alter the rate or extent of absorption of a single 10 mg memantine dose.

### Donepezil Pharmacokinetic Parameters

Attainment of steady-state following multiple dose administration of donepezil was evaluated by linear regression analysis of the pre-dose donepezil concentrations on Days 40, 41, and 42. Based on the p -value for the slope of the regression line, steady-state was attained in a total of 15 subjects (p > 0.05) but not in 4 of the subjects who completed the study (p<0.05). Subjects 1, 5, 6, and 17 had significant non zero slopes (p<0.05). The rate of donepezil absorption following once daily multiple dosing of 10 mg donepezil was moderate with peak plasma concentrations achieved at 3.4 and 3.3 hours without and with memantine, respectively. The mean maximum concentration of donepezil (Cₘₐₓ) was higher by 13% following administration of donepezil with memantine as compared to donepezil alone. Mean AUC₀₋₂₄ increased by 9% and CL/F decreased by 15% when donepezil was administered with memantine as compared to its administration alone.

The 90% confidence interval for the log-transformed AUC₀₋₂₄ was within the range of 80 -125% indicating that single dose administration of 10 mg memantine did not alter the extent of absorption of multiple dose donepezil. The 90% confidence interval for the logtransformed Cₘₐₓ was slightly outside the 80 -125% range (104.2 -126.5%).

After omitting from the statistical comparison of donepezil pharmacokinetic parameters data from the 4 subjects (Subjects 1, 5, 6, and 17) who did not reach steady-state (p < 0.05), the 90% confidence intervals for the comparison of the log-transformed Cₘₐₓ and AUC₀₋₂₄ were within the range of 80 -125%.

### AChE Measurements

The percent maximum inhibition of AChE activity from baseline value (Iₘₐₓ) averaged 77.8% and 81.1% with donepezil alone and with donepezil together with memantine, respectively. The 90% confidence intervals for the logtransformed Iₘₐₓ and AUC_{I} were within the range of 80 -125% indicating that inhibition of RBC AChE activity was not significantly altered by the co-administration of donepezil and memantine as compared to the administration of donepezil alone.

### Conclusion

In this study, single doses of 10 mg memantine alone and in combination with multiple daily doses of 10 mg donepezil were found to be safe and well-tolerated. No pharmacokinetic or pharmacodynamic interaction was observed between memantine and donepezil suggesting that these two drugs can be safely co-administered.

### THERAPY EXAMPLE 4: Evaluation of the Therapeutic Potential in the Treatment of Alzheimer's Disease of a Combination Therapy Comprising Memantine and Donepezil (ARICEPT®)

The goals of treatment for patients with Alzheimer's disease (AD) have been to improve or at least slow the loss of memory and cognition, and to maintain independent function. Symptomatic treatment for AD has focused on augmenting cholinergic neurotransmission as deterioration in cholinergic pathways occurs early and correlates with impairment of memory. Current approved drug therapy for mild - to-moderate AD includes several AChEIs (tacrine, donepezil, rivastigmine, and galantamine), which increase cholinergic neurotransmission by inhibiting the metabolism of acetylcholine via AChE. However, there are currently no approved treatments for the more severely ill AD patients in the US. An effective pharmacotherapy for the more advanced stages of AD, by slowing the rate of dise ase-related cognitive, functional, and global deterioration, would not only improve patient well being and quality of life but also result in improved quality of life for the caregivers and a decrease in the economic impact of the illness through delayed nursing home placement.

Memantine was previously marketed for the treatment of dementia, spasticity, and Parkinson's Disease in Germany and 41 other countries and was recently approved for the treatment of moderately severe to severe AD in all European Union countries. Presently, the only treatment approved for mild to moderate AD available in the US is monotherapy with AChEIs. Memantine, a moderate affinity, noncompetitive NMDA receptor antagonist, provides an additional therapeutic option for the treatment of the disease.

### Study Design

The present study was aimed at evaluating the therapeutic potential in treatment of AD of a combination therapy comprising memantine and donepezil (ARICEPT®). Specifically, the safety and efficacy of memantine (relative to placebo) was assessed in outpatients with probable moderate to severe dementia of the Alzheimer's type who were also receiving concurrent treatment with a stable dose of donepezil.

The study was multi-center, randomized, double-blind, parallel arm, and placebo-controlled. Inclusion criteria were: a diagnosis of probable AD by NINCDS-ADRDA criteria, a Mini Mental State Exam Score (MMSE) of 5 to 14, an MRI or CT scan consistent with the diagnosis of probable AD, and daily donepezil therapy for the past 6 months (stable dose for the past 3 months).

The study consisted of 1 week of single-blind placebo screening period followed by 24 weeks of double-blind treatment. Patients were randomized to 6 months of treatment with either memantine 20 mg per day (10 mg b.i.d.; titrated over a 4 week period) or placebo, in addition to their donepezil therapy. In order to evaluate the effect of treatment on cognition in dementia, patients were administered the Severe Impairment Battery (SIB) (Schmitt et al., Alzheimer Dis. Assoc. Dis., 11 [Suppl. 2]: S51-556, 1997), a performance-based objective cognitive assessment instrument with established sensitivity and validity, and a modified Alzheimer's Disease Cooperative Study Inventory-Activities of Daily Living (ADCS-ADL) (Galasko et al., Alzheimer Dis. Assoc. Dis., 11[Suppl. 2]: S33-S39, 1997), a measure of daily function. A physician's global assessment, the Clinician's Interview-Based Impression of Change-Plus (CIBIC-Plus) was also performed (Schneider et al., Alzheimer Dis. Assoc. Dis., 11 [Suppl. 2]: S22-S32, 1997).

### Study Population

403 patients were enrolled in this study with each of the double-blind treatment groups (memantine or placebo) containing approximately 200 patients. The study population consisted of male and female outpatients who were at least 50 years of age. AD severity ranged from moderate to severe assessed on the basis of Mini Mental State Examination Scores (MMSE) (≥ 5 and ≤ 14). For eligible patients, during the the Screening visit, the results of physical examination, laboratory evaluations and ECG were normal (or abnormal findings could be judged not clinically significant). All eligible patients had been receiving ongoing daily donepezil (ARICEPT^{®}) therapy for at least past 6 months, and at a stable dose (5-10 mg/day) for the last three months.

### Study Procedures

### Efficacy Assessments

*The Severe Impairment Battery (SIB) Test* was administered at each clinic visit starting at baseline. The SIB test evaluated cognition, *i.e.,* memory, language, praxis, orientation and attention. The test was scored 0-100 (with 100 being the worst result).

The *AD Cooperative Study - Activities of Daily Living (ADCS-ADL) Inventory* was used to measure functional capabilities of the study subjects. It consisted of 19 items appropriate for patients with moderate to severe dementia selected from the full 42-item inventory. Galasko et al., Alzheimer's Disease and Associated Disorders 11, Suppl. 2: S33, 1997. The ADCS-ADL was administered at each clinic visit starting at baseline.

*Clinician's Interview-Based Impression of Change-Plus Version (CIBIC-Plus)* is a global activities rating that is derived through an independent, comprehensive interview with the patient and caregiver by a clinician who is barred from knowledge of all other psychometric test scores conducted as part of the protocol. Using the results from baseline for reference, the clinician interviewed the patient and caregiver at the end of Weeks 4, 8, 12, 18 and 24 (or upon early termination), to obtain an "Impression of Change" rating. The format for this scale was derived from the Alzheimer's Disease Cooperative Study - Clinician's Global Impression of Change (ADCS-CGIC) (Schneider et al, Alzheimer's Disease and Associated Disorders, 1997, Vol. 11 (2): S22-S32).

*Resource Utilization in Dementia (RUD) Evaluation* is designed to assess caregiver burden for caregivers responsible for patients with AD (Wimo *et al,* Evaluation of the healthcare resource utilization and caregiver time in anti-dementia drug trials: a quantitative battery. *In:* The Health Economics of Dementia., eds. Wimo et al., Wiley press London, 1998). The assessment consisted of a structured interview with the caregiver of the study patient. The RUD had two parts: Part A was a questionnaire administered at Baseline, and Part B was a follow-up questionnaire. In addition to basic demographic information, the RUD asked the caregiver to recall significant health events that had occurred since the previous questionnaire was administered. It also questioned changes in time spent with the patient, changes in the caregiver's work status and changes in health care utilization. The RUD was conducted at Baseline, and at the end of Week 24 (or upon early termination).

*Functional Assessment Staging (FAST)* is designed to assess progressive functional deterioration in patients with AD (Reisberg, Psychopharmacol. Bull., 24: 653-659, 1988). It evaluates a patient's ability to perform daily and necessary life activities. The FAST is divided into seven major stages from "normal" (Stage 1) to "severe" (Stage 7). Stages 6 and 7 are further subdivided into 11 substages (6a to 6e and 7a to 7f). Each stage is based upon specific deficits in functional ability. The FAST was conducted at Baseline and at the end of Week 24 (or upon early termination).

*Behavioral Rating Scale for Geriatric Patients (BGP)* is an observerrated scale which is completed by the caregiver. This scale is adapted from the Stockton Geriatric Scale and has proven to be a reliable and valid method for measuring functional and behavioral disturbances in geriatric patients with dementia (Diesfeldt, Gerontologie, 11: 205-212, 1980). The scale consists of 35 items. The BGP was conducted at Baseline, and at the end of Week 24 (or upon early termination).

### Study Medication and Dosing Regimen

Memantine (5mg tablets) and placebo medication were supplied as film-coated tablets of identical appearance. Eligible patients were dispensed at baseline double-blind medication. During the first week of treatment, patients randomized to active treatment received 5mg/day of memantine, followed by 10mg/day during the second week and 15mg/day during the third week. Beginning at Week 4, the daily medication consisted of either four placebo tablets or four tablets of memantine. The target dose of 20mg/day was administered (10mg twice a day) starting with the fourth week of double-blind treatment and continued throughout the study. Throughout the double-blind treatment period, patients continued to take four tablets of medication daily.

### Statistical Evaluation

The primary efficacy parameters were the change from baseline in SIB and ADCS-ADL Inventory scores at Week 24.

The secondary efficacy objectives were to compare efficacy of memantine to placebo. The parameter was the CIBIC-plus rating at Week 24.

Comparisons between the memantine and placebo groups for primary efficacy parameters were made using a two-way analysis of covariance (ANCOVA). Primary efficacy analyses focused on the scores obtained at the end of Week 24, examining the change from baseline for the SIB and ADCS-ADL. For the CIBIC-Plus score, the Cochran-Mantel-Haenszel statistic using modified ridit scores (the van Elteren test) was applied to compare the distributions between memantine and placebo groups.

All efficacy analyses were based upon the randomized patients who took at least one dose of study medication and who had at least one post-baseline primary efficacy assessment. All statistical tests were two-sided, and a p value ≤ 0.05 was considered statistically significant. Primary analyses were performed on the ITT population using the Last Observation Carried Forward (LOCF) approach at Week 24. In these analyses, the last observed value before the missing value was carried forward to impute the missing value. The observed cases (OC) approach was used for supportive analyses, where only the observed values at each visit were used for analyses. The LOCF approach was also used at each visit for supportive analyses.

For the change from baseline in the total SIB and ADCS-ADL Inventory scores at Week 24, the comparison between memantine and placebo was performed using two-way analysis of covariance (ANCOVA) with treatment group and center as the two factors, and the baseline scores as covariate. Descriptive statistics were calculated by visit. The CIBIC-plus rating was analyzed using the CMH test, controlling for study center. Descriptive statistics were calculated by visit.

Statistical analyses were performed using SAS (version 6.12 or newer) under a UNIX operating system.

### Results

### Study Group

Of the 403 patients with moderate-to-severe AD, who were randomized and treated at 37 centers with 10 mg b.i.d. memantine (n=202) or placebo (n=201) in addition to their background donepezil (ARICEPT®) therapeutic regimen, 85% of memantine-treated patients and 75% of placebo-treated patients completed the trial. Adverse events were the most common reason for withdrawal, followed by withdrawal of consent.

Mean MMSE at entry was 10. There were no clinically important differences in patient demographic characteristics between the randomized treatment groups.

**Table 6. Patient Demographic and Baseline Characteristics**

| | | Placebo | Memantine | Total |
|---|---|---|---|---|
| | | (N=201) | (N=202) | (N=403) |
| MEAN AGE, years (SD) | | 75.5 (8.7) | 75.5 (8.5) | 75.5 (8.6) |
| SEX | | | | |
| | Male n (%) | 67 (33.3) | 74 (36.6) | 141 (35.0) |
| | Female n (%) | 134 (66.7) | 128 (63.4) | 262 (65.0) |
| ETHNICITY | | | | |
| | Caucasian n (%) | 186 (92.5) | 182 (90.1) | 368 (91.3) |
| | Non-Caucasian n (%) | 15 (7.5) | 20 (9.9) | 35 (8.7) |
| BASELINE MMSE, mean (SD) | | 10.2 (3.0) | 9.9 (3.1) | 10.1 (3.1) |

### Efficacy

At Week 24, a clinical and statistically significant superior efficacy for the combined memantine/donepezil treatment compared to donepezil alone was demonstrated on all three major study endpoints. Thus, patients treated with memantine and donepezil showed clinically and statistically significant improvement (p<0.001) in cognitive function (SIB) as compared to patients treated with donepezil and placebo (Figure 1), and showed significantly less decline (p=0.028) in daily function (ADCS-ADL) (Figure 2). A significant difference in favor of memantine/donepezil was also seen on the global assessment (CIBIC-Plus) (p=0.027; Figure 3).

### Safety

Combined memantine 20 mg/day (10 mg b.i.d.) and donepezil therapy was safe and well tolerated. In general, the incidence of treatment-emergent adverse events was similar in patients treated with memantine/donepezil or placebo/donepezil.

**Table 7. Adverse Events (>3% with memantine). Daily dose range of donepezil 5-10 mg and of memantine 5-20mg. Memantine maintenance dose 20 mg daily.**

| **Adverse event** | **Donep+Mem, n=202** | | **Donep+Plc, n=201** | |
|---|---|---|---|---|
| | **n** | **(%)** | **n** | **(%)** |
| Pat. with any AE | 158 | (78.2) | 144 | (71.6) |
| Agitation | 19 | (9.4) | 24 | (11.9) |
| Confusion | 16 | (7.9) | 4 | (2.0) |
| Fall | 15 | (7.4) | 14 | (7.0) |
| Influenza-like symptoms | 15 | (7.4) | 13 | (6.5) |
| Dizziness | 14 | (6.9) | 16 | (8.0) |
| Headache | 13 | (6.4) | 5 | (2.5) |
| Urinary tract infection | 12 | (5.9) | 10 | (5.0) |
| Urinary incontinence | 11 | (5.4) | 6 | (3.0) |
| Inflicted injury | 10 | (5.0) | 16 | (8.0) |
| Oedema peripheral | 10 | (5.0) | 8 | (4.0) |
| Upper resp. tract infection | 10 | (5.0) | 13 | (6.5) |
| Diarrhoea | 9 | (4.5) | 17 | (8.5) |
| Hypertension | 9 | (4.5) | 3 | (1.5) |
| Depression | 8 | (4.0) | 6 | (3.0) |
| Somnolence | 7 | (3.5) | 7 | (3.5) |
| Vomiting | 7 | (3.5) | 6 | (3.0) |
| Fatigue | 6 | (3.0) | 7 | (3.5) |
| Pain | 6 | (3.0) | 1 | (0.5) |
| Gait abnormal | 6 | (3.0) | 2 | (1.0) |
| Constipation | 6 | (3.0) | 3 | (1.5) |
| Coughing | 6 | (3.0) | 2 | (1.0) |

**Table 8. Most frequent adverse events with ≥ 3% difference of donepezil + memantine vs donepezil + placebo irrespective of relationship to study medication in patients with moderate to severe Alzheimer's disease. Daily dose range of donepezil 5-10 mg and of memantine 5-20mg. Memantine maintenance dose 20 mg daily.**

| **Adverse Event** | **Donep+Mem, n=202** | | **Donep+Plc, n=201** | |
|---|---|---|---|---|
| | **n** | **(%)** | **n** | **(%)** |
| Confusion | 16 | (7.9) | 4 | (2.0) |
| Headache | 13 | (6.4) | 5 | (2.5) |
| Inflicted injury | 10 | (5.0) | 16 | (8.0) |
| Diarrhea | 9 | (4.5) | 17 | (8.5) |
| Hypertension | 9 | (4.5) | 3 | (1.5) |

Based on the usual minimum 3% difference between the percentage of patients on memantine and donepezil vs. the patients on donepezil and placebo, there were only five types of adverse events (confusion, headache, inflicted injury, diarrhoea, and hypertension) with ≥3% different incidence between the combination and donepezil and placebo groups.

Out of these, only for three types of adverse events (confusion, headache, hypertension) a higher frequency was observed with memantine added as compared to donepezil alone. These are known and expected side effects of memantine within the < 10% range. For two adverse events (inflicted injury and diarrhea), a higher frequency was observed with donepezil plus placebo as compared to the donepezil-memantine combination.

Overall, the combination treatment appears not to increase any of the known side effects of the two drugs considered alone. In addition, the combination with memantine appears to lower the typical gastro-intestinal side effects of the cholinergic drug donepezil as established in very large patient numbers. Diarrhea and the the sometimes resulting fecal incontinence are known drivers of the necessary nursing home transfer of these severely ill patients.

Thus, the present results show no evidence of a proportional additive adverse event occurrence based on the known adverse event profiles of each drug administered alone. In contrast, AChEI adverse events rather appear to be modulated while known memantine adverse events remained basically the same both in terms of nature and incidence. In addition, the results disclosed in the instant Example suggest an improved tolerability of the prominent gastrointestinal side effects of the AChEI, which can be attributed to the combination with memantine. In addition, the nature and effect of the cognitive benefit is novel and unexpected, and supports therapeutic superiority for the combined approach.

### Discussion

The above-presented results support the safety and efficacy of memantine therapy for patients with moderate-to-severe AD and demonstrate that treatment with memantine combined with donepezil is superior to donepezil alone. Beneficial effects of adding memantine to a regimen of donepezil were observed on measures of cognition, daily functioning, and clinical global status. The superiority of the combination treatment was seen as early as 4 weeks after randomization and was evident for all measures at the 6 month end of study visit. Memantine, given as a dose of 20 mg/day concomitantly with donepezil, was safe and well tolerated in patients with moderate-to-severe AD.

### Conclusion

Treatment with memantine/donepezil resulted in improved cognitive performance relative to baseline, whereas treatment with donepezil alone was associated with continued cognitive decline. In other words, in contrast to currently used AD treatments, all of which have demonstrated at best a slowing of the expected decline in cognition, the combination treatment disclosed in the present study shows that already within the six month study period memantine in combination with AChEI produces an improvement in cognition. Such results have not been observed with memantine monotherapy either. Accordingly, they are surprising and unexpected.

The present invention is not to be limited in scope by the specific embodiments described herein. Indeed, various modifications of the invention in addition to those described herein will become apparent to those skilled in the art from the foregoing description. Such modifications are intended to fall within the scope of the appended claims.

All patents, applications, publications, test methods, literature, and other materials cited herein are hereby incorporated by reference.

Key embodiments of the invention include the following:
In a first embodiment, the invention comprioses a pharmaceutical product for treatment of dementia associated with a disorder of the central nervous system, said product comprising therapeutically effective dosages of a 1-aminocyclohexane derivative and an acetylcholinesterase inhibitor.
In a second embodiment, the invention comprises the product of the first embodiment, wherein said dosages for the 1-aminocyclohexane derivative and the acetylcholinesterase inhibitor are each in the range 1 to 200 mg.
In a third embodiment, the invention comprises the product of the second embodiment, wherein said dosage for the 1-aminocyclohexane derivative is in the range 10 to 40 mg and said dosage for the acetylcholinesterase inhibitor is in the range 5 to 24 mg.
In a fourth embodiment, the invention comprises the product of any of the first to third embodiments, wherein the 1-aminocyclohexane derivative is a compound of formula (I): 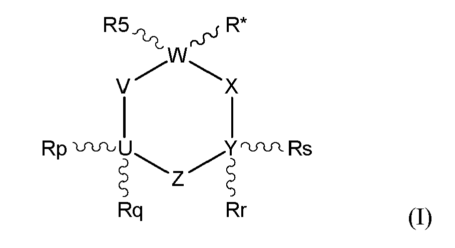 wherein:
   - R^{*} is -(A)ₙ-(CR¹R²)ₘ-NR³R⁴ in which
      n+m = 0, 1, or 2,
      A is selected from linear or branched C₁₋₆ alkylene, linear or branched C₂₋₆ alkenylene and linear or branched C₂₋₆ alkynylene, R¹ and R² are independently selected from hydrogen, linear or branched C₁₋₆ alkyl, linear or branched C₂₋₆ alkenyl, linear or branched C₂₋₆ alkynyl, aryl, substituted aryl and aralkyl,
      R³ and R⁴ are independently selected from hydrogen, linear or branched C₁₋₆ alkyl, linear or branched C₂₋₆ alkenyl and linear or branched C₂₋₆ alkynyl, or together form C₂₋₁₀ alkylene or C₂₋₁₀ alkenylene or together with the N form an optionally C₁₋₆ alkyl- or C₂₋₆ alkenyl-substituted 3-7-membered azacycloalkane or azacycloalkene, or independently R³ or R⁴ may join with R^{p}, R^{q}, R^{r} or R^{s} to form an alkylene chain -CH(R⁶) -
      (CH₂)ₜ- wherein t= 0 or 1, the left side of the alkylene chain is attached to U or Y and the right side of the alkylene chain is attached to N, and R⁶ is selected from hydrogen, linear or branched C₁₋₆ alkyl, linear or branched C₂₋₆ alkenyl, linear or branched C₂₋₆ alkynyl, aryl, substituted aryl and
      aralkyl; or independently R³ or R⁴ may join with R⁵ to form an alkylene chain represented by the formula -CH₂-CH₂-CH₂-(CH₂)ₜ- or an alkenylene chain represented by the formulae -CH=CH-CH₂-(CH₂)ₜ-, - CH=C=CH-(CH₂)ₜ- or -CH₂-CH=CH-(CH₂)ₜ-, wherein t= 0 or 1, the left side of the alkylene or alkenylene chain is attached to W and the right side of the alkylene ring is attached to N;
   - R⁵ is independently selected from hydrogen, linear or branched C₁₋₆ alkyl, linear or branched C₂₋₆ alkenyl and linear or branched C₂₋₆ alkynyl, or R⁵ combines with the carbon to which it is attached and an adjacent ring carbon to form a double bond;
   - R^{p}, R^{q}, R^{r} and R^{s} are independently selected from hydrogen, linear or branched C₁₋₆ alkyl, linear or branched C₂₋₆ alkenyl, linear or branched C₂₋₆ alkynyl, C₃₋₆ cycloalkyl, aryl, substituted aryl and aralkyl, or R^{p}, R^{q}, R^{r} and R^{s} independently may form a double bond with U or with Y to which it is attached, or R^{p}, R^{q}, R^{r} and R^{s} may combine together to represent a C₂₋₅ alkylene or alkenylene bridge which may in turn combine with R⁵ to form an additional C₁₋₃ alkylene or alkenylene bridge; and
   - the symbols U, V, W, X, Y and Z represent carbon atoms;
      or an optical isomer, diastereomer, polymorph, enantiomer, hydrate, pharmaceutically acceptable salt thereof or a mixture of any of the foregoing.

In a fifth embodiment, the invention comprises the product of the fourth embodiment, wherein the 1-aminocyclohexane derivative is selected from:
1-amino-3-phenyl adamantane,
1-amino-methyl adamantane,
1-amino-3,5-dimethyl adamantane (memantine),
1-amino-3-ethyl adamantane,
1-amino-3-isopropyl adamantane,
1-amino-3-n-butyl adamantane,
1-amino-3,5-diethyl adamantane,
1-amino-3,5-diisopropyl adamantane,
1-amino-3,5-di-n-butyl adamantane,
1-amino-3-methyl-5-ethyl adamantane,
1-N-methylamino-3,5-dimethyl adamantane,
1-N-ethylamino-3,5-dimethyl adamantane,
1-N-isopropyl-amino-3,5-dimethyl adamantane,
1-N,N-dimethyl-amino-3,5-dimethyl adamantane,
1-N-methyl-N-isopropyl-amino-3-methyl-5-ethyl adamantane,
1-amino-3-butyl-5-phenyl adamantane,
1-amino-3-pentyl adamantane,
1-amino-3,5-dipentyl adamantane,
1-amino-3-pentyl-5-hexyl adamantane,
1-amino-3-pentyl-5-cyclohexyl adamantane,
1-amino-3-pentyl-5-phenyl adamantane,
1-amino-3-hexyl adamantane,
1-amino-3,5-dihexyl adamantane,
1-amino-3-hexyl-5-cydohexyl adamantane,
1-amino-3-hexyl-5-phenyl adamantane,
1-amino-3-cyclohexyl adamantane,
1-amino-3,5-dicyclohexyl adamantane,
1-amino-3-cyclohexyl-5-phenyl adamantane,
1-amino-3,5-diphenyl adamantane,
1-amino-3,5,7-trimethyl adamantane,
1-amino-3,5-dimethyl-7-ethyl adamantane,
1-amino-3,5-diethyl-7-methyl adamantane,
1-N-pyrrolidino and 1-N-piperidine derivatives,
1-amino-3-methyl-5-propyl adamantane,
1-amino-3-methyl-5-butyl adamantane,
1-amino-3-methyl-5-pentyl adamantane,
1-amino-3-methyl-5-hexyl adamantane,
1-amino-3-methyl-5-cyclohexyl adamantane,
1-amino-3-methyl-5-phenyl adamantane,
1-amino-3-ethyl-5-propyl adamantane,
1-amino-3-ethyl-5-butyl adamantane,
1-amino-3-ethyl-5-pentyl adamantane,
1-amino-3-ethyl-5-hexyl adamantane,
1-amino-3-ethyl-5-cyclohexyl adamantane,
1-amino-3-ethyl-5-phenyl adamantane,
1-amino-3-propyl-5-butyl adamantane,
1-amino-3-propyl-5-pentyl adamantane,
1-amino-3-propyl-5-hexyl adamantane,
1-amino-3-propyl-5-cyclohexyl adamantane,
1-amino-3-propyl-5-phenyl adamantane,
1-amino-3-butyl-5-pentyl adamantane,
1-amino-3-butyl-5-hexyl adamantane,
1-amino-3-butyl-5-cyclohexyl adamantane, optical isomers, diastereomers, enantiomers, hydrates, N-methyl, N,N-dimethyl, N-ethyl and N-propyl derivatives and pharmaceutically acceptable salts thereof and mixtures of any of the foregoing.

In a sixth embodiment, the invention comprises the product of any of the first to third embodiments, wherein the 1-aminocyclohexane derivative is selected from memantine and prodrugs, salts, isomers, analogs and derivatives thereof.

In a seventh embodiment, the invention comprises the product of any of the first to third embodiments, wherein the 1-aminocyclohexane derivative is memantine.

In an eighth embodiment, the invention comprises the product of the fourth embodiment, wherein the 1-aminocyclohexane derivative is selected from:
1-amino-1,3, 5-trimethylcyclohexane,
1-amino-1(trans),3(trans),5-trimethylcyclohexane,
1-amino-1(cis),3(cis),5-trimethylcyclohexane,
1-amino-1,3,3,5-tetramethylcyclohexane,
1-amino-1,3,3,5,5-pentamethylcyclohexane (neramexane),
1-amino-1,3,5,5-tetramethyl-3-ethylcyclohexane,
1-amino-1,5,5-trimethyl-3,3-diethylcyclohexane,
1-amino-1,5,5-trimethyl-cis-3-ethylcyclohexane,
1-amino-(1S,5S)cis-3-ethyl-1,5,5-trimethylcyclohexane,
1-amino-1,5,5-trimethyl-trans-3-ethylcyclohexane,
1-amino-(1R,5S)trans-3-ethyl-1,5,5-trimethylcydohexane,
1-amino-1-ethyl-3,3,5,5-tetramethylcyclohexane,
1-amino-1-propyl-3,3,5,5-tetramethylcyclohexane,
N-methyl-1-amino-1,3,3,5,5-pentamethylcyclohexane,
N-ethyl-1-amino-1,3,3,5,5-pentamethyl-cyclohexane,
N-(1,3,3,5,5-pentamethylcyclohexyl) pyrrolidine,
3,3,5,5-tetramethylcyclohexylmethylamine,
1-amino-1-propyl-3,3,5,5-tetramethylcyclohexane,
1 amino-1,3,3,5(trans)-tetramethylcyclohexane (axial amino group),
3-propyl-1,3,5,5-tetramethylcyclohexylamine semihydrate,
1-amino-1,3,5,5-tetramethyl-3-ethylcyclohexane,
1-amino-1,3,5-trimethylcyclohexane,
1-amino-1,3-dimethyl-3-propylcyclohexane,
1-amino-1,3(trans),5(trans)-trimethyl-3(cis)-propylcyclohexane,
1-amino-1,3-dimethyl-3-ethylcyclohexane,
1-amino-1,3,3-trimethylcyclohexane,
cis-3-ethyl-1(trans)-3(trans)-5-trimethylcyclohexamine,
1-amino-1,3(trans)-dimethylcyclohexane,
1,3,3-trimethyl-5,5-dipropylcyclohexylamine,
1-amino-1-methyl-3(trans)-propylcyclohexane,
1-methyl-3(cis)-propylcyclohexylamine,
1-amino-1-methyl-3 3(trans)-ethylcyclohexane,
1-amino-1,3,3-trimethyl-5(cis)-ethylcyclohexane,
1-amino-1,3,3-trimethyl-5(trans)-ethylcyclohexane,
cis-3-propyl-1,5,5-trimethylcyclohexylamine,
trans-3-propyl-1,5,5-trimethylcyclohexylamine,
N-ethyl-1,3,3,5,5-pentamethylcyclohexylamine,
N-methyl-1-amino-1,3,3,5.5-pentamethylcyclohexane,
1-amino-1-methylcyclohexane,
N,N-dimethyl-1-amino-1,3,3,5,5-pentamethylcyclohexane,
2-(3,3,5,5-tetramethylcyclohexyl)ethylamine,
2-methyl-1-(3,3,5,5-tetramethylcyclohexyl)propyl-2-amine,
2-(1,3,3,5,5-pentamethylcyclohexyl-1)-ethylamine semihydrate,
N-(1,3,3,5,5-pentamethylcyclohexyl)-pyrrolidine,
1-amino-1,3(trans),5(trans)-trimethylcyclohexane,
1-amino-1,3(cis),5(cis)-trimethylcyclohexane,
1-amino-(1R,SS)trans-5-ethyl-1,3,3-trimethylcyclohexane,
1-amino-(1S,SS)cis-5-ethyl-1,3,3-trimethylcyclohexane,
1-amino-1,5, 5-trimethyl-3(cis)-isopropyl-cyclohexane,
1-amino-1,5,5-trimethyl-3(trans)-isopropyl-cyclohexane,
1-amino-1-methyl-3(cis)-ethyl-cyclohexane,
1-amino-1--methyl-3(cis)-methyl-cyclohexane,
1-amino-5,5-diethyl-1,3,3-trimethyl-cyclohexane,
1-amino-1,3,3,5,5-pentamethylcyclohexane,
1-amino-1,5,5-trimethyl-3,3-diethylcyclohexane,
1-amino-1-ethyl-3,3,5,5-tetramethylcyclohexane,
N-ethyl-1-amino-1,3,3,5,5-pentamethylcyclohexane,
N-(1,3,5-trimethylcyclohexyl)pyrrolidine or piperidine,
N-[1,3(trans),5(trans)-trimethylcyclohexyl]pyrrolidine or piperidine,
N-[1,3(cis),5(cis)-trimethylcyclohexyl]pyrrolidine or piperidine,
N-(1,3,3,5-tetramethylcyclohexyl)pyrrolidine or piperidine,
N-(1,3,3,5,5-pentamethylcyclohexyl)pyrrolidine or piperidine,
N-(1,3,5,5-tetramethyl-3-ethylcyclohexyl)pyrrolidine or piperidine,
N-(1,5,5-trimethyl-3,3-diethylcyclohexyl)pyrrolidine or piperidine,
N-(1,3,3-trimethyl-cis-5-ethylcyclohexyl)pyrrolidine or piperidine,
N-[(1S,SS)cis-5-ethyl-1,3,3-trimethylcyclohexyl]pyrrolidine or piperidine,
N-(1,3,3-trimethyl-trans-5-ethylcyclohexyl)pyrrolidine or piperidine,
N-[(1R,SS)trans-5-ethyl,3,3-trimethylcyclohexyl]pyrrolidine or piperidine,
N-(1-ethyl-3,3,5,5-tetramethylyclohexyl)pyrrolidine or piperidine,
N-(1-propyl-3,3,5,5-tetramethylcyclohexyl)pyrrolidine or piperidine,
N-(1,3,3,5,5-pentamethylcyclohexyl)pyrrolidine,
   optical isomers, diastereomers, enantiomers, hydrates and pharmaceutically acceptable salts thereof, and mixtures of any of the foregoing.

In a nineth embodiment, the invention comprises the product of any of the first to third embodiments, wherein the 1-aminocyclohexane derivative is selected from neramexane and prodrugs, salts, isomers, analogs and derivatives thereof.

In a tenth embodiment, the invention comprises the product of any of the first to third embodiments, wherein the 1-aminocyclohexane derivative is neramexane.

In an eleventh embodiment, the invention comprises the product of any of the first to tenth embodiments, wherein the acetylcholinesterase inhibitor is reversible or pseudo-reversible.

In a twelfth embodiment, the invention comprises the product of any of the first to eleventh embodiments, wherein the acetylcholinesterase inhibitor is selected from galantamine, tacrine, donepezil and rivastigmine.

In a thirteenth embodiment, the invention comprises the product of any of the first to twelfth embodiments in the form of a single formulation.

In a fourteenth embodiment, the invention comprises the product of the thirteenth embodiment, wherein the 1-aminocyclohexane derivative and acetylcholinesterase inhibitor are combined together with at least one pharmaceutically acceptable carrier or excipient.

In a fifteenth embodiment, the invention comprises the product of the thirteenth of fourteenth embodiment which is in solid dosage form for oral administration.

In a sixteenth embodiment, the invention comprises the use of a 1-aminocyclohexane derivative and an acetylcholinesterase inhibitor in the manufacture of a medicament for delaying the onset or progression of dementia associated with a disorder of the central nervous system.

In a seventeenth embodiment, the invention comprises the use of the sixteenth embodiment, wherein said disorder is cereobrovascular disease or Down's syndrome.

In an eighteenth embodiment, the invention comprises the use of the sixteenth embodiment, wherein said disorder is Alzheimer's disease.

In a nineteenth embodiment, the invention comprises the use of any of the sixteenth to eighteenth embodiments, wherein said medicament is manufactured for conjoint administration of said 1-aminocyclohexane derivative and said acetylcholinesterase inhibitor.

In a twentieth embodiment, the invention comprises the use of any of the sixteenth to eighteenth embodiments, wherein said medicament is manufactured for combined administration of said 1-aminocyclohexane derivative and said acetylcholinesterase inhibitor as a single composition optionally further containing at least one pharmaceutically acceptable carrier or excipient.

In a twenty-first embodiment, the invention comprises a method for delaying the onset or progression of dementia associated with a disorder of the central nervous system which comprises administering to a patient in need of such treatment the pharmaceutical product of any of the first to fifteenth embodiments.

In a twenty-second embodiment, the invention comprises the method of the twenty-first embodiment, wherein the disorder is cerebrovascular disease or Down's syndrome.

In a twenty-third embodiment, the invention comprises the method of the twenty-first embodiment, wherein the disorder is Alzheimer's disease.

In a twenty-fourth embodiment, the invention comprises the method of the twenty-third embodiment, wherein the amounts of 1-aminocyclohexane derivative and acetylcholinesterase inhibitor are such as to be effective in combination in improving at least one assessment selected from Severe Impairment Battery Test, AD Cooperative Study-Activities of Daily Living Inventory and Cliniciain's Interview-Based Impression of Change Plus Version.

## Claims

1. A 1-aminocyclohexane derivative selected from those of general formula (I): 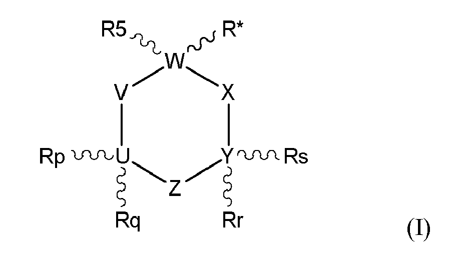 wherein:
-
R^{*} is -(A)ₙ-(CR¹R²)ₘ-NR³R⁴
,
n+m = 0, 1, or 2,
A is selected from linear or branched C₁₋₆alkylene, linear or branched C₂₋₆alkenylene and linear or branched C₂₋₆ alkynylene,
R¹ and R² are independently selected from hydrogen, linear or branched C₁₋₆alkyl, linear or branched C₂₋₆alkenyl, linear or branched C₂₋₆alkynyl, aryl, substituted aryl and arylalkyl,
R³ and R⁴ are independently selected from hydrogen, linear or branched C₁₋₆alkyl, linear or branched C₂₋₆alkenyl and linear or branched C₂₋₆alkynyl, or together form C₂₋₁₀alkylene or C₂₋₁₀alkenylene or together with the N form an optionally C₁₋₆alkyl- or C₂₋₆ alkenyl-substituted 3-7-membered azacycloalkane or azacycloalkene;
- R⁵ is independently selected from hydrogen, linear or branched C₁₋₆alkyl, linear or branched C₂₋₆alkenyl and linear or branched C₂₋₆alkynyl, or R⁵ combines with the carbon to which it is attached and the next adjacent ring carbon atom to form a double bond;
- R^{p}, R^{q}, R^{r}, and R^{s}, are independently selected from hydrogen, linear or branched C₁₋₆alkyl, linear or branched C₂₋₆alkenyl, linear or branched C₂₋₆alkynyl, C₃₋₆cycloalkyl, aryl, substituted aryl and arylalkyl, or R^{p}, R^{q}, R^{r} and R^{s} independently may form a double bond with U or with Y or to which it is attached; and
- the symbols U, V, W, X, Y and Z represent carbon atoms,
and optical isomers, diastereomers, polymorphs, enantiomers, hydrates, pharmaceutically acceptable salts, and mixtures thereof, in combination with an acetylcholinesterase inhibitor (AChEI) selected from donepezil, rivastigmine, tacrine, galantamine, physostigmine, huperzine A, zanapezil, ganstigmine, phenserine, phenethylnorcymserine (PENC), cymserine, thiacymserine, SPH 1371 (galantamine plus), ER 127528, RS 1259, and F3796, for use in the treatment of dementia associated with a disorder of the central nervous system (CNS).

2. The combination as claimed in Claim 1, wherein the 1-aminocyclohexane derivative is selected from 1-amino-1,3,5-trimethylcyclohexane,
1-amino-1(trans),3(trans),5-trimethylcyclohexane,
1-amino-1(cis),3(cis),5-trimethylcyclohexane,
1-amino-1,3,3,5-tetramethylcyclohexane,
1-amino-1,3,3,5,5-pentamethylcyclohexane (neramexane),
1-amino-1,3,5,5-tetramethyl-3-ethylcyclohexane,
1-amino-1,5,5-trimethyl-3,3-diethylcyclohexane,
1-amino-1,5,5-trimethyl-cis-3-ethylcyclohexane,
1-amino-(1S,5S)cis-3-ethyl-1,5,5-trimethylcyclohexane,
1-amino-1,5,5-trimethyl-trans-3-ethylcyclohexane,
1-amino-(1R,5S)trans-3-ethyl-1,5,5-trimethylcyclohexane,
1-amino-1-ethyl-3,3,5,5-tetramethylcyclohexane,
1-amino-1-propyl-3,3,5,5-tetramethylcyclohexane,
N-methyl-1-amino-1,3,3,5,5-pentamethylcyclohexane,
N-ethyl-1-amino-1,3,3,5,5-pentamethyl-cyclohexane,
N-(1,3,3,5,5-pentamethylcyclohexyl) pyrrolidine,
3,3,5,5-tetramethylcyclohexylmethylamine,
1-amino-1-propyl-3,3,5,5-tetramethylcyclohexane,
1 amino-1,3,3,5(trans)-tetramethylcyclohexane (axial amino group),
3-propyl-1,3,5,5-tetramethylcyclohexylamine semihydrate,
1-amino-1,3,5,5-tetramethyl-3-ethylcyclohexane,
1-amino-1,3,5-trimethylcyclohexane,
1-amino-1,3-dimethyl-3-propylcyclohexane,
1-amino-1,3(trans),5(trans)-trimethyl-3(cis)-propylcyclohexane,
1-amino-1,3-dimethyl-3-ethylcyclohexane,
1-amino-1,3,3-trimethylcyclohexane,
cis-3-ethyl-1(trans)-3(trans)-5-trimethylcyclohexamine,
1-amino-1,3(trans)-dimethylcyclohexane,
1,3,3-trimethyl-5,5-dipropylcyclohexylamine,
1-amino-1-methyl-3(trans)-propylcyclohexane,
1-methyl-3(cis)-propylcyclohexylamine,
1-amino-1-methyl-3(trans)-ethylcyclohexane,
1-amino-1,3,3-trimethyl-5(cis)-ethylcyclohexane,
1-amino-1,3,3-trimethyl-5(trans)-ethylcyclohexane,
cis-3-propyl-1,5,5-trimethylcyclohexylamine,
trans-3-propyl-1,5,5-trimethylcyclohexylamine,
N-ethyl-1,3,3,5,5-pentamethylcyclohexylamine,
N-methyl-1-amino-1,3,3,5.5-pentamethylcyclohexane,
1-amino-1-methylcyclohexane,
N,N-dimethyl-1-amino-1,3,3,5,5-pentamethylcyclohexane,
2-(3,3,5,5-tetramethylcyclohexyl)ethylamine,
2-methyl-1-(3,3,5,5-tetramethylcyclohexyl)propyl-2-amine,
2-(1,3,3,5,5-pentamethylcyclohexyl-1)-ethylamine semihydrate,
N-(1,3,3,5,5-pentamethylcyclohexyl)-pyrrolidine,
1-amino-1,3(trans),5(trans)-trimethylcyclohexane,
1-amino-1,3(cis),5(cis)-trimethylcyclohexane,
1-amino-(1R,SS)trans-5-ethyl-1,3,3-trimethylcyclohexane,
1-amino-(1S,SS)cis-5-ethyl-1,3,3-trimethylcyclohexane,
1-amino-1,5, 5-trimethyl-3(cis)-isopropyl-cyclohexane,
1-amino-1,5,5-trimethyl-3(trans)-isopropyl-cyclohexane,
1-amino-1-methyl-3(cis)-ethyl-cyclohexane,
1-amino-1-methyl-3(cis)-methyl-cyclohexane,
1-amino-5,5-diethyl-1,3,3-trimethyl-cyclohexane,
1-amino-1,3,3,5,5-pentamethylcyclohexane,
1-amino-1,5,5-trimethyl-3,3-diethylcyclohexane,
1-amino-1-ethyl-3,3,5,5-tetramethylcyclohexane,
N-ethyl-1-amino-1,3,3,5,5-pentamethylcyclohexane,
N-(1,3,5-trimethylcyclohexyl)pyrrolidine or piperidine,
N-[1,3(trans),5(trans)-trimethylcyclohexyl]pyrrolidine or piperidine,
N-[1,3(cis),5(cis)-trimethylcyclohexyl]pyrrolidine or piperidine,
N-(1,3,3,5-tetramethylcyclohexyl)pyrrolidine or piperidine,
N-(1,3,3,5,5-pentamethylcyclohexyl)pyrrolidine or piperidine,
N-(1,3,5,5-tetramethyl-3-ethylcyclohexyl)pyrrolidine or piperidine,
N-(1,5,5-trimethyl-3,3-diethylcyclohexyl)pyrrolidine or piperidine,
N-(1,3,3-trimethyl-cis-5-ethylcyclohexyl)pyrrolidine or piperidine,
N-[(1S,SS)cis-5-ethyl-1,3,3-trimethylcyclohexyl]pyrrolidine or piperidine,
N-(1,3,3-trimethyl-trans-5-ethylcyclohexyl)pyrrolidine or piperidine,
N-[(1R,SS)trans-5-ethyl,3,3-trimethylcyclohexyl]pyrrolidine or piperidine,
N-(1-ethyl-3,3,5,5-tetramethylyclohexyl)pyrrolidine or piperidine,
N-(1-propyl-3,3,5,5-tetramethylcyclohexyl)pyrrolidine or piperidine,
N-(1,3,3,5,5-pentamethylcyclohexyl)pyrrolidine,
and optical isomers, diastereomers, enantiomers, hydrates, pharmaceutically acceptable salts, and mixtures thereof.

3. The combination as claimed in Claim 1 or 2, wherein the 1-aminocyclohexane derivative is selected from neramexane and salts, isomers, and analogues thereof.

4. The combination as claimed in any one of Claims 1 to 3, wherein the acetylcholinesterase inhibitor (AChEI) is selected from galantamine, tacrine, donepezil, and rivastigmine.

5. The combination as claimed in any one of Claims 1 to 4, wherein the disorder is Alzheimer's disease.

6. The combination as claimed in any one of Claims 1 to 4, wherein the disorder is cerebrovascular disease or Down's syndrome.

7. The combination as claimed in any preceding claim, wherein the dosage for the 1-aminocyclohexane derivative and the acetylcholinesterase inhibitor are each in the range of 1 to 200 mg.

8. The combination as claimed in Claim 7, wherein the dosage for the 1-aminocyclohexane derivative is in the range of 10 to 40 mg and the dosage for the acetylcholinesterase inhibitor is in the range of 5 to 24 mg.
